(19) Europäisches Patentamt
European Patent Office
Office européen des brevets

(11) EP 4 368 724 A1

(12) EUROPEAN PATENT APPLICATION

(43) Date of publication:
**15.05.2024 Bulletin 2024/20**

(21) Application number: **22206451.1**

(22) Date of filing: **09.11.2022**

(51) International Patent Classification (IPC):
***C12Q 1/37*** *(2006.01)*

(52) Cooperative Patent Classification (CPC):
**C12Q 1/37**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
- **Universität Regensburg**
  **93053 Regensburg (DE)**
- **PHILIPPS-UNIVERSITÄT MARBURG**
  **35037 Marburg (DE)**

(72) Inventor: **The designation of the inventor has not yet been filed**

(74) Representative: **Engelhard, Markus**
**Boehmert & Boehmert**
**Anwaltspartnerschaft mbB**
**Pettenkoferstrasse 22**
**80336 München (DE)**

(54) **METHOD, LIPOSOME COLLECTION, AND KIT FOR DETERMINING COMPLEMENT ACTIVITY**

(57) The present invention relates to a method of determining complement activity, wherein said method comprises determining classical complement pathway activity, determining alternative complement pathway activity, and/or determining lectin complement pathway activity. The present invention further relates to a liposome collection for determining complement activity. Furthermore, the present invention relates to a kit for determining complement activity. The present invention also relates to a liposome collection or kit for use in a method of diagnosing, monitoring, or treating a complement associated disorder.



Processed by Luminess, 75001 PARIS (FR)

## Description

FIELD OF THE INVENTION

The present invention relates to a method of determining complement activity. The present invention further relates to a liposome collection for determining complement activity. Furthermore, the present invention relates to a kit for determining complement activity. The present invention also relates to a liposome collection or kit for use in a method of diagnosing, monitoring, or treating a complement associated disorder.

BACKGROUND OF THE INVENTION

The complement system is a part of the immune system that plays a crucial role in fighting infections, whereas abnormalities in this system cause a variety of diseases. The fully functional and balanced complement system plays a role in physiology of a healthy organism and is a multidimensional innate immune surveillance system. On the one hand it removes apoptotic cells, thereby regulating cell survival, and on the other hand it inhibits outgrowth of microorganisms which can cause infectious diseases. The complement system involves an in-concert activity of over 40 proteins which are activated by at least three different stimuli: the classical, lectin and alternative pathway. Activation results in tagging of cells for phagocytosis, release of anaphylatoxins and lysis of cells.

Complement-mediated body reactions are influenced by genetic deficiency, viral and microbial infections, autoimmune reactions, complement targeting therapeutics, and artificial surfaces in hemodialysis and extracorporeal circuits. Up to date, complement activation capacity is primarily measured and used for diagnosis in patients to determine complement deficiency or complement dysregulation in autoimmune reactions in standard clinical diagnostic laboratories.

The present complement activity assays have significant disadvantages. The present complement activity assays are not appropriate to determine high functional levels or dysregulation. Furthermore, the assays are expensive with multiple reagents, washing steps and a long assay time. Furthermore, major disadvantages are the fact that animal products are used, which do not present a long shelf life as well creating massive batch-to-batch variations. The characteristics of the existing complement activity assays cannot keep up with the rapid drug development. In 2020, from the enormous number of 870 clinical studies evaluating various complement-targeted therapeutics involving nearly 2,430,000 patients only 16 studies determined complement activation capacity as an end point measurement.

In recent years, a new class of immunosuppressive drugs, that target the complement system, have been approved for clinical use. These recent advances have created a demand for novel tests such as blood tests to diagnose, follow-up and manage complement-related diseases as well as to monitor treatment response. However, there currently is a large deficit in blood tests and laboratory analysis for the complement system.

Thus, there is a need for efficient complement activity assays, such as methods and tools for determining complement activity. For example, there is a need for inexpensive, fast, and high-throughput assays for complement activity. Particularly, there is a need for means which allow to specifically determine complement activity, such as activity of the three complement pathways. There is also a need for means for treating, diagnosing, and monitoring complement associated disorders.

SUMMARY OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to a method of determining complement activity, wherein said method comprises A) determining classical complement pathway activity, B) determining alternative complement pathway activity, and/or C) determining lectin complement pathway activity; wherein

A) said determining classical complement pathway activity comprises the steps of

a) providing a sample containing or suspected of containing one or more active complement system components, optionally a serum sample, and, separately, providing one or more liposomes comprising a marker;

wherein said one or more liposomes comprise cholesterol, preferably at least 30 mol% cholesterol, more preferably at least 40 mol% cholesterol;
wherein, optionally, said one or more liposomes comprise a classical complement pathway activating agent, preferably selected from antibodies, antigens, and ligands such as biotin or streptavidin;

b) contacting said one or more liposomes with said sample to allow the one or more active complement system components, if present in said sample, to lyse said one or more liposomes; wherein a lysis of said one or more liposomes releases said marker from said one or more liposomes; and
c) detecting said released marker;

B) said determining alternative complement pathway activity comprises the steps of

a) providing a sample containing or suspected of containing one or more active complement system components, optionally a serum sample, and, separately, providing one or more liposomes comprising a marker;

wherein said one or more liposomes comprise an alternative complement pathway activating agent, preferably a lipopolysaccharide, more preferably about 0.01 mol% to about 5 mol% lipopolysaccharide, even more preferably about 1 mol% lipopolysaccharide;
wherein, optionally, said one or more liposomes comprise cholesterol, preferably $\leq$ 10 mol% cholesterol;

b) optionally, contacting said sample and/or said one or more liposomes with a classical complement pathway inhibitor and a lectin complement pathway inhibitor;
wherein, preferably, said classical complement pathway inhibitor and/or said lectin complement pathway inhibitor comprise(s) EGTA, C1-inhibitor, an anti-MASP antibody, and/or C4BP, preferably EGTA and/or a C1-inhibitor;
c) contacting said one or more liposomes with said sample to allow the one or more active complement system components, if present in said sample, to lyse said one or more liposomes; wherein a lysis of said one or more liposomes releases said marker from said one or more liposomes; and
d) detecting said released marker;

C) said determining lectin complement pathway activity comprises the steps of

a) providing a sample containing or suspected of containing one or more active complement system components, optionally a serum sample, and, separately, providing one or more liposomes comprising a marker;

wherein said one or more liposomes comprise a lectin complement pathway activating agent, preferably comprising a sugar moiety, an acetyl moiety and/or sialic acid;
wherein, preferably, said liposome comprises cholesterol, preferably less than 10 mol% cholesterol;

b) optionally, contacting said sample and/or said one or more liposomes with a classical complement pathway inhibitor and/or an alternative complement pathway inhibitor;
wherein, preferably, said classical complement pathway inhibitor comprises an anti-C1q85 antibody or an antigen-binding peptide thereof, and/or said alternative complement pathway inhibitor is selected from complement factor H, mini-complement factor H, an anti-factor B antibody or antigen-binding fragment thereof, an anti-properdin antibody or antigen-binding fragments thereof, and any combination thereof;
c) contacting said one or more liposomes with said sample to allow the one or more active complement system components, if present in said sample, to lyse said one or more liposomes; wherein a lysis of said one or more liposomes releases said marker from said one or more liposomes; and
d) detecting said released marker.

In one embodiment, said A) determining classical complement pathway activity, B) determining alternative complement pathway activity, and/or C) determining lectin complement pathway activity further comprise(s):

a step of detecting a base level of said marker; optionally prior to said step of contacting said one or more liposomes with said sample, at a time point in the range of from about 1 sec to about 20 min, preferably of from about 1 sec to about 15 min, after an initial contact of said one or more liposomes with said sample, and/or using an inactivated serum and/or a buffer;
optionally, a step of determining complement activity by comparing the released marker detected in steps A)c), B)d), and C)d), respectively, with the base level of said marker detected in said step of detecting a base level of said marker,

wherein, preferably, a higher level of said released marker detected in steps A)c), B)d), and C)d), respectively, compared to said base level of said marker indicates complement activity.

In one embodiment, said detecting in step A)c), step B)d), and/or step C)d) comprises or consists of a time-resolved detection of said released marker.

In one embodiment, said detecting in step A)c), step B)d), and/or step C)d) comprises or consists of a time-resolved detection of said released marker, and wherein an increase or decrease in said released marker over time, preferably an increase in said released marker over time, indicates complement activity.

In one embodiment, said marker is selected from a fluorescent marker, preferably pyrenetetrasulfonic acid, sulforhodamine B, indocyanine green such as 2-[2-[2-chloro-3-[2-[1,3-dihydro-3,3-dimethyl-1-(4-sulfobutyl)-2H-indol-2-ylidene]ethylidene]-1-cyclohexen-1-yl]ethenyl]-3,3-dimethyl-1-(4-sulfobutyl)-3H-indolium, or carboxyfluorescein; an electrochemical marker, preferably potassium hexaferricyanide, potassium hexaferrocyanide, or ruthenium hexamine; a colorimetric marker, preferably sulforhodamine B; a chemiluminescent marker, preferably luminol or a derivative of luminol such as mCOOH-luminol; an electrochemiluminescent marker, preferably luminol, a derivative of luminol, or ruthenium bipyridyl; a bioluminescent marker such as GFP, other bioluminescent proteins, or luciferase; and a combination thereof; wherein, preferably, said marker is water-soluble; wherein, preferably, said marker is selected from a fluorescent marker, an electrochemical marker, and a combination thereof;

wherein, more preferably, said marker is selected from sulforhodamine B and mCOOH-luminol.

In one embodiment, said marker is a fluorescent marker and said marker is quenched in a nonlysed liposome;

wherein, preferably, said one or more liposomes are nonlysed liposomes prior to said lysis of said one or more liposomes;

wherein, optionally,

an increase in released marker over time detected in step(s) A)c), step B)d), and/or step C)d) indicates complement activity.

In one embodiment, said one or more liposomes comprising a marker provided in step A)a) comprise a first marker, said one or more liposomes comprising a marker provided in step B)a) comprise a second marker, and said one or more liposomes comprising a marker provided in step C)a) comprise a third marker, wherein said first marker, said second marker, and said third marker are different markers.

In one embodiment, said method comprises determining, preferably simultaneously or sequentially, at least two of A) determining classical complement pathway activity, B) determining alternative complement pathway activity, and C) determining lectin complement pathway activity;

wherein, preferably, said detecting said released marker of steps A)c), B)d), and/or C)d) is performed simultaneously or sequentially, preferably simultaneously.

In one embodiment, said one or more liposomes provided in step A)a) comprise cholesterol and phospholipids; preferably comprise cholesterol, 1,2-dipalmitoyl-sn-glycero-3-phosphocholine, and 1,2-dipalmitoyl-sn-glycero-3-phospho-(1'-rac-glycerol); more preferably comprise at least 30 mol% cholesterol, preferably at least 40 mol% cholesterol, 1,2-dipalmitoyl-sn-glycero-3-phosphocholine in the range of from 10 mol% to 30 mol%, and 1,2-dipalmitoyl-sn-glycero-3-phospho-(1'-rac-glycerol) in the range of from 25 mol% to 45 mol%; wherein, optionally, said one or more liposomes provided in step A)a) further comprise a classical complement pathway activating agent, preferably selected from antibodies, antigens, and ligands such as biotin or streptavidin;

said one or more liposomes provided in step B)a) comprise phospholipids and cholesterol, preferably ≤ 10 mol% cholesterol; preferably comprise 1,2-dipalmitoyl-sn-glycero-3-phosphocholine, 1,2-dipalmitoyl-sn-glycero-3-phospho-(1'-rac-glycerol), and ≤ 10 mol% cholesterol; more preferably comprise 1,2-dipalmitoyl-sn-glycero-3-phosphocholine in the range of from 65 mol% to 85 mol%, 1,2-dipalmitoyl-sn-glycero-3-phospho-(1'-rac-glycerol) in the range of from 10 mol% to 30 mol%, and ≤ 10 mol% cholesterol; and/or

said one or more liposomes provided in step C)a) comprise phospholipids and ≤ 10 mol% cholesterol; preferably comprise 1,2-dipalmitoyl-sn-glycero-3-phosphocholine, 1,2-dipalmitoyl-sn-glycero-3-phospho-(1'-rac-glycerol), and ≤ 10 mol% cholesterol; more preferably comprise 1,2-dipalmitoyl-sn-glycero-3-phosphocholine in the range of from 65 mol% to 85 mol%, 1,2-dipalmitoyl-sn-glycero-3-phospho-(1'-rac-glycerol) in the range of from 10 mol% to 30 mol%, and ≤ 10 mol% cholesterol;

wherein, optionally, said one or more liposomes provided in step A)a), step B)a), and/or C)a) comprise 1,2-dipalmitoyl-sn-glycero-3-ethylphosphocholine;

wherein, optionally, said one or more liposomes provided in step B)a) and/or said one or more liposomes provided in step C)a) comprise a classical complement pathway inhibitor, preferably an anti-C1q85 antibody or an antigen-binding fragment thereof.

In one embodiment, said one or more liposomes provided in step A)a), said one or more liposomes provided in step B)a), and/or said one or more liposomes provided in step C)a) are selected from anionic liposomes, PEGylated liposomes, and cationic liposomes, preferably anionic liposomes.

In one embodiment, step B)b) comprises contacting said sample and/or said one or more liposomes with a classical complement pathway inhibitor and/or a lectin complement pathway inhibitor selected from $Ca^{2+}$ complexing agents, preferably EGTA, in the presence of $Mg^{2+}$, preferably in the presence of at least 0.001 mM MgCl2, more preferably in the presence of at least 0.1 mM MgCl2, even more preferably in the presence of at least 0.5 mM MgCl2, e.g. of about 1 mM MgCl2.

In one embodiment, said method comprises a positive control and/or a negative control;

wherein said positive control comprises contacting said one or more liposomes provided in step A)a), B)a), and/or C)a) with a surfactant and/or solvent, preferably a surfactant, more preferably a surfactant selected from n-octyl-β-d-glucoside, 2-[4-(2,4,4-trimethylpentan-2-yl)phenoxy]ethanol, polyoxyethylene (20) sorbitan monolaurate, and polyoxyethylene (80) sorbitan monooleate; and

wherein said negative control comprises replacing said sample, optionally said serum sample, in step A)a), B)a), and/or C)a) with a negative control sample which comprises or consists of inactive serum.

In a further aspect, the present invention relates to a liposome collection for determining complement activity, preferably for A) determining classical complement pathway activity, B) determining alternative complement pathway activity, and/or C) determining lectin complement pathway activity, comprising:

- A) a liposome for determining classical complement pathway activity, comprising a marker, cholesterol, preferably at least 30 mol% cholesterol, more preferably at least 40 mol% cholesterol, and phospholipids;

  preferably comprising a marker, cholesterol, 1,2-dipalmitoyl-sn-glycero-3-phosphocholine, and 1,2-dipalmitoyl-sn-glycero-3-phospho-(1'-rac-glycerol);

  more preferably comprising a marker, at least 30 mol% cholesterol, preferably at least 40 mol% cholesterol, 1,2-dipalmitoyl-sn-glycero-3-phosphocholine in the range of from 10 mol% to 30 mol%, and 1,2-dipalmitoyl-sn-glycero-3-phospho-(1'-rac-glycerol) in the range of from 25 mol% to 45 mol%;

  wherein, optionally, said liposome further comprises a classical complement pathway activating agent, preferably selected from antibodies, antigens, and ligands such as biotin or streptavidin;

- B) a liposome for determining alternative complement pathway activity comprising a marker, an alternative complement pathway activating agent, cholesterol, preferably ≤ 10 mol% cholesterol, and phospholipids;

  preferably comprising a marker, an alternative complement pathway activating agent, cholesterol, 1,2-dipalmitoyl-sn-glycero-3-phosphocholine, and 1,2-dipalmitoyl-sn-glycero-3-phospho-(1'-rac-glycerol);

  more preferably comprising a marker, an alternative complement pathway activating agent, ≤ 10 mol% cholesterol, 1,2-dipalmitoyl-sn-glycero-3-phosphocholine in the range of from 65 mol% to 85 mol%, and 1,2-dipalmitoyl-sn-glycero-3-phospho-(1'-rac-glycerol) in the range of from 10 mol% to 30 mol%;

  wherein, preferably, said alternative complement pathway activating agent is a lipopolysaccharide, preferably about 0.01 mol% to about 5 mol% lipopolysaccharide, more preferably about 1 mol% lipopolysaccharide; and/or

- C) a liposome for determining lectin complement pathway activity, comprising a marker, a lectin complement pathway activating agent, cholesterol, preferably ≤ 10 mol% cholesterol, and phospholipids;

  preferably comprising a marker, a lectin complement pathway activating agent, cholesterol, 1,2-dipalmitoyl-sn-glycero-3-phosphocholine, and 1,2-dipalmitoyl-sn-glycero-3-phospho-(1'-rac-glycerol);

  more preferably comprising a marker, a lectin complement pathway activating agent, ≤ 10 mol% cholesterol, 1,2-dipalmitoyl-sn-glycero-3-phosphocholine in the range of from 65 mol% to 85 mol%, and 1,2-dipalmitoyl-sn-glycero-3-phospho-(1'-rac-glycerol) in the range of from 10 mol% to 30 mol%;

  wherein, preferably, said lectin complement pathway activating agent comprises a sugar moiety, an acetyl moiety, and/or sialic acid;

wherein, optionally, the liposome for determining alternative complement pathway activity B) and/or the liposome for determining lectin complement pathway activity C) comprise(s) a classical complement pathway inhibitor, preferably an anti-C1q85 antibody or an antigen-binding fragment thereof;

wherein, preferably, said collection comprises A) and B), A) and C), B) and C), or A), B), and C).

In one embodiment, the liposome collection is used for a method of determining complement activity, as defined herein.

In a further aspect, the present invention relates to a kit for determining complement activity, comprising

- a liposome collection as defined herein,
- a buffer, preferably a liposome complement buffer;
- optionally, a classical complement pathway inhibitor, an alternative complement pathway inhibitor, and/or a lectin complement pathway inhibitor; preferably EGTA, C1-inhibitor, an anti-MASP antibody, and/or C4BP; more preferably EGTA and/or a C1-inhibitor;
- optionally, an inactivated serum; wherein, preferably, said serum is inactivated by EDTA, EGTA, heat, ultrasound, and/or a solvent such as a water-soluble organic solvent;
- optionally, a surfactant, preferably n-octyl-β-d-glucoside;
- optionally, an osmolality agent, preferably a sucrose solution;
- optionally, instructions for determining complement activity, preferably instructions for determining classical complement pathway activity, alternative complement pathway activity, and/or lectin complement pathway activity; more preferably instructions for a method of determining complement activity as defined herein.

In one embodiment, the kit is used for a method of determining complement activity, as defined herein.

In a further aspect, the present invention relates to a liposome collection, as defined herein, or to a kit, as defined herein, for use in a method of diagnosing, monitoring, or treating a complement associated disorder, preferably a complement associated disease; wherein, optionally, said complement associated disease is selected from eye diseases, sepsis, kidney diseases, infectious diseases, primary deficiencies in complement genes, and autoimmune diseases, preferably selected from systemic lupus erythematosus, rheumatoid arthritis, hereditary angioedema, meningococcal disease, a disease associated with anti-factor H antibodies, recurrent pyogenic infections, haemolytic uremic syndrome, paroxysmal nocturnal hemoglobinuria, neuromyelitis optica disorder diseases, atypical hemolytic uremic syndrome, and C3 glomerulopathy.

In one embodiment, said use in a method of diagnosing, monitoring, or treating a complement associated disorder comprises performing a method of determining complement activity as defined herein.

In a further aspect, the present invention relates to a method of diagnosing, monitoring, or treating a complement associated disorder, preferably a complement associated disease, comprising administering a liposome collection, as defined herein, or a kit, as defined herein, to a patient in need thereof and/or to a sample of a patient in need thereof.

In one embodiment, said complement associated disease is selected from eye diseases, sepsis, kidney diseases, infectious diseases, primary deficiencies in complement genes, and autoimmune diseases, preferably selected from systemic lupus erythematosus, rheumatoid arthritis, hereditary angioedema, meningococcal disease, a disease associated with anti-factor H antibodies, recurrent pyogenic infections, haemolytic uremic syndrome, paroxysmal nocturnal hemoglobinuria, neuromyelitis optica disorder diseases, atypical hemolytic uremic syndrome, and C3 glomerulopathy.

In one embodiment, said administering comprises administering an effective amount of a liposome collection, as defined herein, and/or of a kit, as defined herein, to a patient in need thereof and/or to a sample of a patient in need thereof.

In one embodiment, said method of diagnosing, monitoring, or treating a complement associated disorder comprises performing a method of determining complement activity as defined herein.

In a further aspect, the present invention relates to a use of a liposome collection, as defined herein, or of a kit, as defined herein, for the manufacture of a medicament, preferably of a medicament for diagnosing, monitoring, or treating a complement associated disorder, preferably a complement associated disease as defined herein.

## DETAILED DESCRIPTION

An aim of the present invention is to provide efficient complement assays such as efficient methods of determining complement activity. For example, an aim of the invention is to provide a standardized, inexpensive, fast and high-throughput assay for the complement pathways. A further aim of the invention is to provide means for specifically determining the activity of each of the three complement pathways.

The present invention relates to a method of determining complement activity, wherein said method comprises A) determining classical complement pathway activity, B) determining alternative complement pathway activity, and/or C) determining lectin complement pathway activity. In one embodiment, the method of determining complement activity comprises determining total complement activity, particularly determining classical complement pathway activity, alternative complement pathway activity, and lectin complement pathway activity. In one embodiment, total complement activity comprises or consists of complement activity of all three complement pathways, particularly of the classical complement pathway, the of alternative complement pathway, and of the lectin complement pathway. In one embodiment, the method of determining complement activity is an *in vitro* and/or *ex vivo* method of determining complement activity.

In one embodiment, said determining classical complement pathway activity comprises providing one or more liposomes comprising a marker; optionally a liposome for determining classical complement pathway activity, as defined herein, particularly a liposome for determining classical complement pathway activity of a liposome collection, as defined herein.

In one embodiment, said one or more liposomes provided in step A)a), particularly one or more liposomes for determining classical complement pathway activity, comprise cholesterol, preferably at least 30 mol% cholesterol, more preferably at least 40 mol% cholesterol, even more preferably cholesterol in an amount of about 40 mol% to about 60 mol% e.g. of about 40 mol% to about 50 mol% or of about 40 mol% to about 45 mol%. The inventors have found that, advantageously, liposomes comprising at least 30 mol% cholesterol, particularly liposomes comprising 40 mol% cholesterol or more, activate the classical complement pathway. Thus, advantageously, liposomes comprising at least 30 mol% cholesterol, particularly liposomes comprising 40 mol% cholesterol or more, may be used to determine classical complement pathway activity. Alternatively or additionally, to activate the classical complement pathway, the one or more liposomes provided in step A)a), particularly one or more liposomes for determining classical complement pathway activity, may comprise a classical complement pathway activating agent. In one embodiment, said one or more liposomes provided in step A)a), particularly one or more liposomes for determining classical complement pathway activity, comprise a classical complement pathway activating agent, preferably selected from antibodies, antigens, and ligands such as biotin or streptavidin. In one embodiment, the one or more liposomes provided in step A)a) are configured to activate the classical complement pathway by comprising at least 30 mol% cholesterol, preferably at least 40 mol% cholesterol, and/or by comprising a classical complement pathway activating agent. In one embodiment, if the one or more liposomes provided in step A)a) comprise less than 30 mol% cholesterol, particularly less than 40 mol% cholesterol, the one or more liposomes comprise a classical complement pathway activating agent. Advantageously, the one or more liposomes provided in step A)a) may be configured to activate the classical complement pathway by a suitable cholesterol content, preferably at least 40 mol% cholesterol, and/or by a classical complement pathway activating agent. The inventors have found that, advantageously, liposomes comprising at least 30 mol% cholesterol, particularly liposomes comprising 40 mol% cholesterol or more, and/or comprising a classical complement pathway activating agent, allow to specifically determine classical complement activity.

In one embodiment, the liposome for determining classical complement pathway activity of the liposome collection of the invention is a liposome as defined provided in step A)a) of the method of determining complement activity of the invention. In one embodiment, said one or more liposomes provided in step A)a) comprise cholesterol, e.g. at least 30 mol% cholesterol, and phospholipids; preferably comprise cholesterol, 1,2-dipalmitoyl-sn-glycero-3-phosphocholine, and 1,2-dipalmitoyl-sn-glycero-3-phospho-(1'-rac-glycerol). In one embodiment, said one or more liposomes provided in step A)a) comprise at least 30 mol% cholesterol, preferably at least 40 mol% cholesterol, 1,2-dipalmitoyl-sn-glycero-3-phosphocholine in the range of from 10 mol% to 30 mol%, and 1,2-dipalmitoyl-sn-glycero-3-phospho-(i'-rac-glycerol) in the range of from 25 mol% to 45 mol%. In one embodiment, said one or more liposomes provided in step A)a) comprise 1,2-dipalmitoyl-sn-glycero-3-ethylphosphocholine.

In one embodiment, said determining alternative complement pathway activity comprises providing one or more liposomes comprising a marker; optionally a liposome for determining alternative complement pathway activity, as defined herein, particularly a liposome for determining alternative complement pathway activity of a liposome collection, as defined herein.

In one embodiment, said one or more liposomes provided in step B)a), particularly one or more liposomes for determining alternative complement pathway activity, comprise an alternative complement pathway activating agent, preferably a lipopolysaccharide, more preferably about 0.01 mol% to about 5 mol% lipopolysaccharide, even more preferably about 1 mol% lipopolysaccharide. Advantageously, liposomes comprising an alternative complement pathway activating agent allow to specifically determine alternative complement pathway activity, particularly in combination with a classical complement pathway inhibitor and a lectin complement pathway inhibitor. In one embodiment, said one or more liposomes provided in step B)a), particularly one or more liposomes for determining alternative complement pathway activity, comprise cholesterol, preferably ≤ 10 mol% cholesterol. The inventors have found that an advantageous stability of the liposomes can be achieved with cholesterol. Particularly, the inventors have found that, advantageously, liposomes comprising ≤ 10 mol% cholesterol have a high stability and abstain from specifically activating the classical complement pathway. In one embodiment, said lipopolysaccharide is present in said liposome in a range of from about 0.001% by weight to about 10 % by weight, preferably of from about 0.01 % by weight to about 2 % by weight, more preferably of about 1 % by weight.

In one embodiment, the liposome for determining alternative complement pathway activity of the liposome collection of the invention is a liposome as defined provided in step B)a) of the method of determining complement activity of the invention. In one embodiment, said one or more liposomes provided in step B)a) comprise phospholipids and cholesterol, preferably ≤ 10 mol% cholesterol; preferably comprise 1,2-dipalmitoyl-sn-glycero-3-phosphocholine, 1,2-dipalmitoyl-sn-glycero-3-phospho-(1'-rac-glycerol), and ≤ 10 mol% cholesterol. In one embodiment, said one or more liposomes provided in step B)a) comprise 1,2-dipalmitoyl-sn-glycero-3-phosphocholine in the range of from 65 mol% to 85 mol%, 1,2-dipalmitoyl-sn-glycero-3-phospho-(1'-rac-glycerol) in the range of from 10 mol% to 30 mol%, and ≤ 10 mol% cholesterol. In one embodiment, said one or more liposomes provided in step B)a) comprise a classical complement pathway inhibitor, preferably an anti-C1q85 antibody or an antigen-binding fragment thereof. In one embodiment, said one or more liposomes provided in step B)a) comprise 1,2-dipalmitoyl-sn-glycero-3-ethylphosphocholine.

In one embodiment, said determining lectin complement pathway activity comprises providing one or more liposomes comprising a marker; optionally a liposome for determining lectin complement pathway activity, as defined herein, particularly a liposome for determining lectin complement pathway activity of a liposome collection, as defined herein.

In one embodiment, said one or more liposomes provided in step C)a), particularly one or more liposomes for determining lectin complement pathway activity, comprise a lectin complement pathway activating agent, preferably comprising a sugar moiety, an acetyl moiety, and/or sialic acid. In one embodiment, a lectin complement pathway activating agent comprises or consists of a sugar moiety, an acetyl moiety, and/or sialic acid.

Advantageously, liposomes comprising a lectin complement pathway activating agent allow to specifically determine lectin complement pathway activity, particularly in combination with a classical complement pathway inhibitor and/or an alternative complement pathway inhibitor. In one embodiment, said one or more liposomes provided in step C)a), particularly one or more liposomes for determining lectin complement pathway activity, comprise cholesterol, preferably ≤ 10 mol% cholesterol. The inventors have found that an advantageous stability of the liposomes can be achieved with cholesterol. Particularly, the inventors have found that, advantageously, liposomes comprising ≤ 10 mol% cholesterol have a high stability and abstain from specifically activating the classical complement pathway.

In one embodiment, the liposome for determining lectin complement pathway activity of the liposome collection of the invention is a liposome as defined provided in step C)a) of the method of determining complement activity of the invention. In one embodiment, said one or more liposomes provided in step C)a) comprise phospholipids and ≤ 10 mol% cholesterol; preferably comprise 1,2-dipalmitoyl-sn-glycero-3-phosphocholine, 1,2-dipalmitoyl-sn-glycero-3-phospho-(1'-rac-glycerol), and ≤ 10 mol% cholesterol. In one embodiment, said one or more liposomes provided in step C)a) comprise 1,2-dipalmitoyl-sn-glycero-3-phosphocholine in the range of from 65 mol% to 85 mol%, 1,2-dipalmitoyl-sn-glycero-3-phospho-(i'-rac-glycerol) in the range of from 10 mol% to 30 mol%, and ≤ 10 mol% cholesterol. In one embodiment, said one or more liposomes provided in step C)a) comprise a classical complement pathway inhibitor, preferably an anti-C1q85 antibody or an antigen-binding fragment thereof. In one embodiment, said one or more liposomes provided in step C)a) comprise 1,2-dipalmitoyl-sn-glycero-3-ethylphosphocholine.

In one embodiment, said one or more liposomes provided in step A)a), said one or more liposomes provided in step B)a), and/or said one or more liposomes provided in step C)a) are selected from anionic liposomes, PEGylated liposomes, and cationic liposomes, preferably anionic liposomes. In one embodiment, a cationic liposome comprises 1,2-dipalmitoyl-sn-glycero-3-ethylphosphocholine. In one embodiment, said one or more liposomes provided in step A)a), step B)a), and/or step C)a) comprise or consist of anionic liposomes. In one embodiment, said one or more liposomes provided in step A)a), step B)a), and/or step C)a) comprise or consist of cationic liposomes, preferably comprising 1,2-dipalmitoyl-sn-glycero-3-ethylphosphocholine. For example, cationic liposomes may comprise cholesterol; 1,2-dipalmitoyl-sn-glycero-3-phosphocholine, preferably in the range of from 65 mol% to 85 mol%, e.g. about 76 %; and 1,2-dipalmitoyl-sn-glycero-3-ethylphosphocholine, preferably in the range of from 10 mol% to 30 mol%. In one embodiment, said PEGylated liposomes are cationic liposomes, anionic liposomes, neutral liposomes, and/or zwitterionic liposomes. In one embodiment, said one or more liposomes provided in step A)a), said one or more liposomes provided in step B)a), and/or said one or more liposomes provided in step C)a) comprise 1,2-dipalmitoyl-sn-glycero-3-phosphoethanolamine (DPPE) and/or 1,2-dimyristoyl-sn-glycero-3-phosphoethanolamine (DMPE). In one embodiment, said one or more liposomes provided in step A)a), said one or more liposomes provided in step B)a), and/or said one or more liposomes provided in step C)a) comprise a lipid with a functional group, preferably a functional group selected from an azide group, a maleimide group, and a SH group.

In one embodiment, said one or more liposomes provided in step A)a), step B)a), and/or step C)a) comprise a glutaryl moiety. Advantageously, a glutaryl moiety may be used to covalently attach further components, such as ligands, to said liposome.

In one embodiment, said one or more liposomes provided in step A)a), step B)a), and/or step C)a) of the method of the invention, and/or the liposome(s) A), B), and/or C) of the liposome collection of the invention, comprise(s) a complement activating agent. The term "complement activating agent", as used herein (e.g. in the context of liposome(s) provided in a method of the invention or in the context of liposome(s) of a liposome collection), relates to an agent e.g. to a molecule that activates the complement system, particularly an agent e.g. a molecule that activates at least one component and/or at least one pathway of the complement system.

In one embodiment, said one or more liposomes provided in step A)a) of the method of the invention, and/or the liposome A) of the liposome collection of the invention, comprise(s) a classical complement pathway activating agent. The term "classical complement pathway activating agent", as used herein (e.g. in the context of liposome(s) provided in a method of the invention or in the context of liposome(s) of a liposome collection), relates to an agent e.g. to a molecule that activates the classical complement pathway, particularly an agent e.g. a molecule that activates at least one component of the classical complement pathway, optionally relates to FITC and/or digoxygenin. In one embodiment, the classical complement pathway activating agent is selected from antibodies, antigens, and ligands such as biotin or streptavidin.

In one embodiment, said one or more liposomes provided in step B)a) of the method of the invention, and/or the liposome B) of the liposome collection of the invention, comprise(s) an alternative complement pathway activating agent. The term "alternative complement pathway activating agent", as used herein (e.g. in the context of liposome(s) provided in a method of the invention or in the context of liposome(s) of a liposome collection), relates to an agent e.g. to a molecule that activates the alternative complement pathway, particularly an agent e.g. a molecule that activates at least one component of the alternative complement pathway. In one embodiment, the alternative complement pathway activating agent is selected from lipopolysaccharides.

In one embodiment, said one or more liposomes provided in step C)a) of the method of the invention, and/or the liposome C) of the liposome collection of the invention, comprise(s) a lectin complement pathway activating agent. The term "lectin complement pathway activating agent", as used herein (e.g. in the context of liposome(s) provided in a method of the invention or in the context of liposome(s) of a liposome collection), relates to an agent e.g. to a molecule that activates the lectin complement pathway, particularly an agent e.g. a molecule that activates at least one component of the lectin complement pathway. In one embodiment, the lectin complement pathway activating agent is selected from a sugar moiety, an acetyl moiety and/or sialic acid.

In one embodiment, said determining classical complement pathway activity comprises the step of contacting said one or more liposomes and/or said sample with a classical complement pathway activating agent, for example a classical complement pathway activating agent selected from antibodies such as anti-biotin antibodies, antigens, and ligands such as biotin or streptavidin. In one embodiment, said determining alternative complement pathway activity comprises the step of contacting said one or more liposomes and/or said sample with an alternative complement pathway activating agent. In one embodiment, said determining lectin complement pathway activity comprises the step of contacting said one or more liposomes and/or said sample with a lectin complement pathway activating agent. For example, said activating agents may be added to a solution, such as a buffer, comprising said sample and/or said liposomes.

In one embodiment, said determining classical complement pathway activity comprises the step of contacting said sample and/or said one or more liposomes with an alternative complement pathway inhibitor and/or a lectin complement pathway inhibitor. In one embodiment, said determining alternative complement pathway activity comprises the step of contacting said sample and/or said one or more liposomes with a classical complement pathway inhibitor, and/or a lectin complement pathway inhibitor. In one embodiment, said determining lectin complement pathway activity comprises the step of contacting said sample and/or said one or more liposomes with an alternative complement pathway inhibitor and/or a classical complement pathway inhibitor. For example, said inhibitors may be added to a solution, such as a buffer, comprising said sample and/or said liposomes.

The term "sample", as used herein, relates to a sample to be tested, preferably a sample of a patient. In one embodiment, the sample is selected from a blood sample such as a serum sample, a urine sample, a tear(s) sample, an aqueous humor sample, a nasal swab sample, a synovial fluid sample, a pleural fluid sample, a peritoneal fluid sample, a cerebrospinal fluid sample, an exudate sample, a tissue fluid sample, a cell culture supernatant, and any combination thereof; preferably the sample is a serum sample. In one embodiment, said sample is a sample obtained from a patient and/or a sample of a patient, preferably a serum sample. In one embodiment, said sample of a patient is a serum sample, optionally a diluted serum sample. In one embodiment, the sample is a sample containing or suspected of containing one or more active complement system components, such as one or more active components of the classical complement pathway, one or more active components of the alternative complement pathway, and/or one or more active components of the lectin complement pathway.

In one embodiment, the contacting said one or more liposomes with said sample in steps A)b), B)c), and/or C)c) comprises incubating said one or more liposomes with said sample. In one embodiment, said contacting, particularly incubating, is performed at a temperature in the range of from about 4°C to about 50°C, preferably of from about 20°C to about 40°C, more preferably of from about 30°C to about 39°C, e.g. of about 37°C. In one embodiment, said contacting, particularly incubating, is performed for about 1 sec to about 120 min, preferably for about 5 min to about 90 min, more preferably for about 15 min to about 60 min.

In one embodiment, said contacting is configured such that the one or more active complement system components, if present in said sample, are allowed to lyse said one or more liposomes. In one embodiment, "active" complement system components are fully functional complement system components, preferably having the same functionality as the respective complement system component of healthy individuals and/or having the physiological functionality of the respective complement system component. In one embodiment, the terms "active complement system component", "fully functional complement system component", and "physiological complement system components" are used interchangeably. In one embodiment, a lysis of said one or more liposomes, particularly a lysis of said liposomes triggered by active complement system components, releases said marker from said one or more liposomes. In one embodiment, the term "active" in the context of the complement system or complement pathways, relates to functional complement, e.g. physiological complement.

In one embodiment, the detecting said released marker in steps A)c), B)d), and/or C)d) comprises any of fluorescent, colorimetric, chemiluminescent, electrochemiluminescent, electrochemical, bioluminescent, and/or visual detection of said marker, optionally detection using a cell phone. In one embodiment, the term "detecting said released marker", as used herein, comprises a direct and/or indirect detection of said released marker, particularly a direct and/or indirect detection of a marker signal, for example using any of fluorescent, colorimetric, chemiluminescent, electrochemiluminescent, electrochemical, bioluminescent, and/or visual detection, optionally detection using a cell phone. For example, a signal of a marker can be detected via i) direct detection, e.g. of a fluorescent or bioluminescent signal, via ii) prior immobilization of liposomes on a surface and subsequent detection, and/or via iii) prior collecting liposomes from a solution and subsequent detection. An advantage of the method of the invention is that it can be performed outside of a central testing lab, such as in a medical doctor’s office. Furthermore, no expensive equipment is needed. In one embodiment, the method of the invention is performed as an onsite assay and/or a point-of-care assay.

In one embodiment, the detecting said released marker in steps A)c), B)d), and/or C)d) comprises detecting an absence or presence of released marker, wherein, preferably, an absence of released marker indicates complement inactivity and a presence of released marker indicates complement activity. In one embodiment, the method of determining complement activity comprises determining complement activity, if a presence of released marker is detected. In one embodiment, the method of determining complement activity comprises determining complement inactivity, if released marker is not detected and/or if an absence of released marker is detected.

In one embodiment, classical complement pathway activity is determined, if a presence of released marker is detected in step A)c), and/or classical complement pathway inactivity is determined, if an absence of released marker is detected in step A)c). In one embodiment, alternative complement pathway activity is determined, if a presence of released marker is detected in step B)d), and/or alternative complement pathway inactivity is determined, if an absence of released marker is detected in step B)d). In one embodiment, lectin complement pathway activity is determined, if a presence of released marker is detected in step C)d), and/or lectin complement pathway inactivity is determined, if an absence of released marker is detected in step C)d).

In one embodiment, the detecting said released marker in steps A)c), B)d), and/or C)d) comprises directly or indirectly detecting said released marker. In one embodiment, indirectly detecting said released marker comprises providing said one or more liposomes comprising a marker, e.g. a chemiluminescent marker, in the form of immobilized liposome(s), and detecting the marker comprised by said immobilized liposome(s) after removing said released marker such as by a washing step. In one embodiment, liposomes are immobilized; for example, via biotin on liposomes and streptavidin on a surface, or via digoxygenin on liposomes and an anti-dig antibody on a surface, or via FITC on liposomes and anti-FITC immobilized on a surface. In one embodiment, said method comprises a step of immobilizing said one or more liposomes provided in steps A)a), B)a), and/or C)a). In one embodiment, the detecting said released marker in steps A)c), B)d), and/or C)d) comprises detecting an absence or a presence of released marker. In one embodiment, the detecting said released marker in steps A)c), B)d), and/or C)d) comprises detecting an absence of released marker, said absence preferably indicating the presence of nonlysed liposomes, and/or comprises detecting a presence of released marker, said presence preferably indicating the presence of lysed liposomes. In one embodiment, the method comprises a step of separating nonlysed liposomes, such as immobilized nonlysed liposomes, from lysed liposomes. In one embodiment, the method of the invention comprises a step of separating lysed liposomes and nonlysed liposomes, preferably by a lateral-flow assay, filtration, chromatography, and/or centrifugation. In one embodiment, said method comprises detecting said released marker and detecting the marker comprised by nonlysed liposomes. In one embodiment, detecting said released marker in steps A)c), B)d), and/or C)d) comprises detecting liposomes that are intact, particularly nonlysed liposomes, preferably by detecting marker comprised by said liposomes that are intact. In one embodiment, said method comprises a step of detecting the marker comprised by nonlysed liposome(s). In one embodiment, said method comprises a step of detecting the marker present in nonlysed liposome(s), such as an electrochemical marker, a colorimetric marker, a chemiluminescent marker, and/or an electrochemiluminescent marker.

In one embodiment, said A) determining classical complement pathway activity, B) determining alternative complement pathway activity, and/or C) determining lectin complement pathway activity comprise(s) a step of detecting a marker comprised by nonlysed liposomes. For example, in the absence of complement activity, said contacting said one or more liposomes with said sample in step A)b), step B)c), and/or step C)c) does not result in a lysis of said one or more liposomes, i.e. the liposomes remain nonlysed liposomes. In one embodiment, the one or more liposomes provided in step A)a), step B)a), and/or step C)a) are nonlysed liposomes. In one embodiment, the marker comprised by nonlysed liposomes is detected. The inventors have found that, advantageously, by the specific interaction of the classical complement with the nonlysed liposomes provided in step A)a), the specific interaction of the alternative complement with the nonlysed liposomes provided in step B)a), and/or the specific interaction of the lectin complement with the nonlysed liposomes provided in step C)a), the respective liposomes are lysed and the marker is released. In one embodiment, said one or more liposomes contacted with said sample in step A)b), step B)c), and/or step C)c) remain nonlysed liposomes in the absence of complement activity.

In one embodiment, said step of detecting a marker comprised by nonlysed liposomes comprises a direct detection, e.g. by measuring a signal of said marker comprised by said nonlysed liposomes, and/or comprises an indirect detection, e.g. by first removing lysed liposomes and/or released marker and then subsequently lysing the nonlysed liposomes and detecting the released marker of said liposomes. In one embodiment, said A) determining classical complement pathway activity, B) determining alternative complement pathway activity, and/or C) determining lectin complement pathway activity comprise(s) a washing step in which lysed liposomes and/or released marker is removed by washing, preferably prior to a step of detecting a marker comprised by nonlysed liposomes. In one embodiment, the detecting said released marker in steps A)c), B)d), and/or C)d) comprises detecting said released marker and detecting a marker comprised by nonlysed liposomes, e.g. by direct detection of the marker comprised by nonlysed liposomes. In one embodiment, said A) determining classical complement pathway activity, B) determining alternative complement pathway activity, and/or C) determining lectin complement pathway activity comprise(s) a step of detecting a marker comprised by nonlysed liposomes, wherein, prior to said detection of marker comprised by nonlysed liposomes, lysed liposomes and/or released marker is removed e.g. washed out, and then the nonlysed liposomes are lysed to release the marker and the released marker is detected.

In one embodiment, said determining alternative complement pathway activity comprises the step of contacting the sample and/or said one or more liposomes with a classical complement pathway inhibitor and a lectin complement pathway inhibitor. In one embodiment, the classical complement pathway inhibitor and the lectin complement pathway inhibitor are the same inhibitor or different inhibitors. In one embodiment, the classical complement pathway inhibitor and the lectin complement pathway inhibitor comprise or consist of the same compound or different compounds. For example, a compound may inhibit both the classical complement pathway and at the lectin complement pathway, e.g. EGTA. In one embodiment, an inhibitor inhibiting the classical complement pathway and the lectin complement pathway is selected from EGTA, C1-inhibitor, and C4BP. In one embodiment, an inhibitor being a classical complement pathway inhibitor and a lectin complement pathway inhibitor is selected from EGTA, C1-inhibitor, and C4BP. In one embodiment, the classical complement pathway inhibitor, particularly the classical complement pathway inhibitor used in determining alternative complement pathway activity, comprises EGTA, an anti-C1q85 antibody or an antigen-binding fragment thereof, C1-inhibitor, and/or C4BP, preferably EGTA and/or a C1-inhibitor. In one embodiment, a specific classical complement pathway inhibitor, particularly a classical complement pathway inhibitor which inhibits only the classical complement pathway, is an anti-C1q85 antibody or an antigen-binding fragment thereof. In one embodiment, the lectin complement pathway inhibitor comprises EGTA, C1-inhibitor, an anti-MASP antibody, and/or C4BP, preferably EGTA and/or a C1-inhibitor. In one embodiment, a specific lectin complement pathway inhibitor, particularly a lectin complement pathway inhibitor which inhibits only the lectin complement pathway, is an anti-MASP antibody.

In a preferred embodiment, the classical complement pathway inhibitor and the lectin complement pathway inhibitor comprise or consist of EGTA. An advantage of EGTA is that it efficiently inhibits both the classical complement pathway and the lectin complement pathway. Furthermore, EGTA is a low cost inhibitor of both the classical complement pathway and the lectin complement pathway. Advantageously, the activity of the alternative complement pathway can be specifically determined when the classical pathway and the lectin pathway are inhibited by a classical complement pathway inhibitor and a lectin complement pathway inhibitor. The alternative pathway specificity of the method of determining complement activity of the present invention is highly increased when the classical pathway and the lectin pathway are inhibited by a classical complement pathway inhibitor and a lectin complement pathway inhibitor, such as EGTA.

In one embodiment, the term "classical complement pathway inhibitor", as used herein (e.g. in the context of liposome(s) provided in a method of the invention or in the context of liposome(s) of a liposome collection), relates to any inhibitor of the classical complement pathway, such as an inhibitor selected from EGTA, an anti-C1q85 antibody or antigen-binding fragment thereof, C1-inhibitor, C4BP, and combinations thereof. In one embodiment, particularly when used in the context of determining lectin complement pathway activity, the classical complement pathway inhibitor is a specific classical complement pathway inhibitor, preferably selected from an anti-C1q85 antibody or an antigen-binding fragment thereof. In one embodiment, the term "lectin complement pathway inhibitor", as used herein (e.g. in the context of liposome(s) provided in a method of the invention or in the context of liposome(s) of a liposome collection), relates to any inhibitor of the lectin complement pathway, such as an inhibitor selected from EGTA, C1-inhibitor, an anti-MASP antibody, C4BP, and combinations thereof. In one embodiment, particularly when used in a context of determining classical complement pathway activity, the lectin complement pathway inhibitor is a specific lectin complement pathway inhibitor. When referring to an antibody in the context of the present invention, for example to inhibitors or activating agents comprising a consisting of an antibody, the reference to such an antibody is meant to be understood as also relating to fragments thereof, particularly antigen binding fragments thereof. In one embodiment, the term "alternative complement pathway inhibitor", as used herein (e.g. in the context of liposome(s) provided in a method of the invention or in the context of liposome(s) of a liposome collection), relates to any inhibitor of the alternative complement pathway, such as an inhibitor selected from complement factor H, mini-complement factor H, an anti-factor B antibody or antigen-binding fragment thereof, an anti-properdin antibody or antigen-binding fragment thereof, and any combination thereof. In one embodiment, each of the classical complement pathway inhibitor, the lectin complement pathway inhibitor, and the alternative complement pathway inhibitor is a specific inhibitor. In one embodiment, the anti-MASP antibody is a human monoclonal antibody targeting mannan-binding lectin-associated serine protease-2; e.g. Narsoplimab. The term "C4BP", as used herein, refers to C4b-binding protein.

In one embodiment, step B)b) comprises contacting said sample and/or said one or more liposomes with a classical complement pathway inhibitor and/or a lectin complement pathway inhibitor selected from $Ca^{2+}$ complexing agents, preferably EGTA, in the presence of $Mg^{2+}$, preferably in the presence of at least 0.001 mM MgCl2, more preferably in the presence of at least 0.1 mM MgCl2, even more preferably in the presence of at least 0.5 mM MgCl2, e.g. of about 1 mM MgCl2. In one embodiment, said EGTA and said Mg have a molar ratio of about 1:1. In one embodiment, said determining alternative complement pathway activity comprises the step of contacting the sample and/or said one or more liposomes with a classical complement pathway inhibitor and a lectin complement pathway inhibitor, preferably EGTA, in the presence of $Mg^{2+}$, preferably in the presence of at least 0.001 mM MgCl2, more preferably in the presence of at least 0.1 mM MgCl2, even more preferably in the presence of at least 0.5 mM MgCl2, e.g. of about 1 mM MgCl2. When using a classical complement pathway inhibitor and a lectin complement pathway inhibitor, lysis of the liposomes occurs merely based on alternative complement pathway activity. In a preferred embodiment, EGTA is provided in an amount of at least 0.2 mM. In one embodiment, EGTA is provided in an amount of at least 0.2 mM, preferably of at least 0.4 mM, preferably in combination with 1 mM MgCl2. In one embodiment, the classical complement pathway inhibitor and/or the lectin complement pathway inhibitor is EGTA at a concentration of at least 0.2 mM, preferably of at least 0.4 mM. In one embodiment, the classical complement pathway inhibitor and/or the lectin complement pathway inhibitor is EGTA at a concentration in a range of from about 0.2 mM to about 1 mM, preferably of from about 0.2 to about 0.8 mM, optionally being provided in combination with 1 mM $Mg^{2+}$. In one embodiment, step B)b) comprises contacting said sample and/or said one or more liposomes with a $Ca^{2+}$ complexing agent, optionally EGTA, preferably in an amount of at least 0.2 mM, preferably at least 0.4 mM; in the presence of $Mg^{2+}$, preferably in the presence of at least 0.001 mM MgCl2, more preferably in the presence of at least 0.1 mM MgCl2, even more preferably in the presence of at least 0.5 mM MgCl2, e.g. of about 1 mM MgCl2.

In one embodiment, said determining lectin complement pathway activity comprises the step of contacting said sample and/or said one or more liposomes with a classical complement pathway inhibitor and/or an alternative complement pathway inhibitor. In one embodiment, the classical complement pathway inhibitor and the alternative complement pathway inhibitor are the same inhibitor or different inhibitors. In one embodiment, the classical complement pathway inhibitor and the alternative complement pathway inhibitor comprise or consist of the same compound or different compounds. In one embodiment, the classical complement pathway inhibitor, particularly the classical complement pathway inhibitor used in determining lectin complement pathway activity, comprises or consists of an anti-C1q85 antibody or an antigen-binding peptide thereof. In one embodiment, the alternative complement pathway inhibitor is selected from complement factor H, mini-complement factor H, an anti-factor B antibody or antigen-binding fragment thereof, an anti-properdin antibody or antigen-binding fragment thereof, and any combination thereof. Advantageously, when inhibiting the classical complement pathway and/or the alternative complement pathway with a classical complement pathway inhibitor and/or an alternative complement pathway inhibitor, respectively, the lectin pathway specificity is very high.

Each of said determining classical complement pathway activity, determining alternative complement pathway activity, and determining lectin complement pathway activity comprises contacting said one or more liposomes with said sample to allow the one or more active complement system components, if present in said sample, to lyse said one or more liposomes. Particularly, when determining classical complement pathway activity, the one or more active complement system components of the classical complement pathway, if present in said sample, lyse said one or more liposomes, particularly said liposome(s) for determining classical complement pathway activity. Particularly, when determining alternative complement pathway activity, the one or more active complement system components of the alternative complement pathway, if present in said sample, lyse said one or more liposomes, particularly said liposome(s) for determining alternative complement pathway activity. Particularly, when determining lectin complement pathway activity, the one or more active complement system components of the lectin complement pathway, if present in said sample, lyse said one or more liposomes, particularly said liposome(s) for determining lectin complement pathway activity. The lysis of the liposome(s) results in the release of the marker which can be detected. For example, by analyzing how much marker is released, the complement activity can be determined. The higher the level of released marker, the higher the complement activity. If the complement is active, the liposomes are lysed. Thus, if the complement is active, released marker is detected. For example, if the classical complement pathway is active, liposome(s) for determining classical complement pathway activity are lysed. For example, if the alternative complement pathway is active, liposome(s) for determining alternative complement pathway activity are lysed. For example, if the lectin complement pathway is active, liposome(s) for determining lectin complement pathway activity are lysed. In the absence of released marker and/or in the absence of liposome lysis, it can be determined that the complement is inactive.

In one embodiment, said A) determining classical complement pathway activity, B) determining alternative complement pathway activity, and/or C) determining lectin complement pathway activity further comprise(s) a step of detecting a base level of said marker. In one embodiment, said base level of said marker is detected prior to said step of contacting said one or more liposomes with said sample, at a time point in the range of from about 1 sec to about 20 min, preferably of from about 1 sec to about 15 min, after an initial contact of said one or more liposomes with said sample, and/or using an inactivated serum and/or a buffer. For example, the base level may comprise or consist of the marker level prior to complement activity, e.g. prior to said contacting or prior to said activity, and/or may comprise or consist of the marker level without complement activity, e.g. the marker level of a negative control comprising inactivated serum and/or buffer. In one embodiment, said detecting a base level comprises performing a negative control. For example, said determining a base level may comprise determining a marker level in a buffer, particularly in buffer without serum, determining a marker level in inactivated serum, and/or determining a marker level at a time point prior to complement activity. In one embodiment, said determining a base level is performed at a temperature in the range of from about 4°C to about 50°C, preferably of from about 20°C to about 40°C, more preferably of from about 30°C to about 39°C, e.g. of about 37°C; and/or is performed for about 1 sec to about 120 min, preferably for about 5 min to about 90 min, more preferably for about 15 min to about 60 min. Preferably, inactivated serum is serum in which the complement system has been inactivated, e.g. by EDTA, EGTA, heat, ultrasound, and/or a solvent such as a water-soluble organic solvent.

In one embodiment, the method of determining competitive activity comprises a step of determining complement activity by comparing the released marker detected in steps A)c), B)d), and C)d), respectively, with a base level of said marker, preferably the base level of said marker detected in said step of detecting a base level of said marker, and/or with a negative control. In one embodiment, a higher level of said released marker detected in steps A)c), B)d), and C)d), respectively, compared to said base level of said marker and/or to a negative control indicates complement activity. In one embodiment, the method of determining complement activity comprises determining complement activity if released marker is detected, particularly if released marker is detected in steps A)c), B)d), and/or C)d). In one embodiment, the method of determining complement activity comprises determining an absence of complement activity, particularly complement inactivity, if released marker is not detected, particularly if released marker is not detected in steps A)c), B)d), and/or C)d), and/or if a level of detected released marker corresponds to a level of released marker of the negative control.

In one embodiment, said released marker detected in steps A)c), B)d), and/or C)d) is compared to a reference signal and/or to a reference value. In one embodiment, said determining complement activity, particularly said determining classical complement pathway activity, determining alternative complement pathway activity, and/or determining lectin complement pathway activity, comprises comparing said released marker detected in steps A)c), B)d), and/or C)d) to a reference signal and/or to a reference value.

In one embodiment, the term "reference signal and/or reference value", as used herein, relates to a signal and/or to a value obtained for a sample having normal complement activity, preferably a sample of a healthy individual. In one embodiment, a method of determining complement activity comprises determining complement activity, if said released marker detected in steps A)c), B)d), and/or C)d), respectively, is equal to or higher than the reference signal and/or the reference value. In one embodiment, a method of determining complement activity comprises determining an absence of complement activity or decreased complement activity, if said released marker detected in steps A)c), B)d), and/or C)d), respectively, is decreased compared to a reference signal and/or to a reference value.

In one embodiment, said method comprises performing a positive control and/or a negative control. In one embodiment, performing a positive control comprises contacting said one or more liposomes provided in step A)a), B)a), and/or C)a) with a surfactant and/or solvent, preferably a surfactant, more preferably a surfactant selected from n-octyl-β-d-glucoside, 2-[4-(2,4,4-trimethylpentan-2-yl)phenoxy]ethanol, polyoxyethylene (20) sorbitan monolaurate, and polyoxyethylene (80) sorbitan monooleate. In one embodiment, the terms "2-[4-(2,4,4-trimethylpentan-2-yl)phenoxy]ethanol" and "Triton X-100" are used interchangeably. In one embodiment, the terms "polyoxyethylene (20) sorbitan monolaurate" and "Tween 20" are used interchangeably. In one embodiment, the terms "polyoxyethylene (80) sorbitan monooleate", "polyoxyethylene (20) sorbitan monooleate", and "Tween 80" are used interchangeably. In one embodiment, performing a negative control comprises replacing said sample, optionally said serum sample, in step A)a), B)a), and/or C)a) with a negative control sample which comprises or consists of inactive serum.

In one embodiment, the marker is quenched as long as said marker is comprised in the liposome. In one embodiment, if the complement is inactive in said sample, said liposome is not lysed, said marker is not released from said liposome, and thus, the signal of the marker comprised by the liposome remains quenched. In one embodiment, if the complement is active in said sample, said liposome is lysed, said marker is released, and thus released marker is detectable.

In one embodiment, said detecting in step A)c), step B)d), and/or step C)d) comprises or consists of a time-resolved detection of said released marker. A time-resolved detection is highly advantageous in that additional details of a complement associated disorder can be assessed, such as a lag in complement activity. In one embodiment, said detecting in step A)c), step B)d), and/or step C)d) comprises or consists of a time-resolved detection of said released marker, and an increase in said released marker over time indicates complement activity.

The term "marker", as used herein, such as used herein in the context of the one or more liposome(s) provided in a method of the invention or in the context of liposome(s) of a liposome collection of the invention, relates to a molecule that can be detected, preferably quantitatively analyzed, via any detection method and/or analysis method known to a person skilled in the art, e.g. via chromatography, electrophoresis, microscopy, photometry, spectroscopy, such as atomic absorption spectroscopy, ultraviolet-visible spectroscopy, x-ray spectroscopy, fluorescence spectroscopy, infrared spectroscopy, Raman spectroscopy, nuclear magnetic resonance spectroscopy, photoemission spectroscopy, mass spectrometry, electrochemical analysis, calorimetry, sequencing, precipitation, or extraction. In one embodiment, the marker is selected from fluorescent markers, preferably pyrenetetrasulfonic acid, sulforhodamine B, indocyanine green such as 2-[2-[2-chloro-3-[2-[1,3-dihydro-3,3-dimethyl-1-(4-sulfobutyl)-2H-indol-2-ylidene]ethylidene]-1-cyclohexen-1-yl]ethenyl]-3,3-dimethyl-1-(4-sulfobutyl)-3H-indolium, or carboxyfluorescein; electrochemical markers, preferably potassium hexaferricyanide, potassium hexaferrocyanide, or ruthenium hexamine; colorimetric markers, preferably sulforhodamine B; chemiluminescent markers, preferably luminol or its derivatives such as mCOOH-luminol; electrochemiluminescent markers, preferably luminol or ruthenium bipyridyl; bioluminescent markers such as GFP, other luminescent proteins, or luciferase; and combinations thereof. In one embodiment, the marker is water-soluble. For example, said marker may be selected from a water-soluble fluorescent marker, a water-soluble electrochemical marker, a water-soluble a chemiluminescent marker, and a water-soluble bioluminescent marker. In one embodiment, the marker is selected from a fluorescent marker, an electrochemical marker, and a combination thereof. In a preferred embodiment, the marker is selected from sulforhodamine B and mCOOH-luminol. In a preferred embodiment, said marker provides a signal only when released from said liposome. In an alternative embodiment, said marker provides a signal only when contained inside the liposome, and said signal dissipates when the marker is released from the liposome. In one embodiment, the term "luminol", as used herein, relates to luminol and its derivatives, particularly its water soluble derivatives such as mCOOH-luminol, preferably relates to luminol and mCOOH-luminol, more preferably relates to mCOOH-luminol.

In one embodiment, said one or more liposomes comprising a marker provided in step A)a) comprise a first marker, said one or more liposomes comprising a marker provided in step B)a) comprise a second marker, and said one or more liposomes comprising a marker provided in step C)a) comprise a third marker, wherein said first marker, said second marker, and said third marker are different markers. Advantageously, using a first marker, a second marker, and a third marker allows to specifically and simultaneously determine the activity of each of the three different complement pathways.

In one embodiment, said detecting said released marker of steps A)c), B)d), and/or C)d) is performed simultaneously or sequentially, preferably simultaneously, e.g. by performing said determining classical complement pathway activity, said determining alternative complement pathway activity, and said determining lectin complement pathway activity in different wells of a multi-well plate. For example, said simultaneously detecting may be performed in separate containers, such as separate wells of a multi-well plate.

In one embodiment, the liposome collection and/or the kit of the invention is/are used in a method of the invention. In one embodiment, the method of the invention is performed using a liposome collection and/or a kit of the invention.

The term "liposome collection", as used herein, relates to a collection e.g. group of liposomes. In one embodiment, the terms "liposome collection" and "liposome assembly" can be used interchangeably. In one embodiment, a liposome collection comprises one or more types of liposomes, particularly one or more, preferably at least two, types of liposomes selected from a liposome for determining classical complement pathway activity, a liposome for determining alternative complement pathway activity, and a liposome for determining lectin complement pathway activity. For example, the liposome collection may comprise a liposome for determining classical complement pathway activity and a liposome for determining alternative complement pathway activity.

In one embodiment, the liposome collection of the invention comprises

- A) a liposome for determining classical complement pathway activity, as defined herein,
- B) a liposome for determining alternative complement pathway activity, as defined herein, and/or
- C) a liposome for determining lectin complement pathway activity, as defined herein;

wherein, optionally, said collection comprises A) and B), A) and C), B) and C), or A), B), and C).

In one embodiment, the liposome for determining classical complement pathway activity of the liposome collection is a liposome as defined in the context of step A)a) of a method of determining complement activity of the invention. In one embodiment, the liposome for determining alternative complement pathway activity of the liposome collection is a liposome as defined in the context of step B)a) of a method of determining complement activity of the invention. In one embodiment, the liposome for determining lectin complement pathway activity of the liposome collection is a liposome as defined in the context of step C)a) of a method of determining complement activity of the invention. It is meant to be understood that all embodiments defining the liposome(s) provided in steps A)a), B)a), and/or C)a) of the method of determining complement activity of the present invention also apply to liposomes A), B), and C), respectively, of the liposome collection of the present invention, vice versa, such as embodiments defining markers of the liposome(s) or components of the liposome(s). For example, all embodiments defining the liposome(s) provided in step A)a) of the method of the present invention also apply to liposome(s) A) of the liposome collection of the invention, vice versa. For example, all embodiments defining the liposome(s) provided in step B)a) of the method of the present invention also apply to liposome(s) B) of the liposome collection of the invention, vice versa. For example, all embodiments defining the liposome(s) provided in step C)a) of the method of the present invention also apply to liposome(s) C) of the liposome collection of the invention, vice versa.

In one embodiment, the liposome collection is used for a method of determining complement activity, as defined herein. The invention also relates to the use of a liposome collection, as defined herein, in a method of determining complement activity and/or in a method of diagnosing, monitoring, or treating a complement associated disorder.

In a further aspect, the present invention relates to a kit for determining complement activity, comprising

- a liposome collection, as defined herein,
- a buffer, preferably a liposome complement buffer;
- optionally, a classical complement pathway inhibitor, an alternative complement pathway inhibitor, and/or a lectin complement pathway inhibitor; preferably EGTA, C1-inhibitor, an anti-MASP antibody, and/or C4BP; more preferably EGTA and/or a C1-inhibitor;
- optionally, an inactivated serum; wherein, preferably, said serum is inactivated by EDTA, EGTA, heat, ultrasound, and/or a solvent such as a water-soluble organic solvent;
- optionally, a surfactant, e.g. a surfactant selected from n-octyl-β-d-glucoside, 2-[4-(2,4,4-trimethylpentan-2-yl)phenoxy]ethanol, polyoxyethylene (20) sorbitan monolaurate, and polyoxyethylene (80) sorbitan monooleate, preferably n-octyl-β-d-glucoside;
- optionally, an osmolality agent, preferably a sucrose solution;
- optionally, instructions for determining complement activity, preferably instructions for determining classical complement pathway activity, alternative complement pathway activity, and/or lectin complement pathway activity; more preferably instructions for a method of determining complement activity as defined herein.

In one embodiment, the kit further comprises a test strip and/or a microtiter plate. In one embodiment, the kit is used for a method of determining complement activity, as defined herein. The invention also relates to the use of a kit, as defined herein, in a method of determining complement activity and/or in a method of diagnosing, monitoring, or treating a complement associated disorder.

In one embodiment, the liposome complement buffer comprises 10 mM HEPES, 150 mM NaCl, 135 nM CaCl2, and 1 mM $MgCl_2$; preferably at about pH 7.4. In one embodiment, inactivated serum is a serum in which the complement system has been inactivated and/or in which one or more, preferably all, components of the complement system have been inactivated. For example, inactivated serum may be provided by activating a serum sample with EDTA, such as 200 mM EDTA, and/or EGTA, such as 0.5 μM EGTA.

In one embodiment, the osmolality agent is a sugar, salt, or polymer providing a certain osmolality for the liposomes. In one embodiment, the osmolality agent is sucrose. Advantageously, the osmolality agent stabilizes the liposomes. In one embodiment, the osmolality agent is configured such that an osmolality is provided that is equal to or higher than an osmolality of a liposome aqueous core, e.g. is about 50 - 75 mmol/kg higher than an osmolality of a liposome aqueous core. For example, the osmolality agent may be provided in buffer to be used for the liposomes.

In one embodiment, the liposome collection, as defined herein, and/or the kit, as defined herein, for use in a method of diagnosing, monitoring, or treating a complement associated disorder, preferably a complement associated disease, is/are used to monitor a complement associated disorder. For example, the liposome collection and/or the kit may be used to determine whether a pathological and/or an inactive complement system normalizes to a functional complement system, i.e. an active complement system, when a complement therapeutic agent is administered to a patient in need thereof. By monitoring complement activity, e.g. using a method of determining complement activity, a kit, and/or a liposome collection of the invention, the therapy of the patient in need thereof can be adjusted with respect to a required dose of complement therapeutic agent and/or with respect to a required duration of the treatment with a complement therapeutic agent. Advantageously, the method of determining complement activity, a kit, and/or a liposome collection of the invention may be used in the context of personalized medicine, for example by determining whether a patient is expected of being successfully treated with a respective complement therapeutic agent. The liposome collection and/or the kit, when used in a method of treating a complement associated disorder, may be used to predict whether a complement therapeutic agent is likely to be effective in a patient. For example, the liposome collection and/or the kit may be a part of a treatment of a complement associated disorder in a patient, wherein the liposome collection and/or the kit is used to determine in vivo and/or ex vivo, prior to the administration of a complement therapeutic agent to a patient, whether the complement therapeutic agent has an effect on the complement of said patient.

The present invention also relates to the liposome collection, as defined herein, and/or the kit, as defined herein, for use in a method of diagnosing a complement associated disorder, preferably a complement associated disease, comprising

i) providing a sample of a patient, preferably a serum sample;
ii) performing a method of determining complement activity, as defined herein, using the liposome collection, as defined herein, or the kit, as defined herein;
iii) diagnosing a complement associated disorder if an absence of released marker or a decreased level of released marker compared to a positive control and/or compared to a reference value is detected.

Advantageously, the method of the invention, e.g. liposome assay, is a homogeneous assay in which no washing steps are needed such as in heterogeneous assays. By incubation with bodily samples, complement activity will lead to liposome lysis, which in turn can be detected. Detection can be accomplished through a variety of different strategies including optical and electrochemical assays.

In contrast to the known CH50 liposome assay, which can detect only the classical pathway, the method of the invention has the advantage that the three complement pathways can be addressed separately. This becomes possible as liposomes are designed to have a specific lipid composition, specific encapsulant concentrations and/or specific ligands. For example, liposomes with a cholesterol content > 30 mol%, such as a 44 mol% cholesterol content, specifically trigger the classical pathway. Liposomes containing 5 mol% cholesterol and 1 mol% lipopolysaccharide may trigger all three pathways, but through the addition of C1-inhibitor or EGTA, only the alternative pathway is detected. For determining the lectin pathway, sugar moieties, Ac-BSA or sialic acid structures can be used. Advantageously, the method of the invention allows to specifically target one or more pathways of the complement system, e.g., the classical or alternative pathway of the complement system.

In a preferred embodiment, the liposomes provided in step A)a) of the method of the invention and/or liposome A) of the liposome collection of the invention comprise at least 30 mol% cholesterol, more preferably at least 40 mol% cholesterol, particularly to specifically trigger the classical complement pathway. Advantageously, the classical pathway can be specifically targeted without any activating agents, i.e. merely by the liposomes comprising a high amount of cholesterol such as at least 30 mol% cholesterol, particularly at least 40 mol% cholesterol.

In a preferred embodiment, the liposomes provided in step B)a) of the method of the invention and/or liposome B) of the liposome collection of the invention comprise 10 mol% cholesterol or less and further comprise an alternative complement pathway activating agent, particularly to specifically trigger the alternative complement pathway. In a preferred embodiment, the liposomes provided in step C)a) of the method of the invention and/or liposome C) of the liposome collection of the invention comprise 10 mol% cholesterol or less and further comprise a lectin complement pathway activating agent, particularly to specifically trigger the lectin complement pathway.

A further advantage of the method of the invention is that the complement activity can be monitored in real time. For example, in the case of fluorescent detection, liposomes entrap a high concentration of a fluorescent molecule, which self-quenches so that intact liposomes, i.e. nonlysed liposomes, show only minimal fluorescent signals. Upon lysis, the dye is diluted into the surrounding solution and a significant increase in fluorescence is observed. Therefore, during incubation of liposomes with bodily fluids, the activity of the complement system can be easily monitored providing real-time signals. The same can be obtained when entrapping electrochemical markers.

A further advantage of the method of the invention is that it allows for a multiplexed analysis of complement activity. For example, by entrapping different markers (for example three distinctly different fluorescent dyes, or three distinctly different electrochemical markers) multiplexing is possible for the simultaneous quantitative detection of each complement pathway separately. A further advantage of the method of the invention is that it is a cost-efficient method of determining complement activity.

In one embodiment, the term "patient" relates to a human or an animal, preferably a human. The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

BRIEF DESCRIPTION OF THE FIGURES

The present invention is now further described by reference to the following figures.

All methods mentioned in the figure descriptions below were carried out as described in detail in the examples.

**Figure 1** shows time-resolved fluorescence intensities of a complement assay investigating a mixture of anionic liposomes AB-PTSA-liposome (**A**, encapsulating 100 mM PTSA, modified with antibodies) + CG-liposome (**B**, encapsulating 10 mM IR783, modified with 1% LPS) + AB-SRB-liposome (**C**, encapsulating 10 mM SRB, modified with antibodies). Complement assays were performed in liposome complement buffer with buffer control (black), active serum (red), inactive serum (blue) and n-octyl-β-d-glucoside (OG) positive control (green). Serum content was 10 %vol. per well. Serum source is IR35577 (i.e. Innovative Research human complement serum batch#35577). PTSA fluorescence: $\lambda_{Ex}$=375 (5) *nm* and $\lambda_{Em}$=405 (5) *nm,* 37 °C, gain=150. CG fluorescence: $\lambda_{Ex}$=790 (13) *nm* and $\lambda_{Em}$=820 (16) *nm,* 37 °C, gain=175. SRB fluorescence: $\lambda_{Ex}$=565 (5) *nm* and $\lambda_{Em}$=585 (5) *nm,* 37 °C, gain=150, n=3.

**Figure 2** shows normalized endpoint data (60 min) of time-resolved fluorescence intensities of a complement assay investigating a mixture of anionic liposomes AB-PTSA-liposome (encapsulating 100 mM PTSA, modified with antibodies) + CG-liposome (encapsulating 10 mM IR783, modified with 1% LPS) + AB-SRB-liposome (encapsulating 10 mM SRB, modified with antibodies). Normalization to positive control (30 mM OG + 10 vol% aS) at 60 min. In yellow the PTSA in violet the SRB and in green the CG fluorescence is given. The patterned bars show the data of the single liposome control samples, the not patterned bars the intensities of the liposome mixtures. Complement assays were performed in liposome complement buffer. Serum content was 10 %vol. per well. Serum source is IR35577. PTSA fluorescence: $\lambda_{Ex}$=375 (5) *nm* and $\lambda_{Em}$=405 (5) *nm,* 37 °C, gain=150. CG fluorescence: $\lambda_{Ex}$=790 (13) *nm* and $\lambda_{Em}$=820 (16) *nm,* 37 °C, gain=175. SRB fluorescence: $\lambda_{Ex}$=565 (5) *nm* and $\lambda_{Em}$=585 (5) *nm,* 37 °C, gain=150, n=3.

**Figure 3** shows normalized endpoint data (60 min) of time-resolved complement assays investigating anionic high cholesterol liposomes (42%, KH210127) with EGTA inactivation. 0.4 mM EGTA in LCB with 1 mM MgCl2 was present in each well (except controls) to inhibit the classical and lectin pathway. EGTA inactivation was done without incubation but prior to well addition. Normalization to positive control (30 mM OG + 10 vol% aS) at 60 min. Complement assays were performed in liposome complement buffer. Serum content for all liposomes was 10 %vol. per well. Serum source is pooled serum 1-4. $\lambda_{Ex}$=565 (5) *nm* and $\lambda_{Em}$=585 (5) *nm,* 37 °C, gain=150, n=3. As demonstrated, high cholesterol liposomes trigger via the classical and/or lectin pathway. This can be concluded as the lysis of these liposomes is inhibited completely in presence of EGTA. If the alternative pathway would be involved, some degree of lysis would have remained. The question whether this type of liposome is specific for one complement pathway and whether it is the classical or lectin pathway is answered with Figures 11 - 21 which show that high cholesterol liposomes are specifically triggered via the classical pathway.

**Figure 4** shows time-resolved complement assays investigating anionic high cholesterol liposomes (42%, KH210127) with EGTA inactivation. 0.4 mM EGTA in LCB with 1 mM $MgCl_2$ was present in each well (except controls) to inhibit the classical and lectin pathway. EGTA inactivation was done without incubation but prior to well addition. Complement assays were performed in liposome complement buffer. Serum content for all liposomes was 10 %vol. per well. Serum source is pooled serum 1-4. $\lambda_{Ex}$=565 (5) *nm* and $\lambda_{Em}$=585 (5) *nm,* 37 °C, gain=150, n=3. As shown, high cholesterol liposomes trigger via the classical and/or lectin pathway. This can be concluded as the lysis of these liposomes is inhibited completely in presence of EGTA. If the alternative pathway would be involved, some degree of lysis would have remained. The question whether this type of liposome is specific for one complement pathway and whether it is the classical or lectin pathway is answered with Figures 11 - 21 which show that high cholesterol liposomes are specifically triggered via the classical pathway.

**Figure 5** shows time-resolved complement assays investigating anionic high cholesterol liposomes (42%, KH210127) with varying 5G9 <anti-C3b> antibody concentrations. Antibodies are incubated with serum prior to well addition (30 min on ice). Final "in-well" antibody concentrations are shown. Complement assays were performed in liposome complement buffer. Serum content for all liposomes was 2 %vol. per well. Serum source is IR35577. $\lambda_{Ex}$=565 (8) *nm* and $\lambda_{Em}$=585 (8) *nm,* 37 °C, gain=150, n=3. As shown, the antibody 5G9 very effectively blocks the complement system. In literature it is stated that it should inhibit the classical pathway. From literature found it is not clear whether it also inhibits the lectin pathway. This set of data shows that antibodies against specific complement proteins work effectively as complement inhibitors.

**Figure 6** shows normalized endpoint data (60 min) of time-resolved complement assays investigating anionic high cholesterol liposomes (42%, KH210127) with varying 5G9 antibody concentrations. Antibodies are incubated with serum prior to well addition (30 min on ice). Final "in-well" antibody concentrations are shown. Normalization to positive control (30 mM OG + aS) at 60 min. Complement assays were performed in liposome complement buffer. Serum content for all liposomes was 2 %vol. per well. Serum source is IR35577. $\lambda_{Ex}$=565 (8) *nm* and $\lambda_{Em}$=585 (8) *nm,* 37 °C, gain=150, n=3. As shown, the antibody 5G9 very effectively blocks the complement system. In literature it is stated that it should inhibit the classical pathway. From literature found it is not clear whether it also inhibits the lectin pathway. This set of data shows that antibodies against specific complement proteins work effectively as complement inhibitors.

**Figure 7** shows complement assays. **A), B)** Normalized endpoint data (60 min) of active serum samples and **C)** Lysis inhibition values of time-resolved complement assays investigating anionic high cholesterol liposomes (42%, KH210127) with varying 5G9 antibody concentrations. Antibodies are incubated with serum prior to well addition (30 min on ice). Final "in-well" antibody concentrations are shown. Normalization in A) and B) to positive control (30 mM OG + aS) at 60 min. Lysis inhibition in C) was calculated by normalizing the endpoint values (60 min) of active serum samples to the positive control without inhibitor. Complement assays were performed in liposome complement buffer. Serum content for all liposomes was 2 %vol. per well. Serum source is IR35577. $\lambda_{Ex}$=565 (8) *nm* and $\lambda_{Em}$=585 (8) *nm,* 37 °C, gain=150, n=3. As shown, the antibody 5G9 very effectively blocks the complement system. In literature it is stated that it should inhibit the classical pathway. From literature found it is not clear whether it also inhibits the lectin pathway. This set of data shows that antibodies against specific complement proteins work effectively as complement inhibitors.

**Figure 8** shows time-resolved complement assays investigating anionic high cholesterol liposomes (42%, KH210127) with varying <anti-C1s> M81 antibody concentrations. Additional, 1 and 4 μg/ml 5G9 <anti-C3b> antibody samples are added as control. Antibodies are incubated with serum prior to well addition (30 min on ice). Final "in-well" antibody concentrations are shown. Complement assays were performed in liposome complement buffer. Serum content for all liposomes was 2 %vol. per well. Serum source is IR35577. $\lambda_{Ex}$=565 (8) *nm* and $\lambda_{Em}$=585 (8) *nm,* 37 °C, gain=150, n=3. As shown, the <anti-C1s> antibody M81 has no effect on the complement activity. Even though stated in literature, that this antibody binds Cis and therefore blocks C4 cleavage and the classical pathway, this cannot be confirmed such as with our liposome assay. However, it should be pointed out that we could not find functional assays detailing the inhibition effect of this antibody using erythrocyte assays.

**Figure 9** shows normalized endpoint data (60 min) of time-resolved complement assays investigating anionic high cholesterol liposomes (42%, KH210127) with varying <anti-C1s> M81 antibody concentrations. Additional, 1 and 4 μg/ml 5G9 <anti-C3b> antibody samples are added as control. Antibodies are incubated with serum prior to well addition (30 min on ice). Final "in-well" antibody concentrations are shown. Normalization to positive control (30 mM OG + aS) at 60 min. Complement assays were performed in liposome complement buffer. Serum content for all liposomes was 2 %vol. per well. Serum source is IR35577. $\lambda_{Ex}$=565 (8) *nm* and $\lambda_{Em}$=585 (8) *nm,* 37 °C, gain=150, n=3.

**Figure 10** shows normalized endpoint data (60 min) of active serum samples of time-resolved complement assays investigating anionic high cholesterol liposomes (42%, KH210127) with varying <anti-C1s> M81 antibody concentrations. Antibodies are incubated with serum prior to well addition (30 min on ice). Final "in-well" antibody concentrations are shown. Normalization to positive control (30 mM OG + aS) at 60 min. Complement assays were performed in liposome complement buffer. Serum content for all liposomes was 2 %vol. per well. Serum source is IR35577. $\lambda_{Ex}$=565 (8) *nm* and $\lambda_{Em}$=585 (8) *nm,* 37 °C, gain=150, n=3.

**Figure 11** shows time-resolved complement assays investigating anionic high cholesterol liposomes (42%, KH210127) with varying <anti-C1q85> antibody concentrations. Additional, 1 and 4 μg/ml 5G9 <anti-C3b> antibody samples are added as control. Antibodies are incubated with serum prior to well addition (30 min on ice). Final "in-well" antibody concentrations are shown. Complement assays were performed in liposome complement buffer. Serum content for all liposomes was 2 %vol. per well. Serum source is IR35577. $\lambda Ex$=565 (8) *nm* and $\lambda Em$=585 (8) *nm,* 37 °C, gain=150, n=3

**Figure 12** shows normalized endpoint data (60 min) of time-resolved complement assays investigating anionic high cholesterol liposomes (42%, KH210127) with varying <anti-C1q85> antibody concentrations. Additional, 1 and 4 μg/ml 5G9 <anti-C3b> antibody samples are added as control. Antibodies are incubated with serum prior to well addition (30 min on ice). Final "in-well" antibody concentrations are shown. Normalization to positive control (30 mM OG + aS) at 60 min. Complement assays were performed in liposome complement buffer. Serum content for all liposomes was 2 %vol. per well. Serum source is IR35577. $\lambda_{Ex}$=565 (8) *nm* and $\lambda Em$=585 (8) *nm,* 37 °C, gain=150, n=3.

**Figure 13** shows normalized endpoint data (60 min) of active serum samples of time-resolved complement assays investigating anionic high cholesterol liposomes (42%, KH210127) with varying <anti-C1q85> antibody concentrations. Antibodies are incubated with serum prior to well addition (30 min on ice). Final "in-well" antibody concentrations are shown. Normalization to positive control (30 mM OG + aS) at 60 min. Complement assays were performed in liposome complement buffer. Serum content for all liposomes was 2 %vol. per well. Serum source is IR35577. $\lambda_{Ex}$=565 (8) *nm* and $\lambda Em$=585 (8) *nm,* 37 °C, gain=150, n=3.

**Figure 14** shows time-resolved complement assays investigating anionic high cholesterol liposomes (42%, KH210127) with varying <anti-C1q85> antibody concentrations. Additional, 4 μg/ml 5G9 <anti-C3b> antibody samples are added as control. C1q depleted serum was also measured. Antibodies are incubated with serum prior to well addition (30 min on ice). Final "in-well" antibody concentrations are shown. Complement assays were performed in liposome complement buffer. Serum content for all liposomes was 2 %vol. per well. Serum source is pooled serum 1-4 or C1q depleted serum (A300, CompTech). $\lambda_{Ex}$=565 (8) *nm* and $\lambda Em$=585 (8) *nm,* 37 °C, gain=150, n=3.

**Figure 15** shows time-resolved complement assays investigating anionic high cholesterol liposomes (42%, KH210127) with C1q depleted serum. Serum was incubated 30 min on ice prior to well addition to be comparable to antibody containing samples. Inactive serum and positive control were prepared using pooled serum 1-4 to save C1q depleted serum. Complement assays were performed in liposome complement buffer. Serum content for all liposomes was 2 %vol. per well. Serum source is pooled serum 1-4 or C1q depleted serum (A300, CompTech). $\lambda Ex$=565 (8) *nm* and $\lambda Em$=585 (8) *nm,* 37 °C, gain=150, n=3.

**Figure 16** shows normalized endpoint data (60 min) of time-resolved complement assays investigating anionic high cholesterol liposomes (42%, KH210127) with varying <anti-C1q85> antibody concentrations. Additional, 4 μg/ml 5G9 <anti-C3b> antibody samples are added as control. C1q depleted serum was also measured. Antibodies are incubated with serum prior to well addition (30 min on ice). Final "in-well" antibody concentrations are shown. Normalization to positive control (30 mM OG + aS) at 60 min. Complement assays were performed in liposome complement buffer. Serum content for all liposomes was 2 %vol. per well. Serum source is pooled serum 1-4 or C1q depleted serum (A300, CompTech). $\lambda_{Ex}$=565 (8) *nm* and $\lambda Em$=585 (8) *nm,* 37 °C, gain=150, n=3.

**Figure 17** shows normalized endpoint data (60 min) of time-resolved complement assays investigating anionic high cholesterol liposomes (42%, KH210127) with C1q depleted serum. Normalization to positive control (30 mM OG + aS) at 60 min. Complement assays were performed in liposome complement buffer. Serum content for all liposomes was 2 %vol. per well. Serum source is pooled serum 1-4 or C1q depleted serum (A300, CompTech). $\lambda_{Ex}$=565 (8) *nm* and $\lambda Em$=585 (8) *nm,* 37 °C, gain=150, n=3.

**Figure 18** shows normalized endpoint data (60 min) of active serum samples of time-resolved complement assays investigating anionic high cholesterol liposomes (42%, KH210127) with varying <anti-C1q85> antibody concentrations. Antibodies are incubated with serum prior to well addition (30 min on ice). Final "in-well" antibody concentrations are shown. Normalization to positive control (30 mM OG + aS) at 60 min. Complement assays were performed in liposome complement buffer. Serum content for all liposomes was 2 %vol. per well. Serum source is pooled serum 1-4. $\lambda_{Ex}$=565 (8) *nm* and $\lambda Em$=585 (8) *nm,* 37 °C, gain=150, n=3.

**Figure 19** shows lysis inhibition values of time-resolved complement assays investigating anionic high cholesterol liposomes (42%, KH210127) with varying <anti-C1q85> antibody concentrations. Antibodies are incubated with serum prior to well addition (30 min on ice). Final "in-well" antibody concentrations are shown. Lysis inhibition was calculated by subtracting the inactive serum sample intensities as background and normalizing the endpoint values (60 min) of active serum samples to the positive control without inhibitor. Complement assays were performed in liposome complement buffer. Serum content for all liposomes was 2 %vol. per well. Serum source is pooled serum 1-4. $\lambda_{Ex}$=565 (8) nm and $\lambda Em$=585 (8) *nm*, 37 °C, gain=150, n=3.

**Figure 20** shows time-resolved complement assays investigating anionic high cholesterol (42%, KH210127, 1 μM tL), LPS (1% LPS, AG201006-1, **1** μM tL) and antibody modified liposomes (AG210412-2, 0.5 ml% <anti-biotin> AB, 1 μM tL) with C1q depleted serum. As control, all liposomes are additionally measured with IR35577 serum. Antibodies were incubated with liposomes for 1h at RT. Complement assays were performed in liposome complement buffer. Serum content for all liposomes was 2 %vol. per well. Serum source is IR35577 or C1q depleted serum (A300, CompTech). $\lambda Ex$=565 (8) *nm* and $\lambda Em$=585 (8) *nm*, 37 °C, gain=150, n=3.

**Figure 21** shows normalized endpoint data (60 min) of time-resolved complement assays investigating anionic high cholesterol (42%, KH210127), LPS (1% LPS, AG201006-1) and antibody modified liposomes (AG210412-2, 0.5 ml% <anti-biotin> AB) with C1q depleted serum. As control, all liposomes are additionally measured with IR35577 serum. Antibodies were incubated with liposomes for 1h at RT. Normalization to positive control (30 mM OG + aS) at 60 min. Complement assays were performed in liposome complement buffer. Serum content for all liposomes was 2 %vol. per well. Serum source is IR35577 or C1q depleted serum (A300, CompTech). $\lambda Ex$=565 (8) *nm* and $\lambda Em$=585 (8) *nm,* 37 °C, gain=150, n=3.

**Figure 22** shows time-resolved complement assays investigating anionic 1 mol% LPS liposomes (AG201006-1, 10 μM) with a dilution curve of Ci-INH incubated with serum prior to well addition (60 min on ice). Ci-INH was added to inhibit the classical and lectin pathway. Complement assays were performed in liposome complement buffer. Serum content for all liposomes was 5 %vol. per well. Stated Ci-INH content is the concentration in the well. Serum source is Innovative Research human complement serum. $\lambda_{Ex}$*=565 nm and* $\lambda_{Em}$*=585 nm,* 37 °C, gain=150, n=3. As shown, lysis of LPS modified liposomes is inhibited to a certain degree in presence of C1-INH. Ci-INH is known to inhibit the classical and lectin pathway. As LPS is used as trigger, the alternative pathway is involved as well and therefore some degree of lysis remains (alternative pathway related lysis). This data set shows, that the Ci-INH is suitable as inhibitor in the liposome complement assay and can be used for alternative pathway specific lysis in combination with LPS modified liposomes.

**Figure 23** shows time-resolved complement assay active serum samples investigating anionic 1 mol% LPS liposomes (AG201006-1, 10 μM) with Ci-INH incubated (60 min on ice) with serum prior to well addition. Ci-INH was added to inhibit the classical and lectin pathway. Complement assays were performed in liposome complement buffer. Serum content for all liposomes was 5 %vol. per well. Serum source is Innovative Research human complement serum. $\lambda_{Ex}$=565 *nm* and $\lambda_{Em}$=585 *nm,* 37 °C, gain=150, n=3.

**Figure 24** shows fluorescence intensity values after 60 minutes of the complement assay active serum samples investigating anionic 1 mol% LPS liposomes (AG201006-1, 10 μM) with Ci-INH incubated (60 min on ice) with serum prior to well addition. Complement assays were performed in liposome complement buffer. Serum content for all liposomes was 5 %vol. per well. Serum source is Innovative Research human complement serum. $\lambda_{Ex}$=565 *nm* and $\lambda_{Em}$=585 *nm,* 37 °C, gain=150, n=3.

**Figure 25** shows normalized endpoint data (60 min) of time-resolved complement assays investigating anionic 1 mol% LPS liposomes (AG201006-1, 10 μM) with Ci-INH incubated (60 min on ice) with serum prior to well addition. Normalization to positive control (30 mM OG + 10 vol% aS) at 60 min. Complement assays were performed in liposome complement buffer. Serum content for all liposomes was 5 %vol. per well. Serum source is Innovative Research human complement serum. $\lambda_{Ex}$=565 *nm* and $\lambda_{Em}$=585 *nm,* 37 °C, gain=150, n=3.

**Figure 26** shows time-resolved complement assays investigating anionic 1 mol% LPS liposomes (AG201006-1, 10 μM) with EGTA preincubation. EGTA was added prior to well addition (5 min, RT) in a 1:1 ratio to serum and finally diluted to 0 to 1 mM EGTA. EGTA was added to inhibit the classical and lectin pathway. Complement assays were performed in liposome complement buffer. Serum content for all liposomes was 10 %vol. per well. Serum source is pooled serum 1-4. $\lambda_{Ex}$=565 *nm* and $\lambda_{Em}$=585 *nm,* 37 °C, gain=150, n=3

**Figure 27** shows normalized endpoint data (60 min) of time-resolved complement assays investigating anionic 1 mol% LPS liposomes (AG201006-1, 10 μM) with EGTA preincubation. EGTA was added prior to well addition (5 min, RT) in a 1:1 ratio to serum and finally diluted to 0 to 1 mM EGTA. EGTA was added to inhibit the classical and lectin pathway. Normalization to positive control (30 mM OG + 10 vol% aS) at 60 min. Complement assays were performed in liposome complement buffer. Serum content for all liposomes was 10 %vol. per well. Serum source is pooled serum 1-4. $\lambda_{Ex}$=565 *nm* and $\lambda_{Em}$=585 *nm,* 37 °C, gain=150, n=3.

**Figure 28** shows active serum samples of time-resolved complement assays investigating anionic 1 mol% LPS liposomes (AG201006-1, 10 μM) with EGTA preincubation. EGTA was added prior to well addition (5 min, RT) in a 1:1 ratio to serum and finally diluted to 0 to 1 mM EGTA. EGTA was added to inhibit the classical and lectin pathway. Complement assays were performed in liposome complement buffer. Serum content for all liposomes was 10 %vol. per well. Serum source is pooled serum 1-4. $\lambda_{Ex}$=565 *nm* and $\lambda_{Em}$=585 *nm,* 37 °C, gain=150, n=3.

**Figure 29** shows time-resolved complement assays investigating anionic 1 mol% LPS liposomes (AG201006-1, 10 μM) with varying $MgCl_2$ levels (0.2 to 1 mM) and EGTA preincubation. EGTA was added prior to well addition (5 min, RT) in a 1:1 ratio to serum and finally diluted to 0 or 0.4 mM EGTA. EGTA was added to inhibit the classical and lectin pathway. Complement assays were performed in liposome complement buffer. Serum content for all liposomes was 10 %vol. per well. Serum source is pooled serum 1-4. $\lambda_{Ex}$=565 *nm* and $\lambda_{Em}$=585 *nm,* 37 °C, gain=150, n=3

**Figure 30** shows normalized endpoint data (60 min) of time-resolved complement assays investigating anionic 1 mol% LPS liposomes (AG201006-1, 10 μM) with varying $MgCl_2$ levels (0.2 to 1 mM) and EGTA preincubation. EGTA was added prior to well addition (5 min, RT) in a 1:1 ratio to serum and finally diluted to 0 or 0.4 mM EGTA. Normalization to positive control (30 mM OG + 10 vol% aS) at 60 min. Complement assays were performed in liposome complement buffer. Serum content for all liposomes was 10 %vol. per well. Serum source is pooled serum 1-4. $\lambda_{Ex}$=*565 nm and* $\lambda_{Em}$=*585 nm,* 37 °C, gain=150, n=3.

**Figure 31** shows active serum samples of time-resolved complement assays investigating anionic 1 mol% LPS liposomes (AG201006-1, 10 μM) with varying $MgCl_2$ levels (0.2 to 1 mM) and EGTA preincubation. EGTA was added prior to well addition (5 min, RT) in a 1:1 ratio to serum and finally diluted to 0 or 0.4 mM EGTA. EGTA was added to inhibit the classical and lectin pathway. Complement assays were performed in liposome complement buffer. Serum content for all liposomes was 10 %vol. per well. Serum source is pooled serum 1-4. $\lambda_{Ex}$=565 *nm* and $\lambda_{Em}$=585 *nm,* 37 °C, gain=150, n=3.

**Figure 32** shows time-resolved complement assays investigating anionic 1 mol% LPS liposomes (AG201006-1, 10 μM) with EGTA preincubation. EGTA was added prior to well addition (5 min, RT) in a 1:1 ratio to serum and finally diluted to 0 to 0.4 mM EGTA. EGTA was added to inhibit the classical and lectin pathway. Complement assays were performed in liposome complement buffer. Serum content for all liposomes was 10 %vol. per well. Serum source is pooled serum 1-4. $\lambda_{Ex}$=565 *nm* and $\lambda_{Em}$=585 *nm,* 37 °C, gain=150, n=3.

**Figure 33** shows normalized endpoint data (60 min) of time-resolved complement assays investigating anionic 1 mol% LPS liposomes (AG201006-1, 10 μM) with EGTA preincubation. EGTA was added prior to well addition (5 min, RT) in a 1:1 ratio to serum and finally diluted to 0 to 0.4 mM EGTA. EGTA was added to inhibit the classical and lectin pathway. Normalization to positive control (30 mM OG + 10 vol% aS) at 60 min. Complement assays were performed in liposome complement buffer. Serum content for all liposomes was 10 %vol. per well. Serum source is pooled serum 1-4. $\lambda_{Ex}$=565 *nm* and $\lambda_{Em}$=585 *nm,* 37 °C, gain=150, n=3.

**Figure 34** shows active serum samples of time-resolved complement assays investigating anionic 1 mol% LPS liposomes (AG201006-1, 10 μM) with EGTA preincubation. EGTA was added prior to well addition (5 min, RT) in a 1:1 ratio to serum and finally diluted to 0 to 0.4 mM EGTA. EGTA was added to inhibit the classical and lectin pathway. Complement assays were performed in liposome complement buffer. Serum content for all liposomes was 10 %vol. per well. Serum source is pooled serum 1-4. $\lambda_{Ex}$=565 *nm* and $\lambda_{Em}$=585 *nm,* 37 °C, gain=150, n=3.

**Figure 35** shows time-resolved complement assays investigating anionic 1 mol% LPS liposomes (AG201006-1, 10 μM) with varying EGTA inactivation times (0-35 min). EGTA was incubated 1:1 with serum prior to well addition and the concentration was constant at 0.4 mM EGTA in LCB with 1 mM MgCl2 in each well (except controls) to inhibit the classical and lectin pathway. Complement assays were performed in liposome complement buffer. Serum content for all liposomes was 10 %vol. per well. Serum source is pooled serum 1-4. $\lambda_{Ex}$=565 *nm* and $\lambda_{Em}$=585 *nm,* 37 °C, gain=150, n=3.

**Figure 36** shows active serum samples of time-resolved complement assays investigating anionic 1 mol% LPS liposomes (AG201006-1, 10 μM) with varying EGTA inactivation times (0-35 min). EGTA was incubated 1:1 with serum prior to well addition and the concentration was constant at 0.4 mM EGTA in LCB with 1 mM $MgCl_2$ in each well (except controls) to inhibit the classical and lectin pathway. Complement assays were performed in liposome complement buffer. Serum content for all liposomes was 10 %vol. per well. Serum source is pooled serum 1-4. $\lambda_{Ex}$*=565 nm and* $\lambda_{Em}$*=585 nm,* 37 °C, gain=150, n=3

**Figure 37** shows normalized endpoint data (60 min) of time-resolved complement assays investigating anionic 1 mol% LPS liposomes (AG201006-1, 10 μM) with varying EGTA inactivation times (0-35 min). EGTA was incubated 1:1 with serum prior to well addition and the concentration was constant at 0.4 mM EGTA in LCB with 1 mM MgCl2 in each well (except controls) to inhibit the classical and lectin pathway. Normalization to positive control (30 mM OG + 10 vol% aS) at 60 min. Complement assays were performed in liposome complement buffer. Serum content for all liposomes was 10 %vol. per well. Serum source is pooled serum 1-4. $\lambda_{Ex}$=565 *nm* and $\lambda_{Em}$=585 *nm,* 37 °C, gain=150, n=3.

In the following, reference is made to the examples, which are given to illustrate, not to limit the present invention.

EXAMPLES

**Example 1:** Alternative pathway

Solutions followed the mentioned protocol below:

- Liposomes (in this case modified with 1 mol% lipopolysaccharide)
- Buffer:

  liposome complement buffer (LCB)
  10 mM HEPES, 150 mM NaCl, 135 nM $CaCl_2$, 1 mM $MgCl_2$, pH 7.4

- Inactivation buffer (for negative control, to inactivate the complement system):

  liposome inactivation complement buffer (iaCB)
  90% liposome complement buffer and 200 mM EDTA, 0.5 μM EGTA pH ~ 8

- Inhibitor solution (to inactivate the classical and lectin pathway of the complement system)

  EGTA containing liposome complement buffer, pH 7.4
  or C1-Inh (for control experiments)

- Sucrose containing liposome complement buffer (used in all wells, to adjust the sucrose content in each well to ensure better liposome stability)
- Human serum
- Detergent containing solution (used in the positive control, to lyse all liposomes) 300 mM n-octyl-β-d-glucoside (OG) in bidest. H2O

Preparations for the assay:

1. Prepare liposome stock solution

   - mix liposomes with buffer to achieve a 10x concentrated stock solution which is needed later

2. Prepare respective inhibitor stock solution

   - mix inhibitor with buffer to achieve a 10x concentrated stock solution

3. Prepare a black micro titer plate on ice

   - Add buffer to all wells (volume is different for buffer control, active serum, negative control and positive control, all wells should end up at 100 μl once all solutions are added in the end)
   - Add sucrose solution (1 M stock, 20 μl in each well)

- Add inactivation buffer (10 µl in respective negative control wells)

4. Add detergent solution (10 µl in respective positive control wells)
5. Preheat fluorescence plate reader to 37 °C

Steps to perform the assay:

6. Mix serum with the respective amounts of inhibitors (prepared in 2.)
7. Incubate mixtures for respective time

- 60 min on ice for C1-Inh (only used in control experiments)
- 5 min at room temperature for EGTA studies (if not stated differently)

8. Meanwhile, add liposomes (prepared in 1.), 10 µl in each well
9. Add respective amounts of serum-inhibitor mixtures to all serum containing samples (positive control, negative control and active serum, addition to active serum should be last)

All wells should now include 100 µl total volume.
10. Measure time-resolved fluorescence intensities
The fluorescence intensity was measured with a BioTek SYNERGY neo2 fluorescence reader. The wavelengths were $\lambda_{Ex}$ = 565 (5) *nm* and $\lambda_{Em}$ = 585 (5) *nm.* Measurement temperature in the device was constant at 37 °C. Measurements were performed with a gain of 150. Measurement was performed for 60 minutes. The first 15 minutes the measurement interval was 1.5 minutes with three consecutive measurements per time stamp. The following 45 minutes were performed with a 5-minute interval again with three consecutive measurements per time stamp. Each sample is prepared in triplicates.

**Example 2:** Classical pathway

Solutions followed the mentioned protocol below:

- Liposomes (in this case with high cholesterol content, eg. 44 or 42 mol%)
- Buffer:

  liposome complement buffer (LCB)
  10 mM HEPES, 150 mM NaCl, 135 nM $CaCl_2$, 1 mM $MgCl_2$, pH 7.4

- Inactivation buffer (for negative control, to inactivate the complement system):

  liposome inactivation complement buffer (iaCB)
  90% liposome complement buffer and 200 mM EDTA, 0.5 µM EGTA pH ~ 8

- Sucrose containing liposome complement buffer (used in all wells, to adjust the sucrose content in each well to ensure better liposome stability)
- Human serum
- Detergent containing solution (used in the positive control, to lyse all liposomes) 300 mM n-octyl-β-d-glucoside (OG) in bidest. H2O

Preparations for the assay:

1. Prepare liposome stock solution

- mix liposomes with buffer to achieve a 10x concentrated stock solution which is needed later

2. Prepare a black micro titer plate on ice

- Add buffer to all wells (volume is different for buffer control, active serum, negative control and positive control, all wells should end up at 100 µl once all solutions are added in the end)
- Add sucrose solution (1 M stock, 20 µl in each well)

- Add inactivation buffer (10 µl in respective negative control wells)

3. Add detergent solution (10 µl in respective positive control wells)
4. Preheat fluorescence plate reader to 37 °C

Steps to perform the assay:

5. Add liposomes (prepared in 1.), 10 µl in each well
6. Add respective amounts of serum to all serum containing samples (positive control, negative control and active serum, addition to active serum should be last)

All wells should now include 100 µl total volume.
7. Measure time-resolved fluorescence intensities
The fluorescence intensity was measured with a BioTek SYNERGY neo2 fluorescence reader. The wavelengths were $\lambda_{Ex}$ = 565 (5) *nm* and $\lambda_{Em}$ = 585 (5) *nm.* Measurement temperature in the device was constant at 37 °C. Measurements were performed with a gain of 150. Measurement was performed for 60 minutes. The first 15 minutes the measurement interval was 1.5 minutes with three consecutive measurements per time stamp. The following 45 minutes were performed with a 5-minute interval again with three consecutive measurements per time stamp.
Each sample is prepared in triplicates.

**Example 3:** Identification of liposomes selective for the classical pathway

Objective: The focus of this study was to identify liposomes that can be used for the identification of the classical pathway.
To determine activation of the alternative pathway, EGTA was added to the samples, which blocks the classical and lectin pathways (see Figure 3). For the differentiation between lectin and classical pathways, several antibodies from Hycult Biotech and from Sanquin were kindly provided.

(i) mAb HM2285 (C3b/iC3b, Human, clone 5G9) should inhibit the classical pathway and not the alternative pathway. But as it binds C3b(i), we were not sure whether it also blocks lectin pathway or not.
(ii) <anti-Cis> M81 (HM2108) provided by Hycult Biotech binds Cis at the binding site of C4/C4b and therefore should block C4-cleavage and further classical pathway activation.
(iii) an <anti-C1q> antibody termed "<anti-C1q85>" provided by Sanquin. This antibody should bind C1q in the globular head region (GR) and effectively block the complement system, i.e. selectively block the classical pathway.

Summary of results: We could demonstrate (see Figures 3 and 4) that liposomes with high concentrations of cholesterol activate the complement system through the classical pathway. This was demonstrated through three strategies: (i) upon addition of EGTA no liposome lysis occurred; (ii) upon addition of antibodies blocking the classical pathway, an inhibition curve of complement activation could be observed including complete inhibition at the higher antibody concentrations tested; (iii) using C1q-depleted serum led to no activation of the complement system by the liposomes. In comparison to the erythrocyte assay, where antibodies need to be added to specifically target the classical pathway, this is not necessary here.
Conclusion: On a broader perspective and including data not shown in this application - we are now able to produce pathway selective complement lysis for two of the three pathways:

- The classical pathway can be investigated with the high cholesterol liposomes. It can also be addressed using ligand-bearing liposomes and an <anti-ligand> as demonstrated with our biotinylated liposomes and <anti-biotin>
- The alternative pathway can be examined with LPS-containing liposomes in combination with EGTA (to block classical and lectin pathway). The remaining lysis is a measure for the alternative pathway activity.

Materials

| Liposome | Keywords | Composition | tL concentration [mM] |
|---|---|---|---|
| KH210127 | 42% chol, anionic | 42 mol% Cholesterol<br>35 mol% DPPC<br>20 mol% DPPG<br>2 mol% DPPE-Biotin | 6.44 |

- Liposome complement buffer (LCB)
  10 mM HEPES, 150 mM NaCl, 135 nM $CaCl_2$, 1 mM $MgCl_2$, pH 7.4
- Liposome inactivation complement buffer (iaCB)
  90% liposome complement buffer and 200 mM EDTA, 0.5 μM EGTA pH ~ 8
- Antibodies:

  - mAb HM2285 (C3b/iC3b, Human, clone 5G9, 100 μg/ml)
  - mAb HM2108 (Cis, Human, mAb M81, 1.02 mg/ml, Hycult)
  - mAb <anti-C1q85> (mouse, 0.84 mg/ml, Sanquin)

- 1 M sucrose solution in LCB
- Human serum

  - C1q depleted serum (CompTech, A300)
  - Innovative Research human complement serum batch IR35577
  - Pooled serum from volunteers 1-4, $1\,(^1/_6)$, $2\,(^1/_6)$, 3 (⅓) and 4 (⅓)
    Active serum thawed in hand and added to the wells/mixed accordingly.

- 300 mM n-octyl-β-d-glucoside (OG) in bidest. $H_2O$

Method

All dose response curves with the antibodies were prepared with the same protocol, just exchanging the antibody.

If not stated differently a concentration range of 0, 0.5, 1, 2, 4 and 8 μg/ml antibody in combination with 2% IR35577 serum was prepared with 1 μM total lipids (tL) of high cholesterol liposomes KH210127. These values refer to the final conditions in the well. For antibody containing samples only the active serum sample is measured to save antibody.

Procedure (also prepare respective controls with PBS addition instead of antibody for buffer, aS, iaS and OG control):

1. Prepare 10 μM (tL) liposome solution (in LCB)
2. Prepare black MTP on ice with LCB, sucrose (1 M stock in LCB, 20 μl in each well), iaCB (10 μl in respective wells) and 300 mM OG (10 μl in respective wells)
3. Add liposomes, 10 μl in the respective wells (results finally in 1 μM tL per well)
4. Preheat BioTek Reader to 37 °C
5. Mix active serum with the respective amounts of inhibitor (8 μl serum + 32 μl of respective inhibitor, 100, 50, 25, 12.5 or 6.25 μg/ml stock). Important is that the final concentrations in the well are correct.
6. Incubate serum-inhibitor-mix 30 min on ice
7. Add respective amounts of serum-inhibitor mixtures (10 μl) to achieve 2% serum content and the right inhibitor concentration in the well to inactive serum and OG wells
8. Add respective amounts of serum-inhibitor mixtures (10 μl) to achieve 2% serum content and the right inhibitor concentration in the well to active serum wells
9. All wells should now include 100 μl total volume
10. Measure time-resolved fluorescence intensities

The fluorescence intensity was measured with a BioTek SYNERGY neo2 fluorescence reader. The wavelengths were $\lambda_{Ex}$ = 565 (8) *nm* and $\lambda_{Em}$ = 585 (8) *nm.* Measurement temperature in the device was constant at 37 °C. Measurements were performed with a gain of 150. Variations included <anti-C1q> antibody "anti-C1q85" tested with an updated concentration range and pooled serum 1-4. Concentration range of 0,2,3,4,5,6,7 and 8 μg/ml <anti-C1q85>

antibody in combination with 2% pooled serum 1-4 and 1 μM tL KH210127 liposomes. Additionally, 1 μM tL KH210127 liposomes are also measured with 2% C1q depleted serum. Again only active serum is measured to save C1q depleted serum. Samples with C1q depleted serum followed the same protocol as the inhibitor solutions, just with PBS addition instead of antibody addition. This ensures comparability between the samples.

To confirm that the C1q-depleted serum has an otherwise functional complement system, high cholesterol, antibody or LPS modified liposomes were tested at 1 μM tL and 2% C1q depleted serum. As reference, normal human serum 2% IR35577 was investigated with the same liposomes.

Results mAb HM2285 (C3b/iC3b, Human, clone 5G9)

The antibody 5G9 very effectively blocks the complement system. In literature it is stated that it should inhibit the classical pathway. From literature found it is not clear whether it inhibits also the lectin pathway. Varying the concentrations of the antibody from 0.5 - 8 μg/mL (in-well concentrations) an expected inhibition response curve is obtained, where complete inhibition is obtained at 8 μg/ml of 5G9 antibody. Results are shown as raw time course data (Figure 5) with associated bar graph of the final fluorescence signals obtained in the time-course experiments (Figure 6). In Figure 7, three different data representations are provided to analyze the data. The relationship between antibody concentration and resulting liposome lysis is plotted and shows the typical logarithmic relationship of an inhibition reaction.

Results <anti-C1s> antibody M81

Surprisingly, the <anti-C1s> antibody M81 has no effect on the complement activity. Even though stated in literature that this antibody binds Cis and therefore blocks C4 cleavage and the classical pathway, this cannot be confirmed in a real patient sample with our liposome assay. However, it should be pointed out that we could not find functional assays detailing the inhibition effect of this antibody using erythrocyte assays. Data shown include the rad fluorescence time course data (Figure 8), the associated bar graph of the final endpoint fluorescence signals (Figure 9) and the inhibition curve showing lysis vs. antibody concentration (Figure 10). It appears that the liposome assay of the invention properly reflects the functionality of the complement system and is superior to complement assays which are merely based on protein assays.

Results <anti-C1q85> (Sanquin), IR35577 serum

The <anti-C1q85> antibody was initially tested in commercially available, pooled human serum (IR35577). Again, a typical inhibition curve was found for concentrations ranging between 2 (almost no inhibition) and 8 μg/ml (complete inhibition). It has to be noted that the control sample without inhibitor shows higher lysis than expected. Usually a lysis of around 70% has been achieved under the exact same conditions in previous experiments. Nevertheless, a clear trend can be seen. Raw data are provided in **Fehler! Verweisquelle konnte nicht gefunden werden.** 11 and the associated bar graph of fluorescence signals at the end point (Figure 12). The final inhibition curve shown as lysis vs. antibody concentration demonstrates the inhibition potential of the antibody in this functional liposome assay (Figure 13).

Inhibition with <anti-C1q85> appears more effective compared to 5G9 at the same concentration (4 μg/ml). These data suggest that the high cholesterol liposomes only activate the classical pathway. Thus, advantageously, high cholesterol liposomes, e.g. liposomes with a cholesterol content of at least 30 mol% cholesterol, can be used to specifically target the classical pathway.

Results <anti-C1q85> (Sanquin), pooled serum 1-4, C1q-depleted serum

Results indicated that high cholesterol liposomes are triggered by the classical pathway only. To confirm that this is not due to irregularities stemming from the human serum used, the following two studies were performed: (i) testing these liposomes with C1q deficient serum, (ii) testing these liposomes with the <antiC1q85> with 2% pooled serum 1-4 with a slightly optimized concentration range of 2, 3, 4,5, 6, 7, 8 μg/ml.

It was found that the pooled serum provided the same result as the prior used IR serum, just with a slightly shifted active range to smaller antibody amounts. (Figures 14, 16, 18, and 19). Furthermore, liposomes were not lysed in the C1q-depleted serum sample (Figure 15). These data prove the findings that these high cholesterol liposomes only trigger the classical pathway is correct, not related to the serum source used, and also evident in sera that do not allow the classical pathway to be activated.

Results C1q-depleted serum

To confirm that the C1q-depleted serum is able to actually cause complement induced lysis to liposomes, a control experiment was performed with high cholesterol, antibody or LPS modified liposomes. It was expected that at least LPS modified liposomes show a certain degree of complement induced lysis in this serum.

This indeed held true. LPS modified liposomes could be partially lysed with C1q depleted serum, as not only the classical pathway is activated by this trigger. Still, the amount of lysis is reduced in C1q depleted compared to the positive control with normal human serum (Figure 20 and 21). This effect could also be seen for studies including LPS modified liposomes with EGTA (not shown). Indicating that the classical pathway is responsible for a decent amount of lysis also for the LPS modified liposomes.

Antibody modified and high cholesterol liposomes both show no lysis increase in C1q-depleted serum which suggests solely classical pathway activation by these triggers.

**Example 4:** Identification of liposomes selective for the alternative pathway

Objective: The focus of this study was to identify liposomes that can be used for the identification of the alternative pathway. It was found that liposomes modified with LPS on the lipid membrane activate the complement system. In this experiment, the focus is on understanding whether the alternative pathway is activated and can be investigated separately from the two remaining pathways.

To determine activation of the alternative pathway, C1-Inh was added to the samples, which is a known complement inhibitor of the classical and lectin pathway. EGTA as selective complexing agent with higher affinity to $Ca^{2+}$ than to $Mg^{2+}$ was investigated as cheaper C1-Inh alternative, as the alternative pathway is the only pathway being active under $Ca^{2+}$ deficient conditions.

Summary of results: We could demonstrate that liposomes with LPS on the liposome surface partially activate complement system through the alternative pathway. This was demonstrated through two strategies: (i) upon addition of C1-Inh blocking classical and lectin pathway, liposome lysis was reduced in a dose dependent manner; (ii) upon addition of EGTA with adjusted $Mg^{2*}$ content, an inhibition curve of complement activation could be observed until a remaining constant lysis rate at the higher EGTA concentrations tested. This was optimized to a CH50 like assay, allowing serum specific alternative pathway complement activation interpretations. Thus, advantageously, the alternative complement pathway can be specifically targeted.

Conclusion: On a broader perspective and including data not shown in this application - we are now able to produce pathway selective complement lysis for two of the three pathways:

- The classical pathway can be investigated with the high cholesterol liposomes. It can also be addressed using ligand-bearing liposomes and an <anti-ligand> as demonstrated with our biotinylated liposomes and <anti-biotin>
- The alternative pathway can be examined with LPS-containing liposomes in combination with EGTA (to block classical and lectin pathway). The remaining lysis is a measure for the alternative pathway activity.

Materials

| Liposome | Keywords | Composition | tL concentration [mM] |
|---|---|---|---|
| **AG201006-1** | Anionic, 1% LPS | 5 mol% Cholesterol<br>77 mol% DPPC<br>18 mol% DPPG<br>2 mol% DPPE-Biotin<br>1 mol% LPS | 13.31 |

- Liposome complement buffer (LCB)
  10 mM HEPES, 150 mM NaCl, 135 nM $CaCl_2$, 1 mM $MgCl_2$, pH 7.4
- Liposome inactivation complement buffer (iaCB)
  90% Liposome complement buffer and 200 mM EDTA, 0.5 μM EGTA pH ~ 8
- C1-inhibitor (C1-INH) (Cinryze from VarioPharma) 200 U/mL in sterile water
- 1 M sucrose
- EGTA inactivation buffer (inactivation of classical and lectin pathway)

  100 mM EGTA in LCB, pH 7.4
  Used to prepare various EGTA solutions.

- Human serum
    - Pooled serum 1 (1/6), 2 (1/6), 3 (⅓) and 4 (⅓)
    - Innovative Research human pooled complement serum
        - Active serum thawed and added to the wells.
        - Serum was inactivated by adding 10 μl iaCB into the respective wells
- 300 mM n-octyl-β-d-glucoside (OG) in bidest. $H_2O$

Method

All assays were performed following a similar basic procedure with slight adjustments in incubation time and serum content which are described separately for each experiment if changed.
Ci-INH including studies were performed with 5, 2.5, 1.25, 0.625 and 0 U/ml of Ci-INH in each well with a serum content of 5%. This means 0.5, 0.25, 0.125, 0.0625 U/well (well volume 100 μl) and 5 μl serum per well.
Procedure:

1. Prepare 100 μM (tL) liposome solution
2. Prepare respective inhibitor stock solution dilution curve
3. Mix serum with the respective amounts of inhibitors. Important is that the final concentrations in the well are correct. It has to be calculated for each inhibitor separately as the dilution factors are not constant.
4. Incubate mixtures for respective time

    - 60 min on ice for C1-Inh
    - 5 min at RT for EGTA studies (if not stated differently)

5. Prepare black MTP on ice with LCB, sucrose (1 M stock, 20 μl in each well), iaCB (10 μl in respective wells) and 300 mM OG (10 μl in respective wells)
6. Add liposomes, 10 μl in the respective wells (results finally in 10 μM tL per well)
7. Preheat BioTek Reader to 37 °C
8. Add respective amounts of serum-inhibitor mixtures to achieve desired serum content and the right inhibitor concentration in the well to inactive serum and OG wells
9. Add respective amounts of serum-inhibitor mixtures to achieve desired serum content and the right inhibitor concentration in the well to active serum wells
10. All wells should now include 100 μl total volume
11. Measure time-resolved fluorescence intensities

The fluorescence intensity was measured with a BioTek SYNERGY neo2 fluorescence reader. The wavelengths were $\lambda_{Ex}$ = 565 (5) *nm* and $\lambda_{Em}$ = 585 (5) *nm.* Measurement temperature in the device was constant at 37 °C. Measurements were performed with a gain of 150.

Results: complement assay including Ci-INH with LPS-liposomes

Note: 5 vol% serum samples with 10 μM tL are investigated!
As it can be seen in Figure 22 and Figure 23, Ci-INH inhibits LPS-liposome lysis. It is reduced from 45% lysis without Ci-INH being present to 30% for the highest investigated inhibitor concentration (5 U/ml with 5% serum). Ci-INH is known to inhibit the classical and lectin pathway. These results are in good correlation with this assumption as full inhibition is not achieved. To confirm, that higher concentrations of inhibitor do not result in complete inhibition 10 U/ml Ci-INH were tested in a separate experiment under the same conditions and lead to the same result as 5 U/ml (data not shown). According to this data, LPS liposomes trigger not only via the alternative pathway but also via the classical and/or lectin pathway and blocking the latter two with the Ci-INH causes reduced liposome lysis. Hence, the remaining lysis after C1-INH addition is a measure for the alternative pathway activity of the respective serum.

Results: complement assay including EGTA

A cheaper than, but similar effective way as C1-INH to inactivate classical and lectin pathway is the use of

EGTA/$Mg^{2+}$ buffer. EGTA complexes $Ca^{2+}$ which is needed for classical and lectin pathway activity but not in the alternative pathway. The alternative pathway only needs $Mg^{2+}$ to be functional.

The EGTA content was varied during 1:1 incubation with serum. After 5 minutes incubation, serum EGTA mixture was diluted 1/5 in the well directly. This resulted in EGTA concentrations of 0, 0.2, 0.4, 0.8 and 1 mM EGTA. $MgCl_2$ was removed from the liposome complement buffer and included in the serum incubation directly. Meaning 10 mM $MgCl_2$ in each stock solution, 5 mM $MgCl_2$ during 1:1 incubation and 1 mM $MgCl_2$ in each well. This ensures, that EGTA has always at least equally high amounts of $Mg^{2+}$ to complex. As control, a "normal" LCB with $Mg^{2+}$ samples was prepared as well.

Serum content in each well was 10 vol.%, liposome concentration was 10 μM total lipid.

It can be seen that it makes no difference whether the $MgCl_2$ is included during preincubation or in the well, as both controls without EGTA show similar lysis of the LPS liposomes. Advantageously, 0.4 mM EGTA is already enough to block the classical and lectin pathway pathways efficiently, resulting in a lysis value of 46%. Increasing the EGTA content to higher values till 1 mM EGTA (final concentration in the well) does not provide higher inhibition effects. Thus, it can be assumed, that classical and lectin pathway are efficiently blocked with an EGTA content of 0.4 mM and 1 mM $MgCl_2$.

In most literature it was stated, that $Mg^{2+}$ levels should be kept similar to the EGTA levels to ensure $Mg^{2+}$ levels close to physiological conditions upon $Ca^{2+}$ complexation. Too low $Mg^{2+}$ levels would leave "free uncomplexed" EGTA which will also complex the $Mg^{2+}$ content provided by the serum (additionally to the $Ca^{2+}$ provided by the serum itself).

A drastic drop in lysis can be observed after the EGTA level equals or outcompetes the theoretical $Ca^{2+}$ levels in serum (~2.5 mM $Ca^{2+}$, 100% serum). If the EGTA level is below, classical and lectin pathway seem to work similar well. If the EGTA level lies above, even if it is only minor, the classical and lectin pathway seem to be blocked. This correlates well with the fact that only 135 nM $Ca^{2+}$ concentrations are included in the general buffer (Liposome complement buffer, LCB). 0.4 mM EGTA was determined as most suitable condition for further experiments.

Results: complement assay with EGTA, $Mg^{2+}$ optimization

Based on the above findings, various $MgCl_2$ concentrations in combination with 0.4 mM EGTA (final concentration in well) were investigated. Thus, $Mg^{2+}$ content was varied between 0.2, 0.4, 0.6 and 1 mM $MgCl_2$ in the final well with and without EGTA addition. Again, EGTA was incubated 1:1 with serum. After 5 minutes incubation, the serum EGTA mixture was diluted 1/5 in the well directly.

It can be seen that varying the $MgCl_2$ content without EGTA addition has no effect on the complement activity and causes full lysis of the LPS liposomes. This is probably caused by the fact that the serum already includes physiological $Mg^{2+}$ concentrations and therefore does not need additional $Mg^{2+}$ to function properly. But as soon as EGTA is involved, rising $Mg^{2+}$ levels cause rising lysis values for the investigated concentration range. Lysis is fully blocked for $Mg^{2+}$ concentrations below the EGTA content and starts rising as the ratio $Mg^{2+}$ to EGTA increases. With the most lysis observed with 0.4 mM EGTA and 1 mM $Mg^{2+}$. These were already the most suitable conditions in the previous experiment.

In the next experiment, a more refined study between 0.2 mM and 0.4 mM is done.

Results: complement assay with EGTA. refined EGTA content study

Considering the above, another refined study of EGTA addition to serum prior to the complement assay addition was done within a concentration range of 0.2 to 0.4 mM EGTA (final concentration in well, stock solution has 4 mM) with a $Mg^{2+}$ content of 1 mM (final concentration in well, stock solution has 10 mM).

With this refined concentration range for EGTA a dose response curve could be obtained. Full lysis is seen for 0 or 0.2 mM EGTA concentrations. For 0.25 mM EGTA inactivation starts and causes a lysis value of ~90%. We assume that the classical and lectin pathway is only partially inactivated here. This seems to be right on the edge and would confirm the theoretical $Ca^{2+}$-levels of ~2.5 mM in 100% serum. In contrast, 0.3 mM EGTA already seems to cause complete inhibition of the classical and lectin pathway, similar to 0.4 mM EGTA.

Results: complement assay including EGTA. preincubation time optimization

In the examples above, 0.4 mM EGTA (final concentration in well, stock solution 10x concentrated) was determined as suitable EGTA concentration for preincubation 1:1 with serum prior to assay addition. (Buffer contains 4 mM EGTA and 10 mM $MgCl_2$ in LCB). But variations in lysis were obtained while working with the same concentration conditions. Thus, the incubation time with EGTA was investigated with further detail and was varied between 0 and 35 minutes in this study prior to assay addition.

EGTA content was mixed 1:1 with serum for different periods of time before the addition to the well. 0, 5, 10, 15, 20, 25 and 35 minutes of incubation were chosen. Final EGTA concentrations in all cases is 0.4 mM EGTA and 1

mM $MgCl_2$ in the well. Serum content in each well was 10 vol.%. Liposome concentration was 10 μM total lipid.

Results show that within the first 20 minutes of incubation of EGTA with serum (1:1), the lysis rate drops for about 10 %. After that, the lysis rate keeps constant at ~ 42% for incubation at RT until 35 minutes. It is also visible that EGTA inactivation is instant.

Overall, 0.4 mM EGTA and 1 mM $Mg^{2+}$ seem to be the most suitable conditions to investigate alternative pathway activity (Figure 35, Figure 36, and Figure 37). EGTA inactivation can be performed by addition of the respective amounts into the well directly prior to serum addition. This facilitates assay preparation and reproducibility.

The features of the present invention disclosed in the specification, the claims, and/or in the accompanying figures may, both separately and in any combination thereof, be material for realizing the invention in various forms thereof.

## Claims

**1.** Method of determining complement activity, wherein said method comprises A) determining classical complement pathway activity, B) determining alternative complement pathway activity, and/or C) determining lectin complement pathway activity; wherein

A) said determining classical complement pathway activity comprises the steps of

a) providing a sample containing or suspected of containing one or more active complement system components, optionally a serum sample, and, separately, providing one or more liposomes comprising a marker;

wherein said one or more liposomes comprise cholesterol, preferably at least 30 mol% cholesterol, more preferably at least 40 mol% cholesterol;
wherein, optionally, said one or more liposomes comprise a classical complement pathway activating agent, preferably selected from antibodies, antigens, and ligands such as biotin or streptavidin;

b) contacting said one or more liposomes with said sample to allow the one or more active complement system components, if present in said sample, to lyse said one or more liposomes; wherein a lysis of said one or more liposomes releases said marker from said one or more liposomes; and
c) detecting said released marker;

B) said determining alternative complement pathway activity comprises the steps of

a) providing a sample containing or suspected of containing one or more active complement system components, optionally a serum sample, and, separately, providing one or more liposomes comprising a marker;

wherein said one or more liposomes comprise an alternative complement pathway activating agent, preferably a lipopolysaccharide, more preferably about 0.01 mol% to about 5 mol% lipopolysaccharide, even more preferably about 1 mol% lipopolysaccharide;
wherein, optionally, said one or more liposomes comprise cholesterol, preferably ≤ 10 mol% cholesterol;

b) optionally, contacting said sample and/or said one or more liposomes with a classical complement pathway inhibitor and a lectin complement pathway inhibitor;
wherein, preferably, said classical complement pathway inhibitor and/or said lectin complement pathway inhibitor comprise(s) EGTA, C1-inhibitor, an anti-MASP antibody, and/or C4BP, preferably EGTA and/or a C1-inhibitor;
c) contacting said one or more liposomes with said sample to allow the one or more active complement system components, if present in said sample, to lyse said one or more liposomes; wherein a lysis of said one or more liposomes releases said marker from said one or more liposomes; and
d) detecting said released marker;

C) said determining lectin complement pathway activity comprises the steps of

a) providing a sample containing or suspected of containing one or more active complement system components, optionally a serum sample, and, separately, providing one or more liposomes comprising a marker;

wherein said one or more liposomes comprise a lectin complement pathway activating agent, preferably comprising a sugar moiety, an acetyl moiety and/or sialic acid;
wherein, preferably, said liposome comprises cholesterol, preferably less than 10 mol% cholesterol;

b) optionally, contacting said sample and/or said one or more liposomes with a classical complement pathway inhibitor and/or an alternative complement pathway inhibitor;
wherein, preferably, said classical complement pathway inhibitor comprises an anti-C1q85 antibody or an antigen-binding peptide thereof, and/or said alternative complement pathway inhibitor is selected from complement factor H, mini-complement factor H, an anti-factor B antibody or antigen-binding fragments thereof, an anti-properdin antibody or antigen-binding fragments thereof, and any combination thereof;
c) contacting said one or more liposomes with said sample to allow the one or more active complement system components, if present in said sample, to lyse said one or more liposomes; wherein a lysis of said one or more liposomes releases said marker from said one or more liposomes; and
d) detecting said released marker.

**2.** The method according to claim 1, wherein said A) determining classical complement pathway activity, B) determining alternative complement pathway activity, and/or C) determining lectin complement pathway activity further comprise(s):

a step of detecting a base level of said marker, optionally prior to said step of contacting said one or more liposomes with said sample, at a time point in the range of from about 1 sec to about 20 min, preferably of from about 1 sec to about 15 min, after an initial contact of said one or more liposomes with said sample, and/or using an inactivated serum and/or a buffer;
optionally, a step of determining complement activity by comparing the released marker detected in steps A)c), B)d), and C)d), respectively, with the base level of said marker detected in said step of detecting a base level of said marker, wherein, preferably, a higher level of said released marker detected in steps A)c), B)d), and C)d), respectively, compared to said base level of said marker indicates complement activity.

**3.** The method according to claim 1 or 2, wherein said detecting in step A)c), step B)d), and/or step C)d) comprises or consists of a time-resolved detection of said released marker.

**4.** The method according to any one of the foregoing claims, wherein said detecting in step A)c), step B)d), and/or step C)d) comprises or consists of a time-resolved detection of said released marker, and wherein an increase or decrease in said released marker over time, preferably an increase in said released marker over time, indicates complement activity.

**5.** The method according to any one of the foregoing claims, wherein said marker is selected from a fluorescent marker, preferably pyrenetetrasulfonic acid, sulforhodamine B, indocyanine green such as 2-[2-[2-chloro-3-[2-[1,3-dihydro-3,3-dimethyl-1-(4-sulfobutyl)-2H-indol-2-ylidene]ethylidene]-1-cyclohexen-1-yl]ethenyl]-3,3-dimethyl-1-(4-sulfobutyl)-3H-indolium, or carboxyfluorescein; an electrochemical marker, preferably potassium hexaferricyanide, potassium hexaferrocyanide, or ruthenium hexamine; a colorimetric marker, preferably sulforhodamine B; a chemiluminescent marker, preferably luminol; an electrochemiluminescent marker, preferably luminol or ruthenium bipyridyl; a bioluminescent marker such as GFP; and a combination thereof; wherein, preferably, said marker is water-soluble;

wherein, preferably, said marker is selected from a fluorescent marker, an electrochemical marker, and a combination thereof;
wherein, more preferably, said marker is selected from sulforhodamine B and luminol.

**6.** The method according to any one of the foregoing claims, wherein said marker is a fluorescent marker and wherein said marker is quenched in a nonlysed liposome;

wherein, preferably, said one or more liposomes are nonlysed liposomes prior to said lysis of said one or more liposomes;
wherein, optionally,
an increase in released marker over time detected in step(s) A)c), step B)d), and/or step C)d) indicates complement activity.

**7.** The method according to any one of the foregoing claims, wherein said one or more liposomes comprising a marker provided in step A)a) comprise a first marker, said one or more liposomes comprising a marker provided in step B)a) comprise a second marker, and said one or more liposomes comprising a marker provided in step C)a) comprise a third marker, wherein said first marker, said second marker, and said third marker are different markers.

**8.** The method according to any one of the foregoing claims, wherein said method comprises determining, preferably simultaneously or sequentially, at least two of A) determining classical complement pathway activity, B) determining alternative complement pathway activity, and C) determining lectin complement pathway activity;
wherein, preferably, said detecting said released marker of steps A)c), B)d), and/or C)d) is performed simultaneously or sequentially, preferably simultaneously.

**9.** The method according to any one of the foregoing claims, wherein

said one or more liposomes provided in step A)a) comprise cholesterol and phospholipids; preferably comprise cholesterol, 1,2-dipalmitoyl-sn-glycero-3-phosphocholine, and 1,2-dipalmitoyl-sn-glycero-3-phospho-(1'-rac-glycerol); more preferably comprise at least 30 mol% cholesterol, preferably at least 40 mol% cholesterol, 1,2-dipalmitoyl-sn-glycero-3-phosphocholine in the range of from 10 mol% to 30 mol%, and 1,2-dipalmitoyl-sn-glycero-3-phospho-(1'-rac-glycerol) in the range of from 25 mol% to 45 mol%;
wherein, optionally, said one or more liposomes provided in step A)a) further comprise a classical complement pathway activating agent, preferably selected from antibodies, antigens, and ligands such as biotin or streptavidin;
said one or more liposomes provided in step B)a) comprise phospholipids and cholesterol, preferably ≤ 10 mol% cholesterol; preferably comprise 1,2-dipalmitoyl-sn-glycero-3-phosphocholine, 1,2-dipalmitoyl-sn-glycero-3-phospho-(1'-rac-glycerol), and ≤ 10 mol% cholesterol; more preferably comprise 1,2-dipalmitoyl-sn-glycero-3-phosphocholine in the range of from 65 mol% to 85 mol%, 1,2-dipalmitoyl-sn-glycero-3-phospho-(1'-rac-glycerol) in the range of from 10 mol% to 30 mol%, and ≤ 10 mol% cholesterol; and/or
said one or more liposomes provided in step C)a) comprise phospholipids and ≤ 10 mol% cholesterol; preferably comprise 1,2-dipalmitoyl-sn-glycero-3-phosphocholine, 1,2-dipalmitoyl-sn-glycero-3-phospho-(1'-rac-glycerol), and ≤ 10 mol% cholesterol; more preferably comprise 1,2-dipalmitoyl-sn-glycero-3-phosphocholine in the range of from 65 mol% to 85 mol%, 1,2-dipalmitoyl-sn-glycero-3-phospho-(1'-rac-glycerol) in the range of from 10 mol% to 30 mol%, and ≤ 10 mol% cholesterol;
wherein, optionally, said one or more liposomes provided in step A)a), step B)a), and/or C)a) comprise 1,2-dipalmitoyl-sn-glycero-3-ethylphosphocholine;
wherein, optionally, said one or more liposomes provided in step B)a) and/or said one or more liposomes provided in step C)a) comprise a classical complement pathway inhibitor, preferably an anti-C1q85 antibody or an antigen-binding fragment thereof.

**10.** The method according to any one of the foregoing claims, wherein said one or more liposomes provided in step A)a), said one or more liposomes provided in step B)a), and/or said one or more liposomes provided in step C)a) are selected from anionic liposomes, PEGylated liposomes, and cationic liposomes, preferably anionic liposomes.

**11.** The method according to any one of the foregoing claims, wherein step B)b) comprises contacting said sample and/or said one or more liposomes with a classical complement pathway inhibitor and/or a lectin complement pathway inhibitor selected from $Ca^{2+}$ complexing agents, preferably EGTA, in the presence of $Mg^{2+}$, preferably in the presence of at least 0.001 mM $MgCl_2$, more preferably in the presence of at least 0.1 mM $MgCl_2$, even more preferably in the presence of at least 0.5 mM $MgCl_2$, e.g. of about 1 mM $MgCl_2$.

**12.** The method according to any one of the foregoing claims, wherein said method comprises a positive control and/or a negative control;

wherein said positive control comprises contacting said one or more liposomes provided in step A)a), B)a), and/or C)a) with a surfactant and/or solvent, preferably a surfactant, more preferably a surfactant selected from n-octyl-β-d-glucoside, 2-[4-(2,4,4-trimethylpentan-2-yl)phenoxy]ethanol, polyoxyethylene (20) sorbitan monolaurate, and polyoxyethylene (80) sorbitan monooleate; and
wherein said negative control comprises replacing said sample, optionally said serum sample, in step A)a), B)a), and/or C)a) with a negative control sample which comprises or consists of inactive serum.

**13.** Liposome collection for determining complement activity, preferably for A) determining classical complement pathway activity, B) determining alternative complement pathway activity, and/or C) determining lectin complement pathway activity, comprising:

- A) a liposome for determining classical complement pathway activity, comprising a marker, cholesterol, preferably at least 30 mol% cholesterol, more preferably at least 40 mol% cholesterol, and phospholipids;

preferably comprising a marker, cholesterol, 1,2-dipalmitoyl-sn-glycero-3-phosphocholine, and 1,2-dipalmitoyl-sn-glycero-3-phospho-(1'-rac-glycerol);
more preferably comprising a marker, at least 30 mol% cholesterol, preferably at least 40 mol% cholesterol, 1,2-dipalmitoyl-sn-glycero-3-phosphocholine in the range of from 10 mol% to 30 mol%, and 1,2-dipalmitoyl-sn-glycero-3-phospho-(1'-rac-glycerol) in the range of from 25 mol% to 45 mol%;
wherein, optionally, said liposome further comprises a classical complement pathway activating agent, preferably selected from antibodies, antigens, and ligands such as biotin or streptavidin;

- B) a liposome for determining alternative complement pathway activity comprising a marker, an alternative complement pathway activating agent, cholesterol, preferably ≤ 10 mol% cholesterol, and phospholipids;

preferably comprising a marker, an alternative complement pathway activating agent, cholesterol, 1,2-dipalmitoyl-sn-glycero-3-phosphocholine, and 1,2-dipalmitoyl-sn-glycero-3-phospho-(1'-rac-glycerol);
more preferably comprising a marker, an alternative complement pathway activating agent, ≤ 10 mol% cholesterol, 1,2-dipalmitoyl-sn-glycero-3-phosphocholine in the range of from 65 mol% to 85 mol%, and 1,2-dipalmitoyl-sn-glycero-3-phospho-(1'-rac-glycerol) in the range of from 10 mol% to 30 mol%;
wherein, preferably, said alternative complement pathway activating agent is a lipopolysaccharide, preferably about 0.01 mol% to about 5 mol% lipopolysaccharide, more preferably about 1 mol% lipopolysaccharide; and/or

- C) a liposome for determining lectin complement pathway activity, comprising a marker, a lectin complement pathway activating agent, cholesterol, preferably ≤ 10 mol% cholesterol, and phospholipids;

preferably comprising a marker, a lectin complement pathway activating agent, cholesterol, 1,2-dipalmitoyl-sn-glycero-3-phosphocholine, and 1,2-dipalmitoyl-sn-glycero-3-phospho-(1'-rac-glycerol);
more preferably comprising a marker, a lectin complement pathway activating agent, ≤ 10 mol% cholesterol, 1,2-dipalmitoyl-sn-glycero-3-phosphocholine in the range of from 65 mol% to 85 mol%, and 1,2-dipalmitoyl-sn-glycero-3-phospho-(1'-rac-glycerol) in the range of from 10 mol% to 30 mol%;
wherein, preferably, said lectin complement pathway activating agent comprises a sugar moiety, an acetyl moiety, and/or sialic acid;

wherein, optionally, the liposome for determining alternative complement pathway activity B) and/or the liposome for determining lectin complement pathway activity C) comprise(s) a classical complement pathway inhibitor, preferably an anti-C1q85 antibody or an antigen-binding fragment thereof;
wherein, preferably, said collection comprises A) and B), A) and C), B) and C), or A), B), and C).

**14.** Kit for determining complement activity, comprising

- a liposome collection according to claim 13,
- a buffer, preferably a liposome complement buffer;
- optionally, a classical complement pathway inhibitor, an alternative complement pathway inhibitor, and/or a lectin complement pathway inhibitor; preferably EGTA, C1-inhibitor, an anti-MASP antibody, and/or C4BP; more preferably EGTA and/or a C1-inhibitor;
- optionally, an inactivated serum; wherein, preferably, said serum is inactivated by EDTA, EGTA, heat, ultrasound, and/or a solvent such as a water-soluble organic solvent;
- optionally, a surfactant, preferably n-octyl-β-d-glucoside;
- optionally, an osmolality agent, preferably a sucrose solution;
- optionally, instructions for determining complement activity, preferably instructions for determining classical complement pathway activity, alternative complement pathway activity, and/or lectin complement pathway activity; more preferably instructions for a method according to any one of claims 1-12.

**15.** Liposome collection according to claim 13 or kit according to claim 14 for use in a method of diagnosing, monitoring, or treating a complement associated disorder, preferably a complement associated disease; wherein, optionally, said complement associated disease is selected from eye diseases, sepsis, kidney diseases, infectious diseases, primary deficiencies in complement genes, and autoimmune diseases, preferably selected from systemic lupus erythematosus, rheumatoid arthritis, hereditary angioedema, meningococcal disease, a disease associated with anti-factor H antibodies, recurrent pyogenic infections, haemolytic uremic syndrome, paroxysmal nocturnal hemoglobinuria, neuromyelitis optica disorder diseases, atypical hemolytic uremic syndrome, and C3 glomerulopathy.

Figure 1

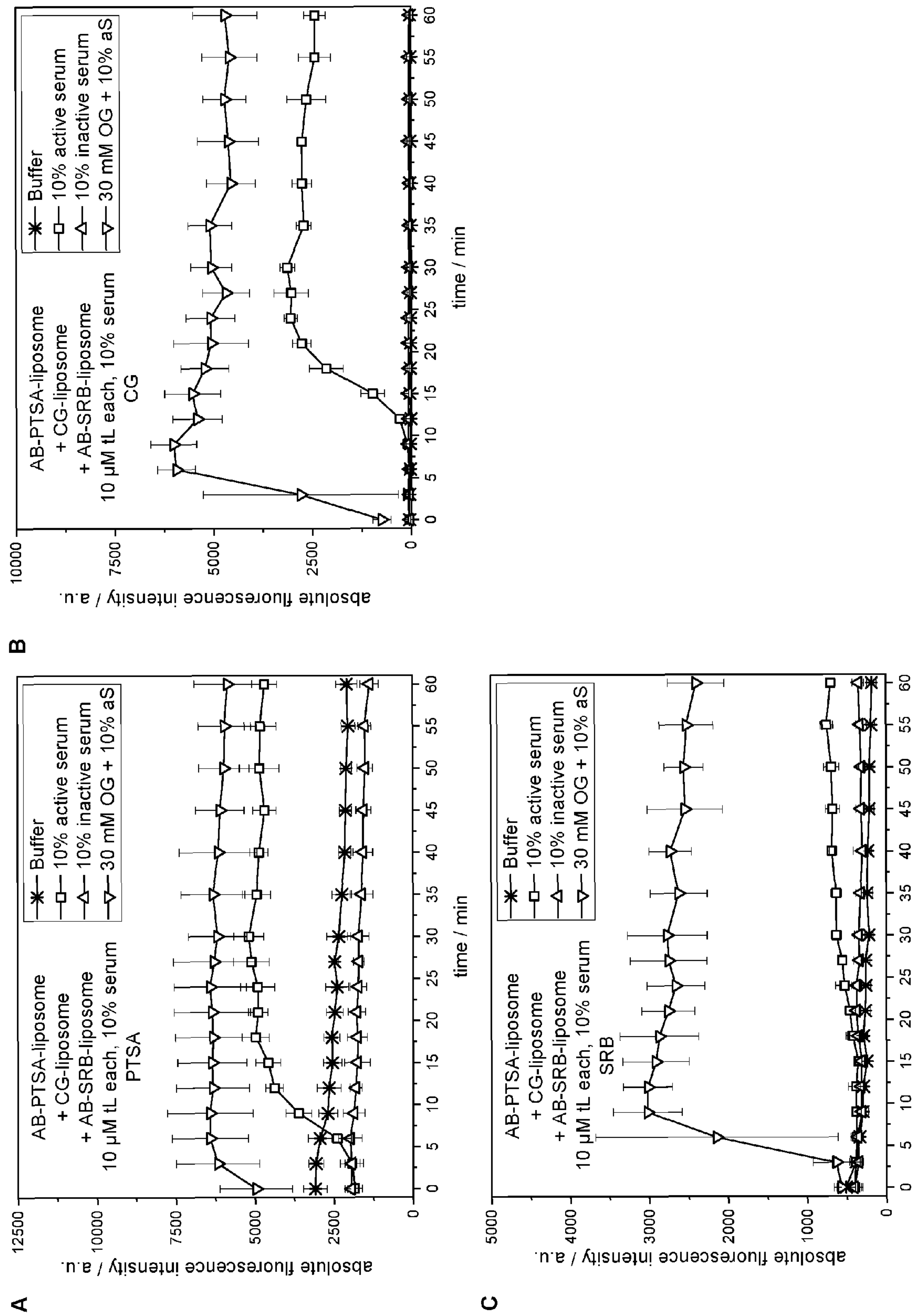
A
B
C
AB-PTSA-liposome
+ CG-liposome
+ AB-SRB-liposome
10 µM tL each, 10% serum
PTSA
CG
SRB
Buffer
10% active serum
10% inactive serum
30 mM OG + 10% aS
absolute fluorescence intensity / a.u.
time / min

Figure 2

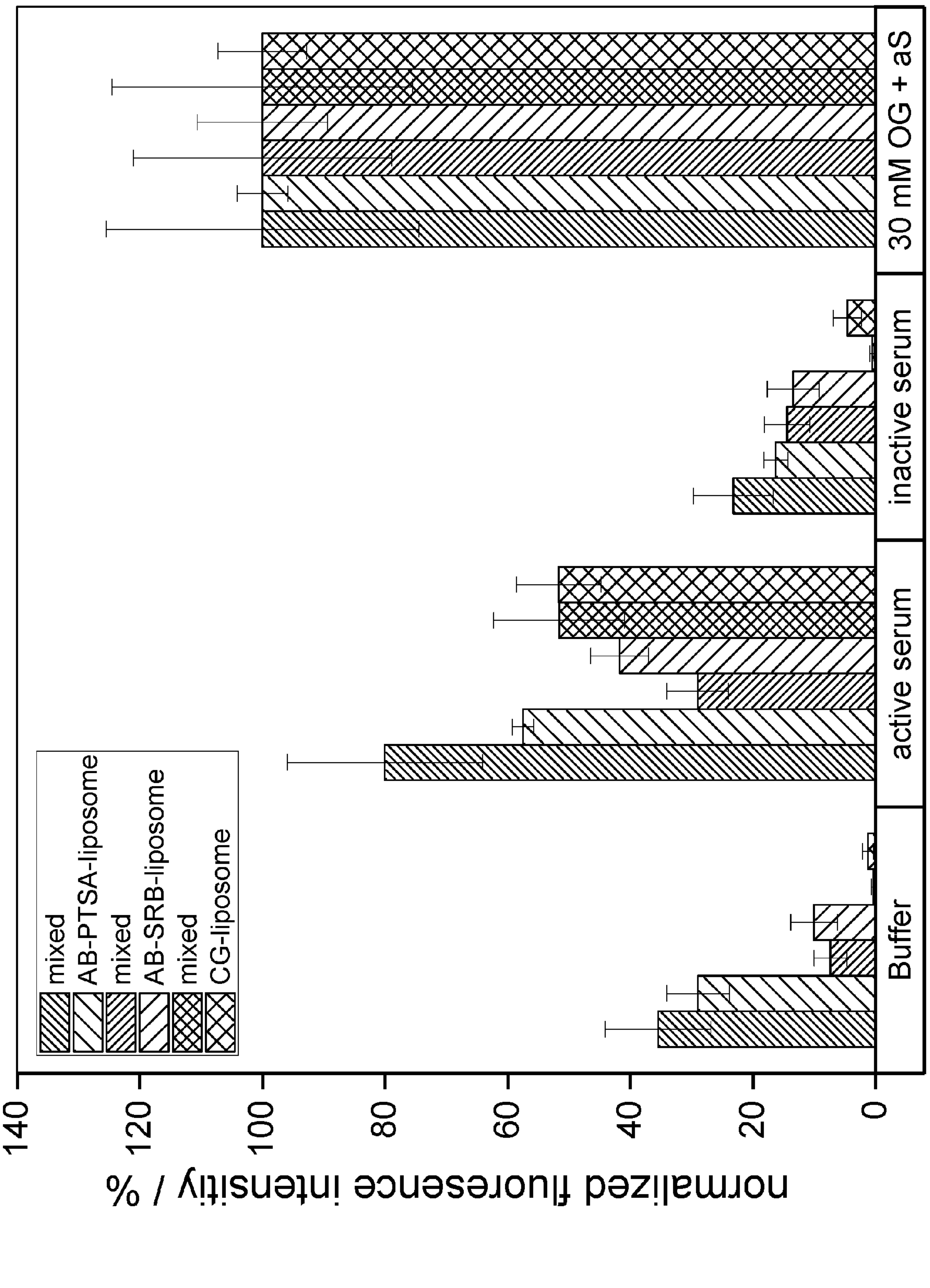
normalized fluoresence intensity / %
0
20
40
60
80
100
120
140
mixed
AB-PTSA-liposome
mixed
AB-SRB-liposome
mixed
CG-liposome
Buffer
active serum
inactive serum
30 mM OG + aS

Figure 3

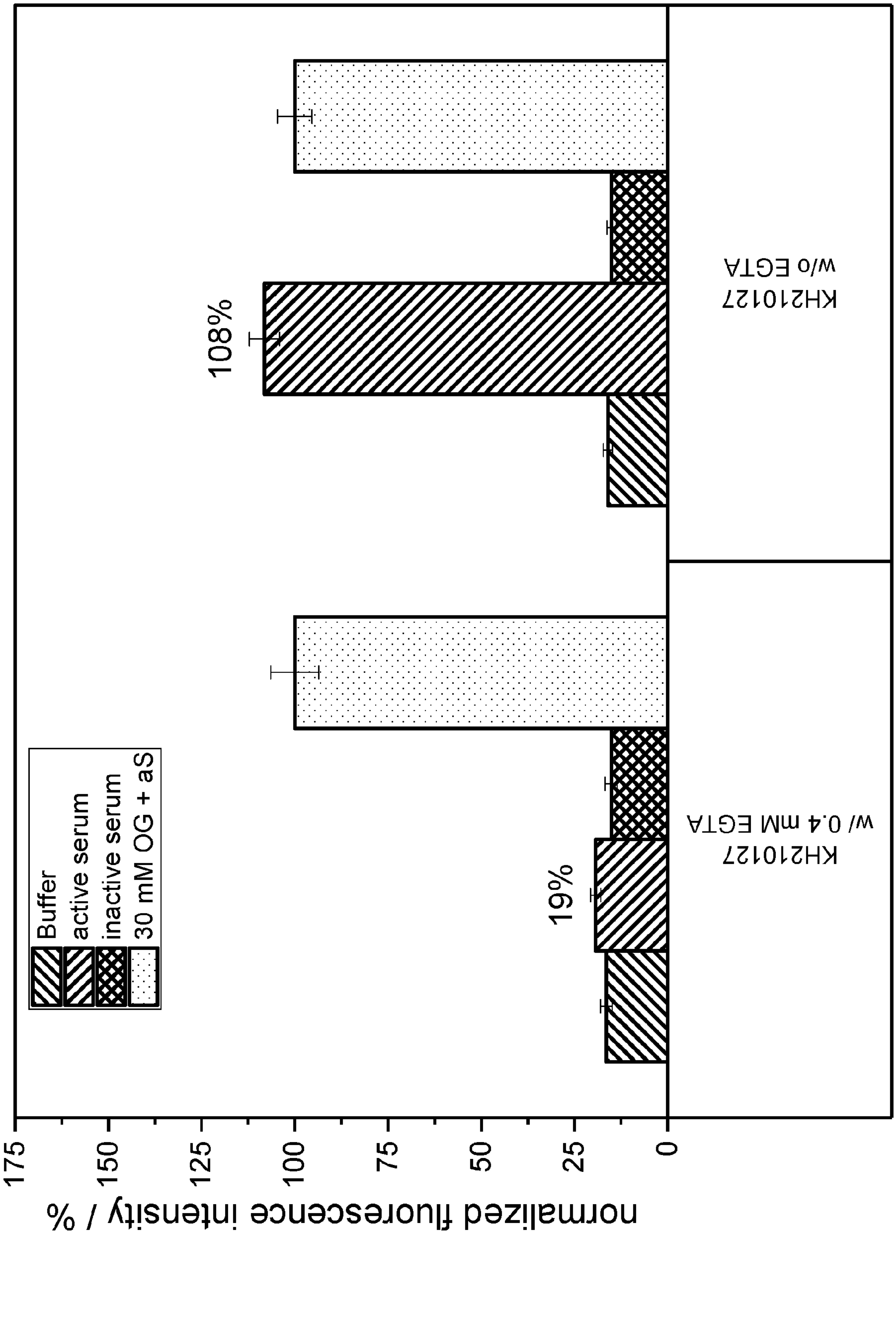
normalized fluorescence intensity / %
175
150
125
100
75
50
25
0
Buffer
active serum
inactive serum
30 mM OG + aS
19%
108%
KH210127
w/ 0.4 mM EGTA
KH210127
w/o EGTA

# Figure 4

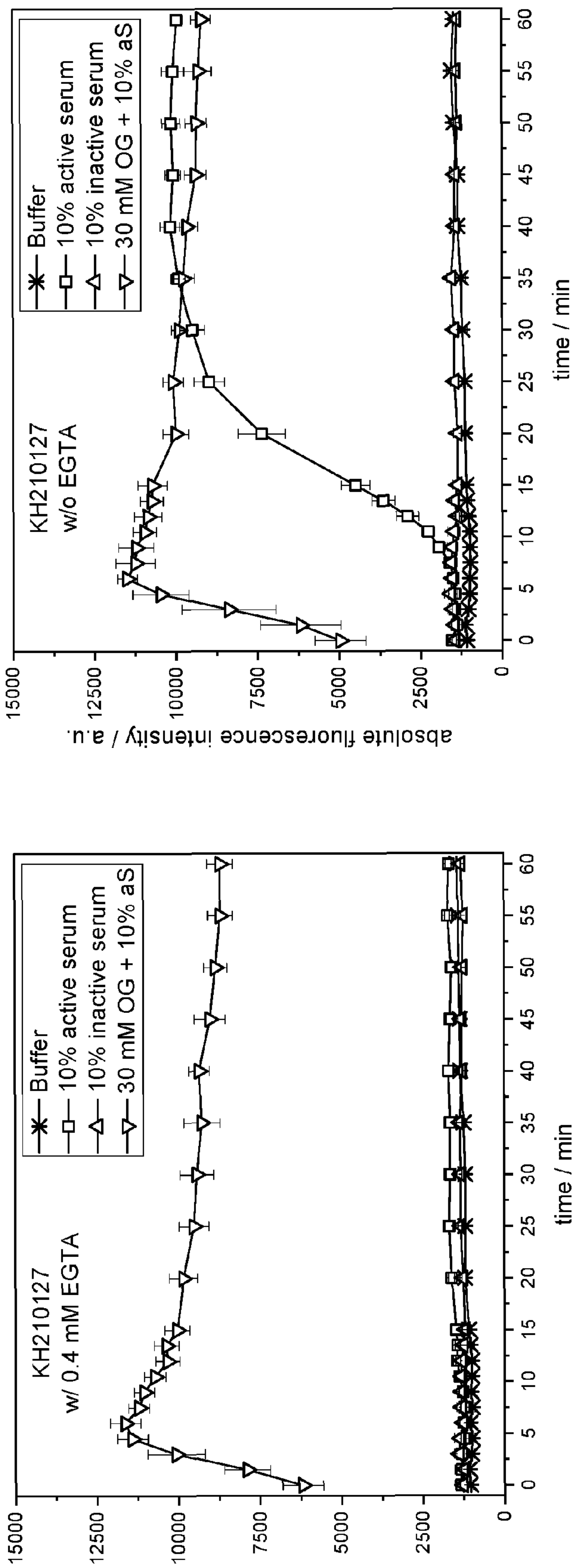
KH210127
w/o EGTA
Buffer
10% active serum
10% inactive serum
30 mM OG + 10% aS
absolute fluorescence intensity / a.u.
time / min
KH210127
w/ 0.4 mM EGTA
Buffer
10% active serum
10% inactive serum
30 mM OG + 10% aS
absolute fluorescence intensity / a.u.
time / min

Figure 5

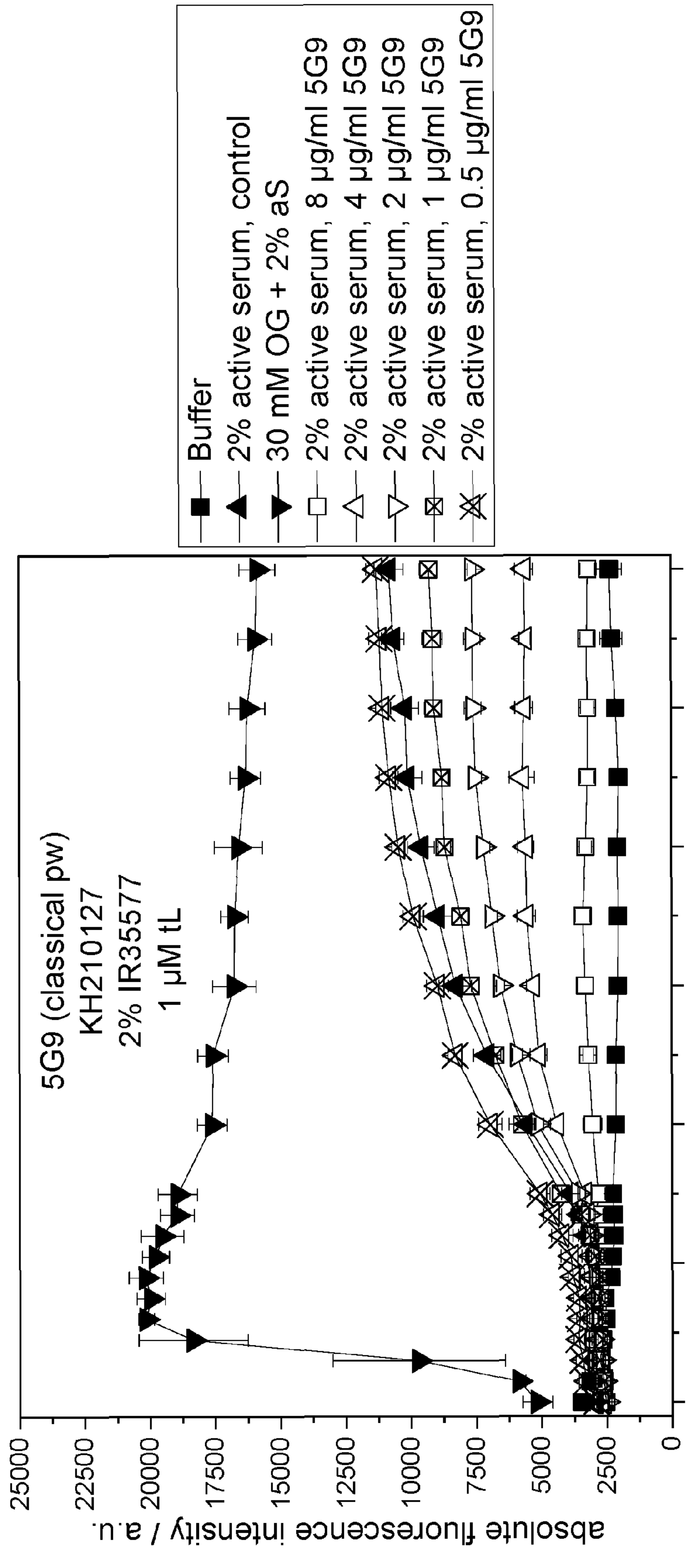
5G9 (classical pw)
KH210127
2% IR35577
1 µM tL
absolute fluorescence intensity / a.u.
time / min
Buffer
2% active serum, control
30 mM OG + 2% aS
2% active serum, 8 µg/ml 5G9
2% active serum, 4 µg/ml 5G9
2% active serum, 2 µg/ml 5G9
2% active serum, 1 µg/ml 5G9
2% active serum, 0.5 µg/ml 5G9

Figure 6

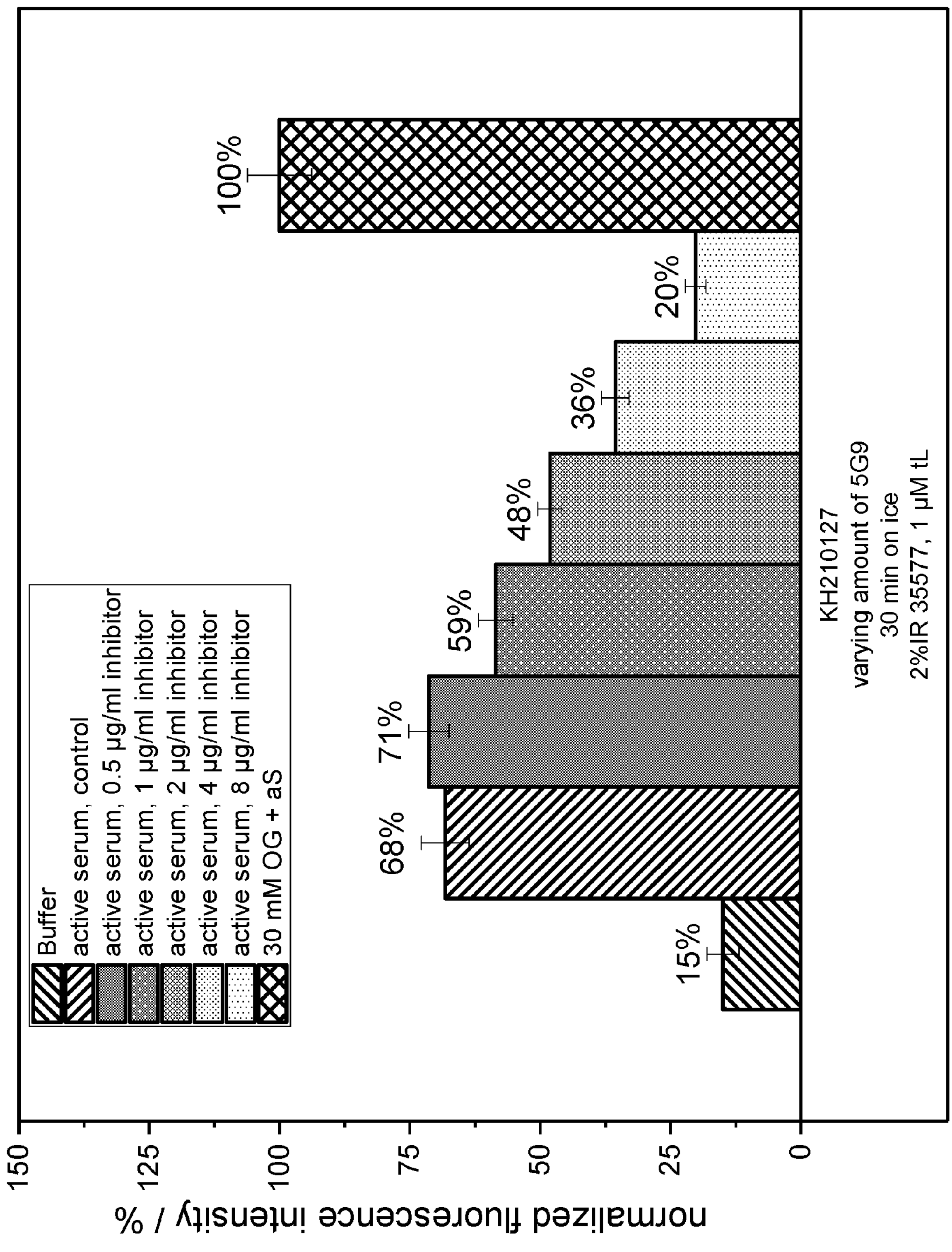
normalized fluorescence intensity / %
0
25
50
75
100
125
150
Buffer
active serum, control
active serum, 0.5 µg/ml inhibitor
active serum, 1 µg/ml inhibitor
active serum, 2 µg/ml inhibitor
active serum, 4 µg/ml inhibitor
active serum, 8 µg/ml inhibitor
30 mM OG + aS
15%
68%
71%
59%
48%
36%
20%
100%
KH210127
varying amount of 5G9
30 min on ice
2%IR 35577, 1 µM tL

**Figure 7**

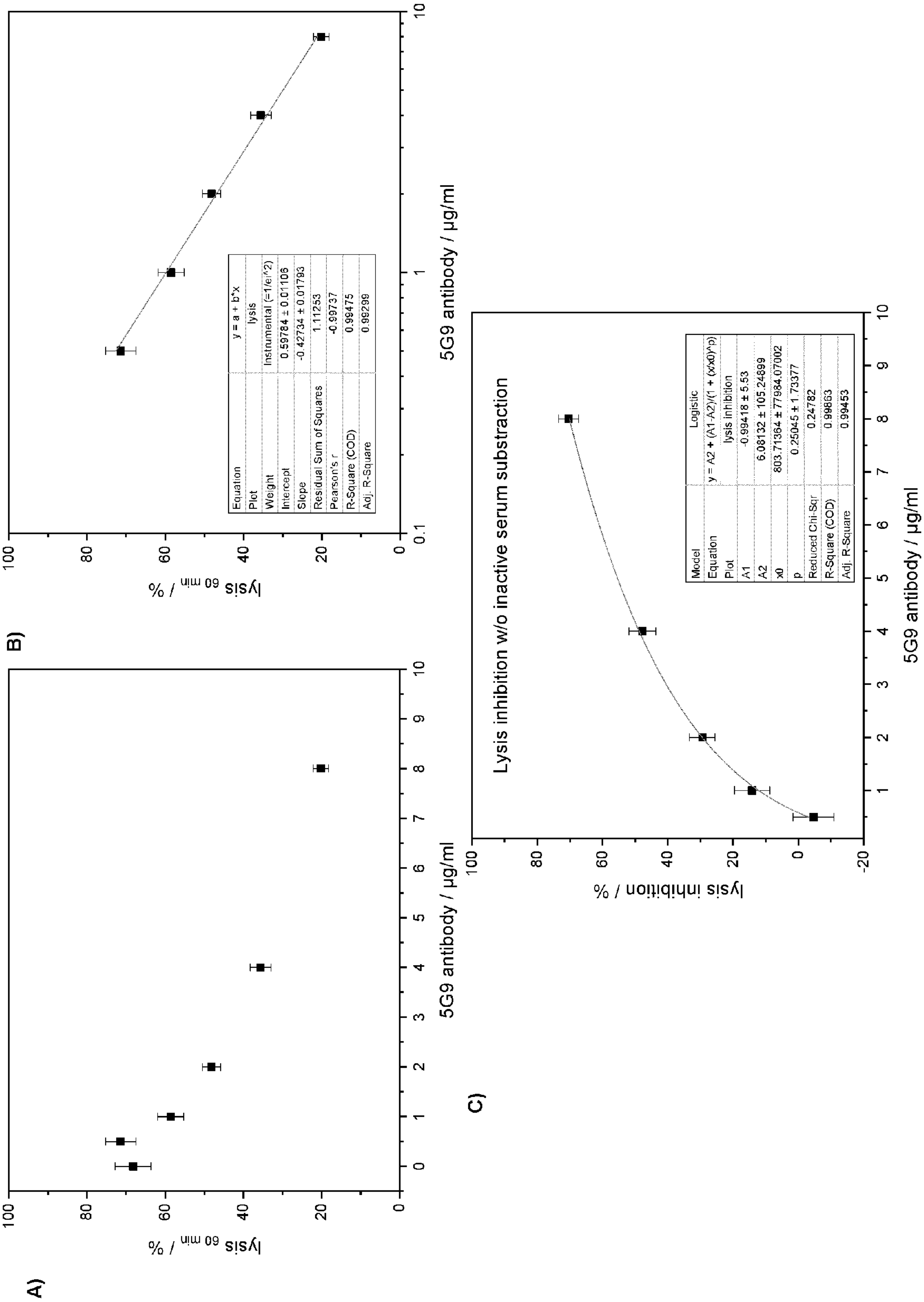
A)
lysis 60 min / %
5G9 antibody / µg/ml
B)
lysis 60 min / %
5G9 antibody / µg/ml
Equation
y = a + b*x
Plot
lysis
Weight
Instrumental (=1/ei^2)
Intercept
0.59784 ± 0.01106
Slope
-0.42734 ± 0.01793
Residual Sum of Squares
1.11253
Pearson's r
-0.99737
R-Square (COD)
0.99475
Adj. R-Square
0.99299
C)
Lysis inhibition w/o inactive serum substraction
lysis inhibition / %
5G9 antibody / µg/ml
Model
Logistic
Equation
y = A2 + (A1-A2)/(1 + (x/x0)^p)
Plot
lysis inhibition
A1
-0.99418 ± 5.53
A2
6.08132 ± 105.24899
x0
803.71364 ± 77984.07002
p
0.25045 ± 1.73377
Reduced Chi-Sqr
0.24782
R-Square (COD)
0.99863
Adj. R-Square
0.99453

Figure 8

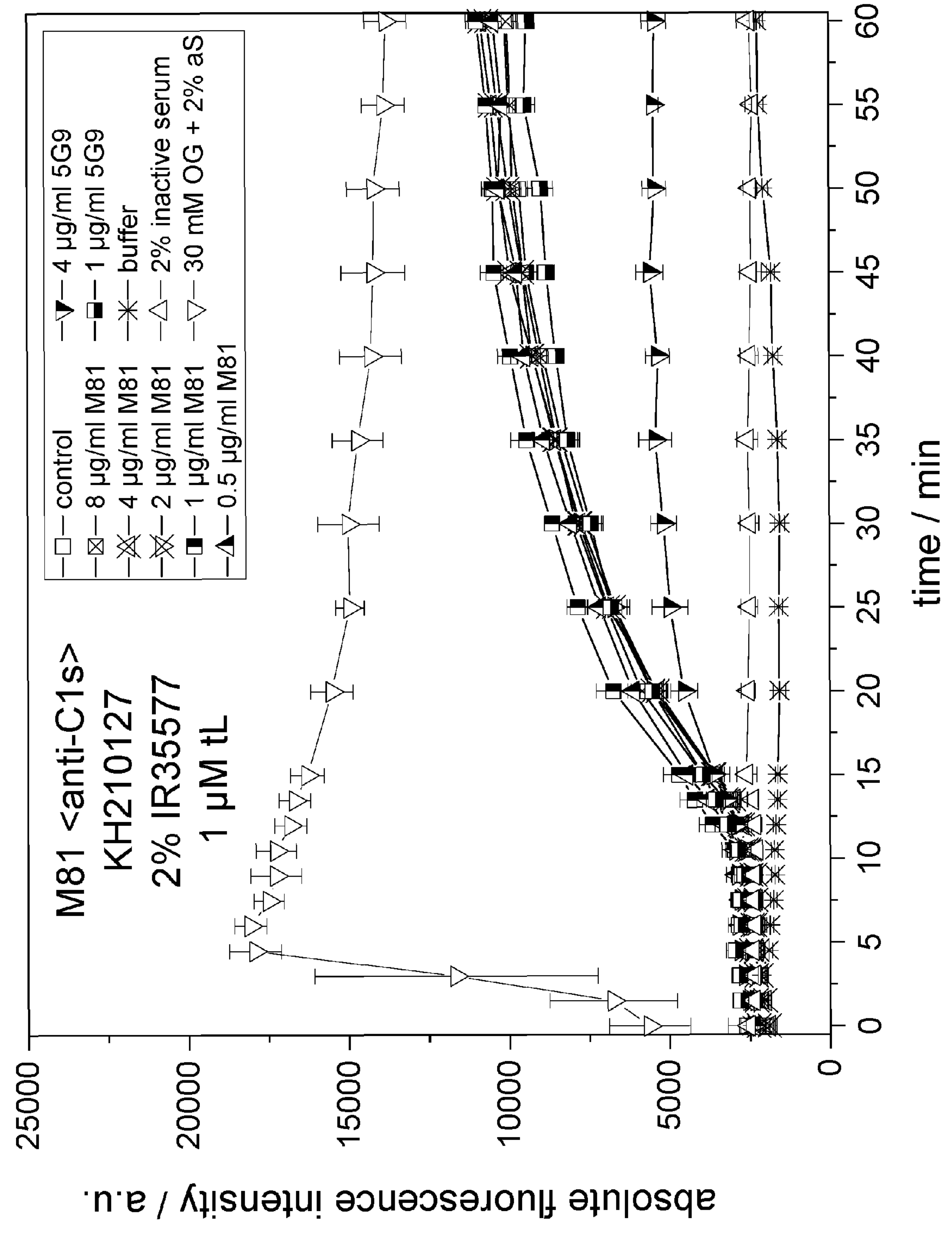
M81 <anti-C1s>
KH210127
2% IR35577
1 µM tL
control
8 µg/ml M81
4 µg/ml M81
2 µg/ml M81
1 µg/ml M81
0.5 µg/ml M81
4 µg/ml 5G9
1 µg/ml 5G9
buffer
2% inactive serum
30 mM OG + 2% aS
absolute fluorescence intensity / a.u.
time / min
0
5000
10000
15000
20000
25000
0
5
10
15
20
25
30
35
40
45
50
55
60

Figure 9

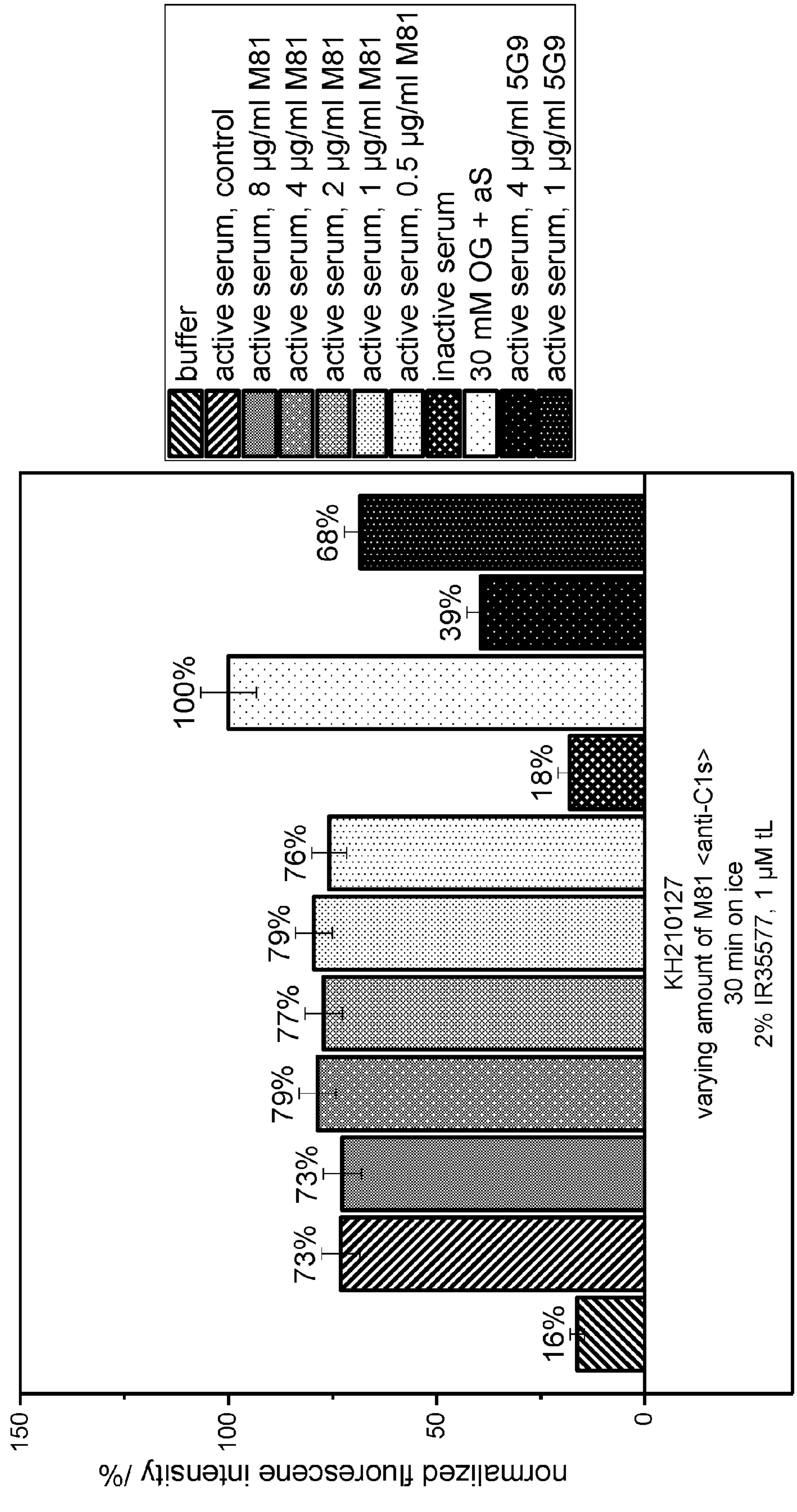
buffer
active serum, control
active serum, 8 µg/ml M81
active serum, 4 µg/ml M81
active serum, 2 µg/ml M81
active serum, 1 µg/ml M81
active serum, 0.5 µg/ml M81
inactive serum
30 mM OG + aS
active serum, 4 µg/ml 5G9
active serum, 1 µg/ml 5G9
16%
73%
73%
79%
77%
79%
76%
18%
100%
39%
68%
KH210127
varying amount of M81 <anti-C1s>
30 min on ice
2% IR35577, 1 µM tL
normalized fluorescene intensity /%
150
100
50
0

Figure 10

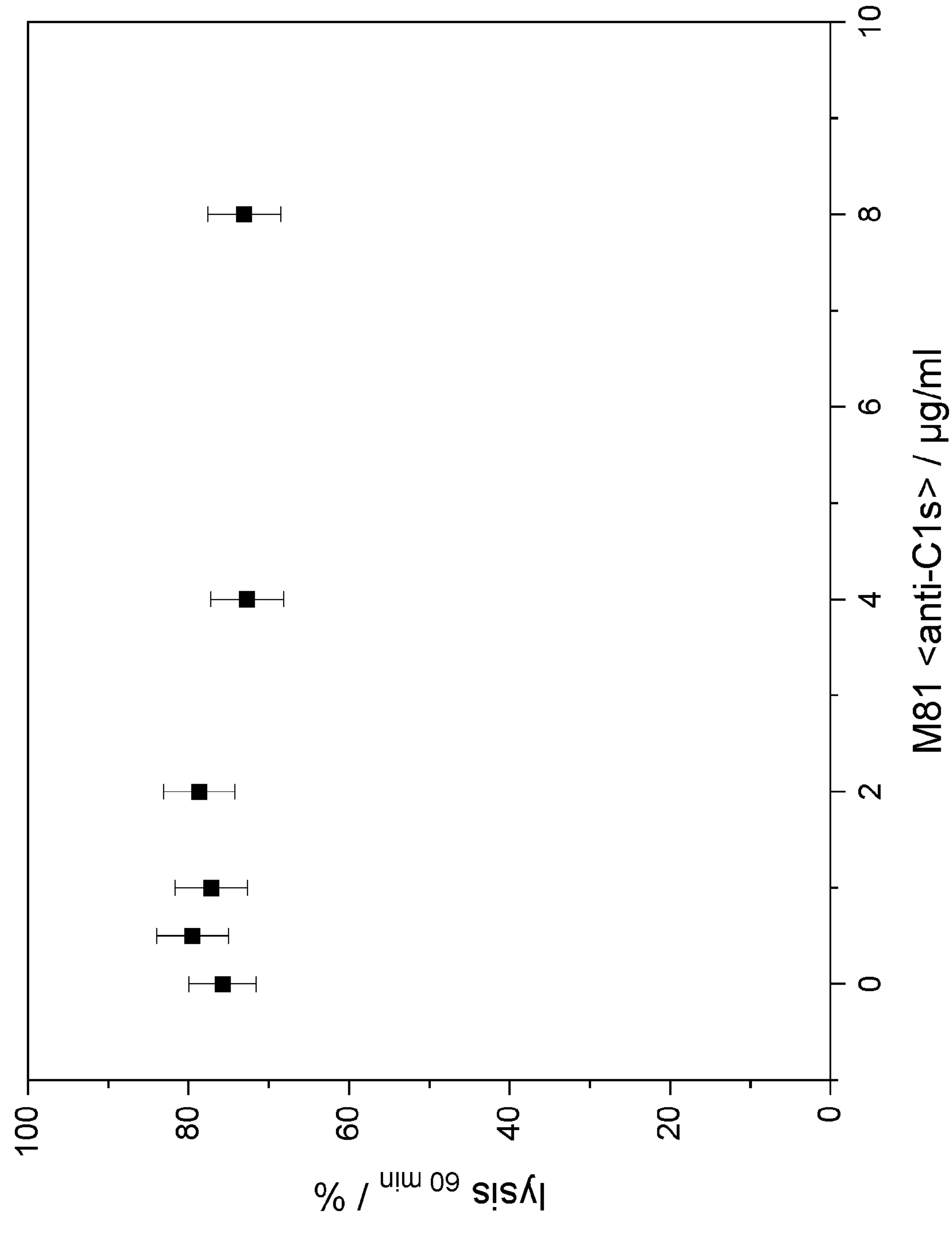

Figure 11

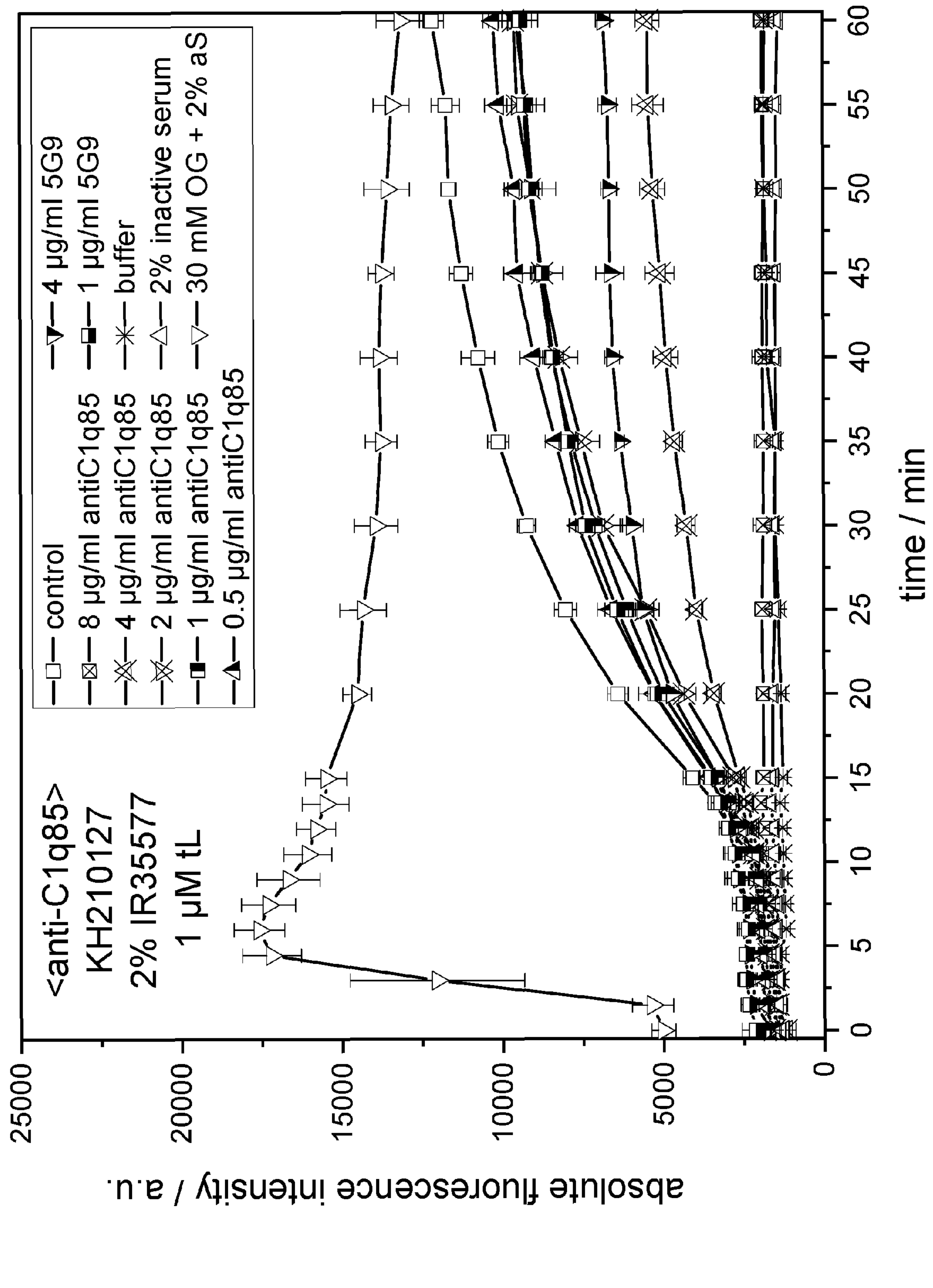
<anti-C1q85>
KH210127
2% IR35577
1 µM tL
control
8 µg/ml antiC1q85
4 µg/ml antiC1q85
2 µg/ml antiC1q85
1 µg/ml antiC1q85
0.5 µg/ml antiC1q85
4 µg/ml 5G9
1 µg/ml 5G9
buffer
2% inactive serum
30 mM OG + 2% aS
absolute fluorescence intensity / a.u.
time / min
0
5000
10000
15000
20000
25000
0
5
10
15
20
25
30
35
40
45
50
55
60

Figure 12

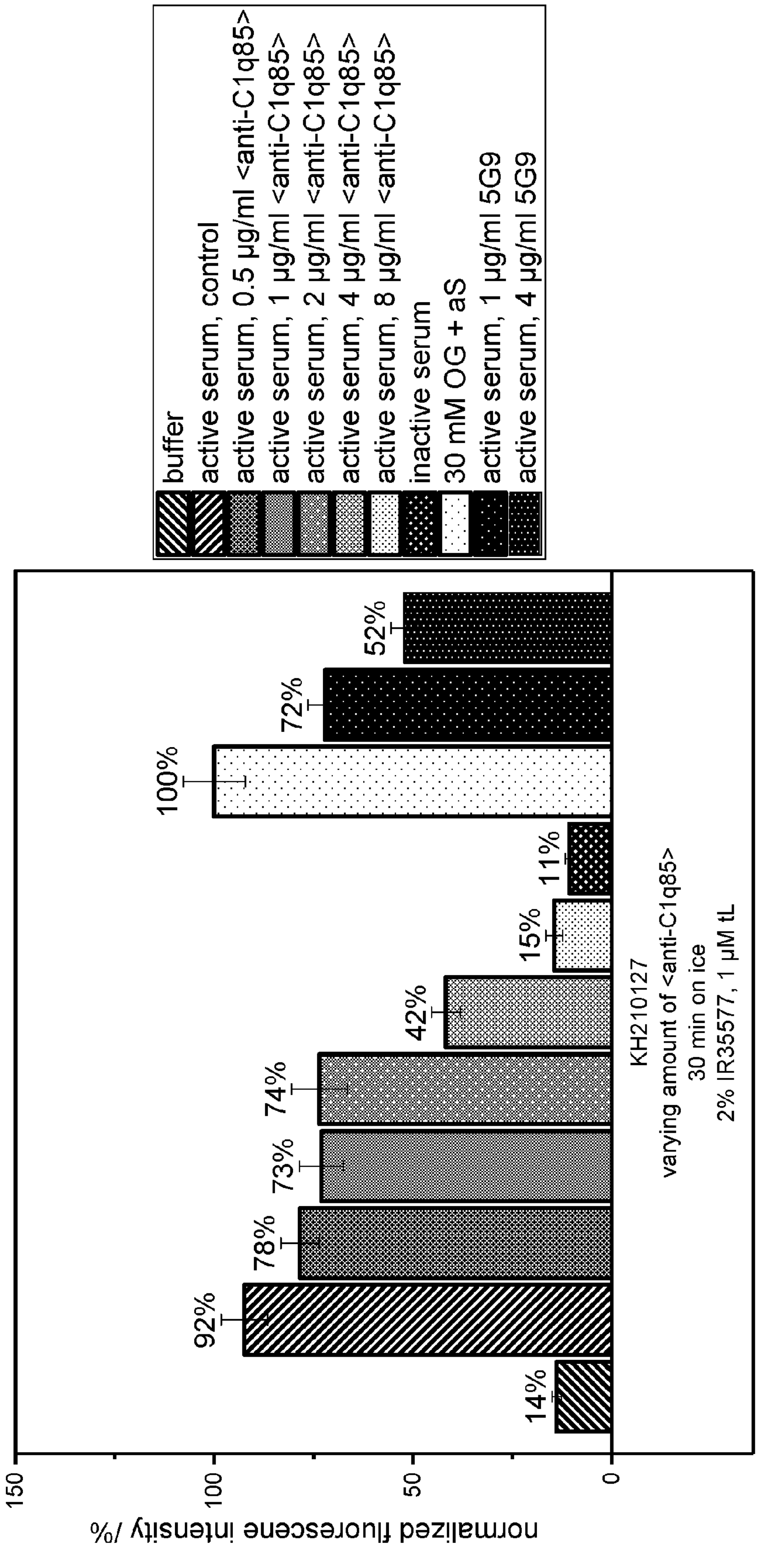
buffer
active serum, control
active serum, 0.5 µg/ml <anti-C1q85>
active serum, 1 µg/ml <anti-C1q85>
active serum, 2 µg/ml <anti-C1q85>
active serum, 4 µg/ml <anti-C1q85>
active serum, 8 µg/ml <anti-C1q85>
inactive serum
30 mM OG + aS
active serum, 1 µg/ml 5G9
active serum, 4 µg/ml 5G9
14%
92%
78%
73%
74%
42%
15%
11%
100%
72%
52%
KH210127
varying amount of <anti-C1q85>
30 min on ice
2% IR35577, 1 µM tL
normalized fluorescene intensity /%
150
100
50
0

Figure 13

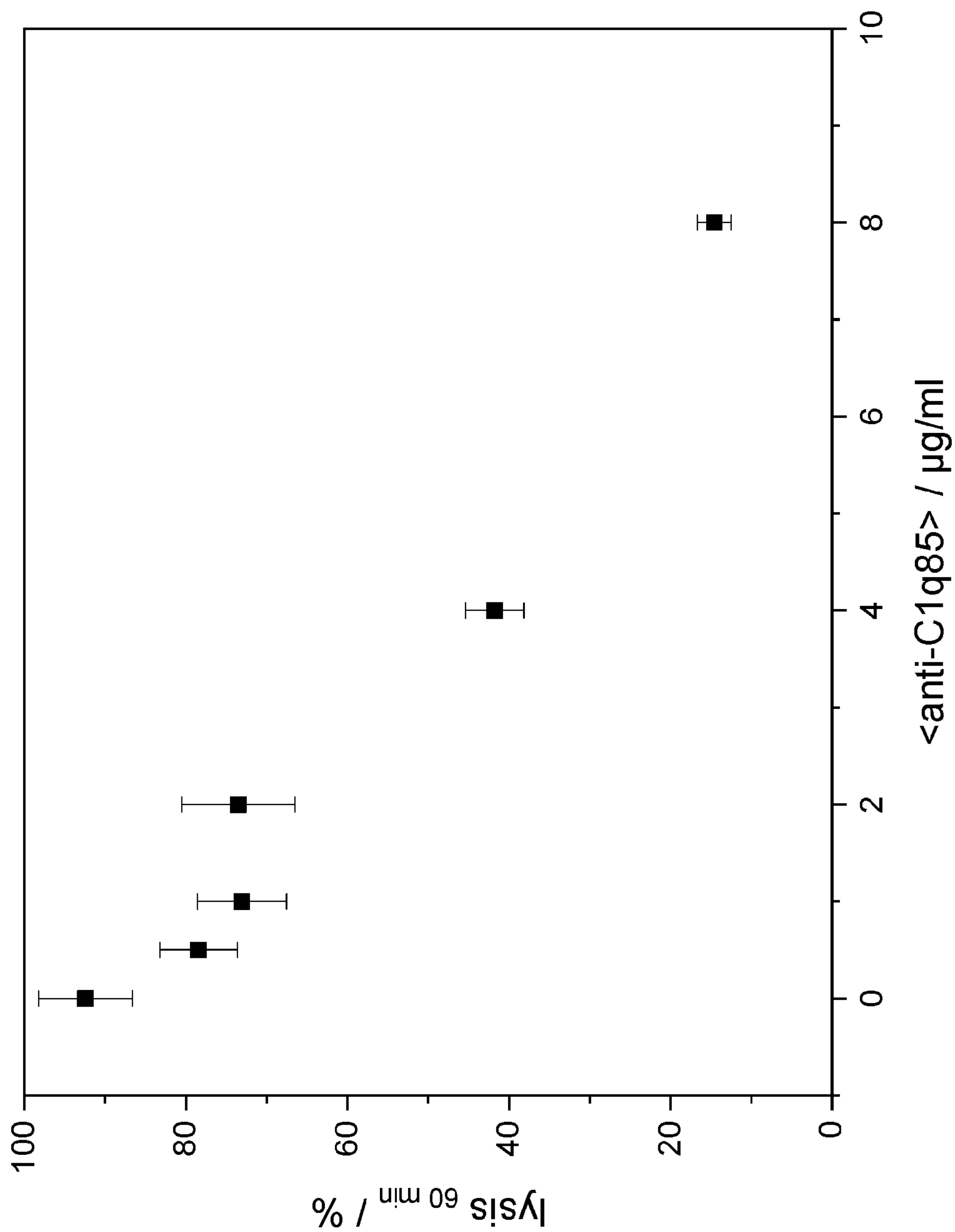

Figure 14

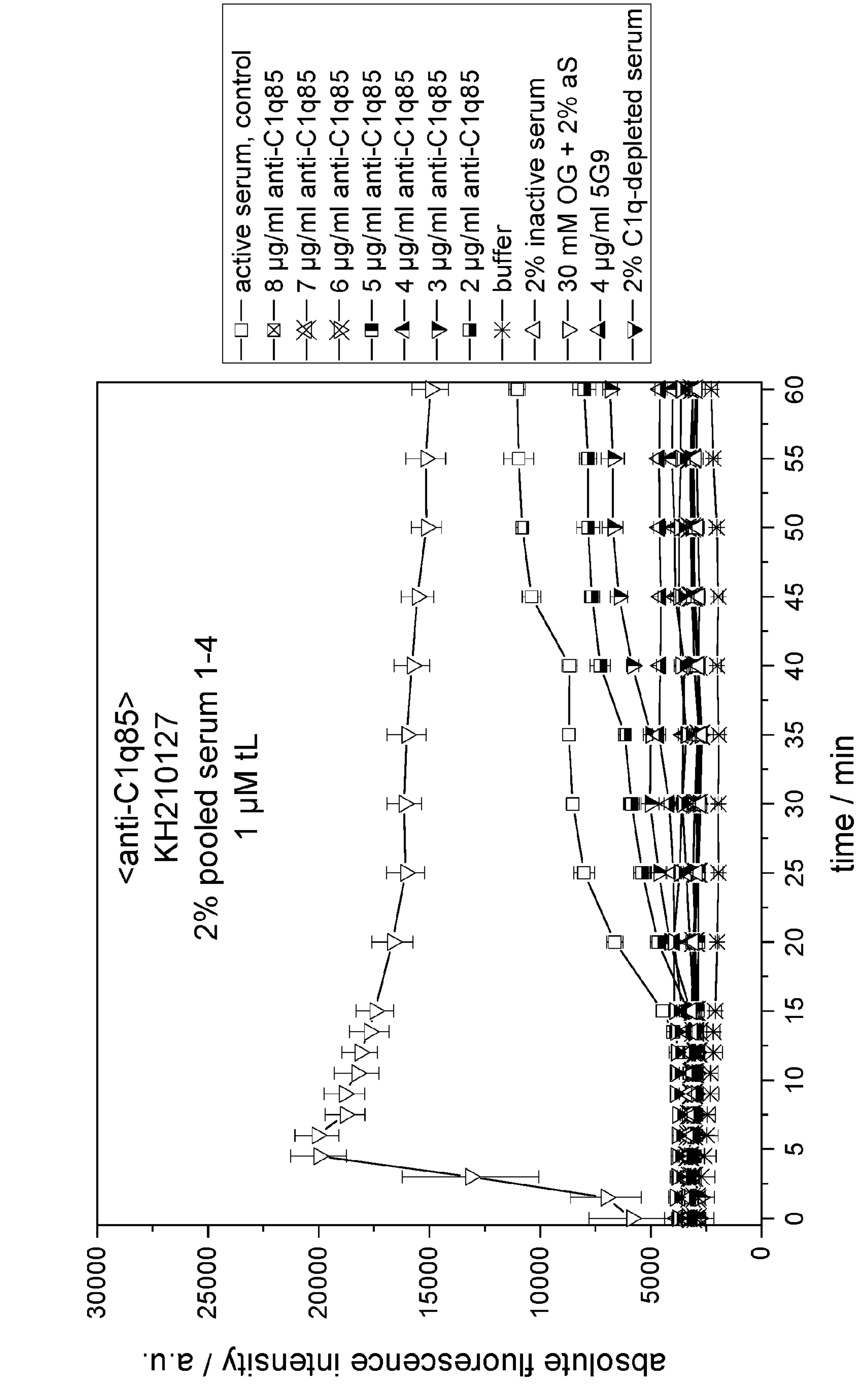
<anti-C1q85>
KH210127
2% pooled serum 1-4
1 µM tL
absolute fluorescence intensity / a.u.
time / min
0
5000
10000
15000
20000
25000
30000
0
5
10
15
20
25
30
35
40
45
50
55
60
active serum, control
8 µg/ml anti-C1q85
7 µg/ml anti-C1q85
6 µg/ml anti-C1q85
5 µg/ml anti-C1q85
4 µg/ml anti-C1q85
3 µg/ml anti-C1q85
2 µg/ml anti-C1q85
buffer
2% inactive serum
30 mM OG + 2% aS
4 µg/ml 5G9
2% C1q-depleted serum

Figure 15

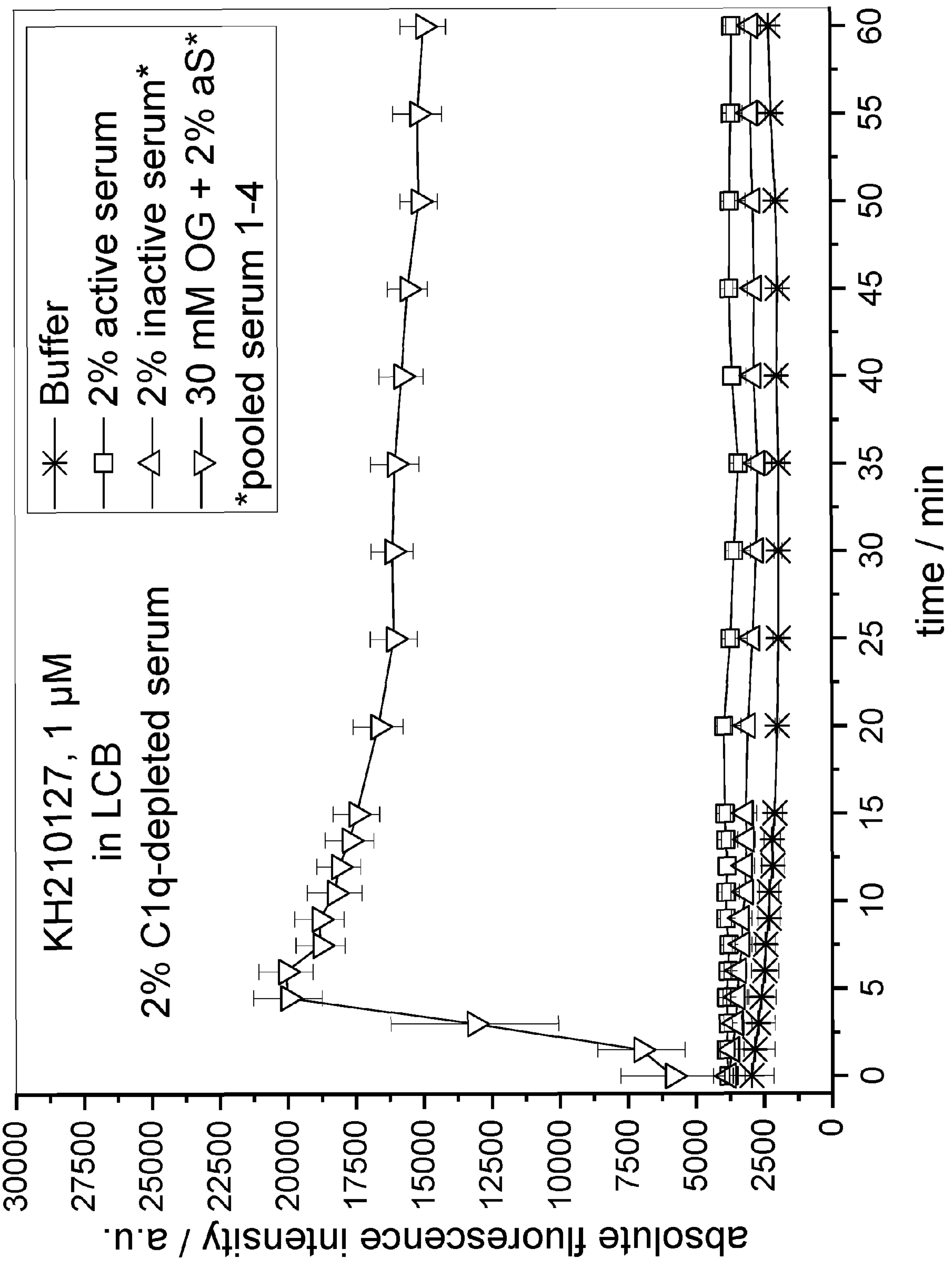
KH210127, 1 µM
in LCB
2% C1q-depleted serum
Buffer
2% active serum
2% inactive serum*
30 mM OG + 2% aS*
*pooled serum 1-4
absolute fluorescence intensity / a.u.
time / min
0
2500
5000
7500
10000
12500
15000
17500
20000
22500
25000
27500
30000
0
5
10
15
20
25
30
35
40
45
50
55
60

# Figure 16

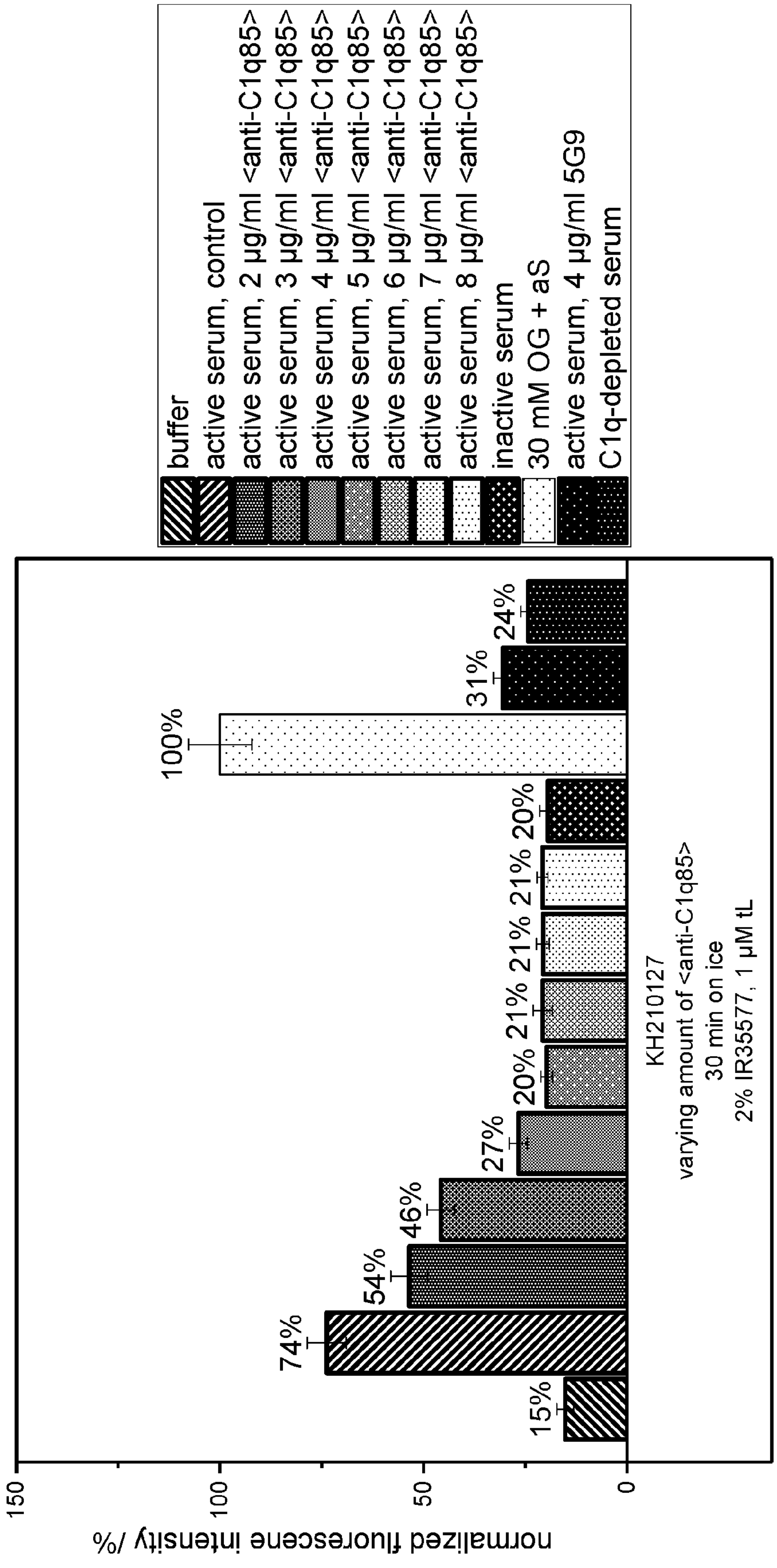
buffer
active serum, control
active serum, 2 µg/ml <anti-C1q85>
active serum, 3 µg/ml <anti-C1q85>
active serum, 4 µg/ml <anti-C1q85>
active serum, 5 µg/ml <anti-C1q85>
active serum, 6 µg/ml <anti-C1q85>
active serum, 7 µg/ml <anti-C1q85>
active serum, 8 µg/ml <anti-C1q85>
inactive serum
30 mM OG + aS
active serum, 4 µg/ml 5G9
C1q-depleted serum
15%
74%
54%
46%
27%
20%
21%
21%
21%
20%
100%
31%
24%
KH210127
varying amount of <anti-C1q85>
30 min on ice
2% IR35577, 1 µM tL
normalized fluorescene intensity /%
0
50
100
150

Figure 17

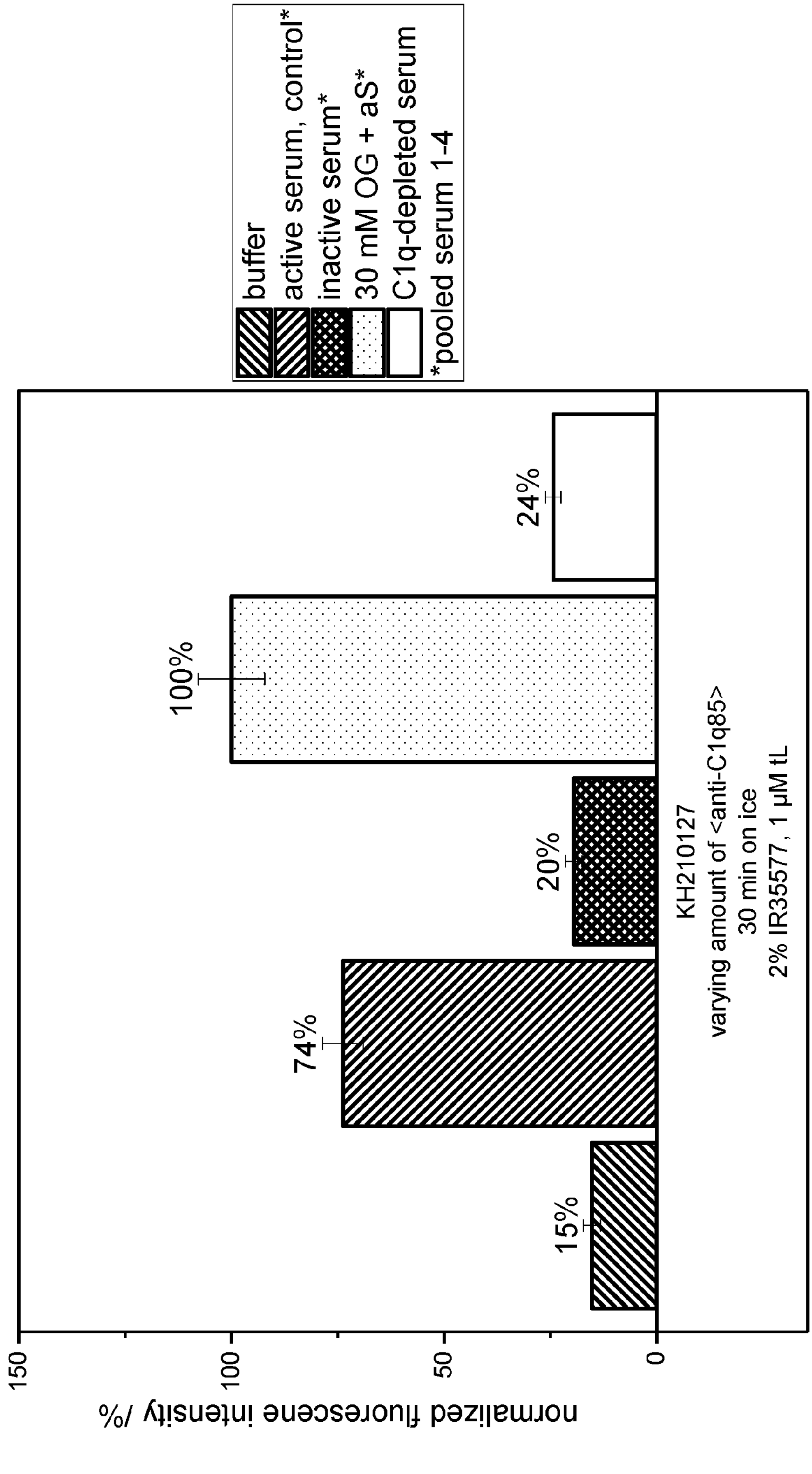
buffer
active serum, control*
inactive serum*
30 mM OG + aS*
C1q-depleted serum
*pooled serum 1-4
15%
74%
20%
100%
24%
KH210127
varying amount of <anti-C1q85>
30 min on ice
2% IR35577, 1 µM tL
normalized fluorescene intensity /%
150
100
50
0

Figure 18

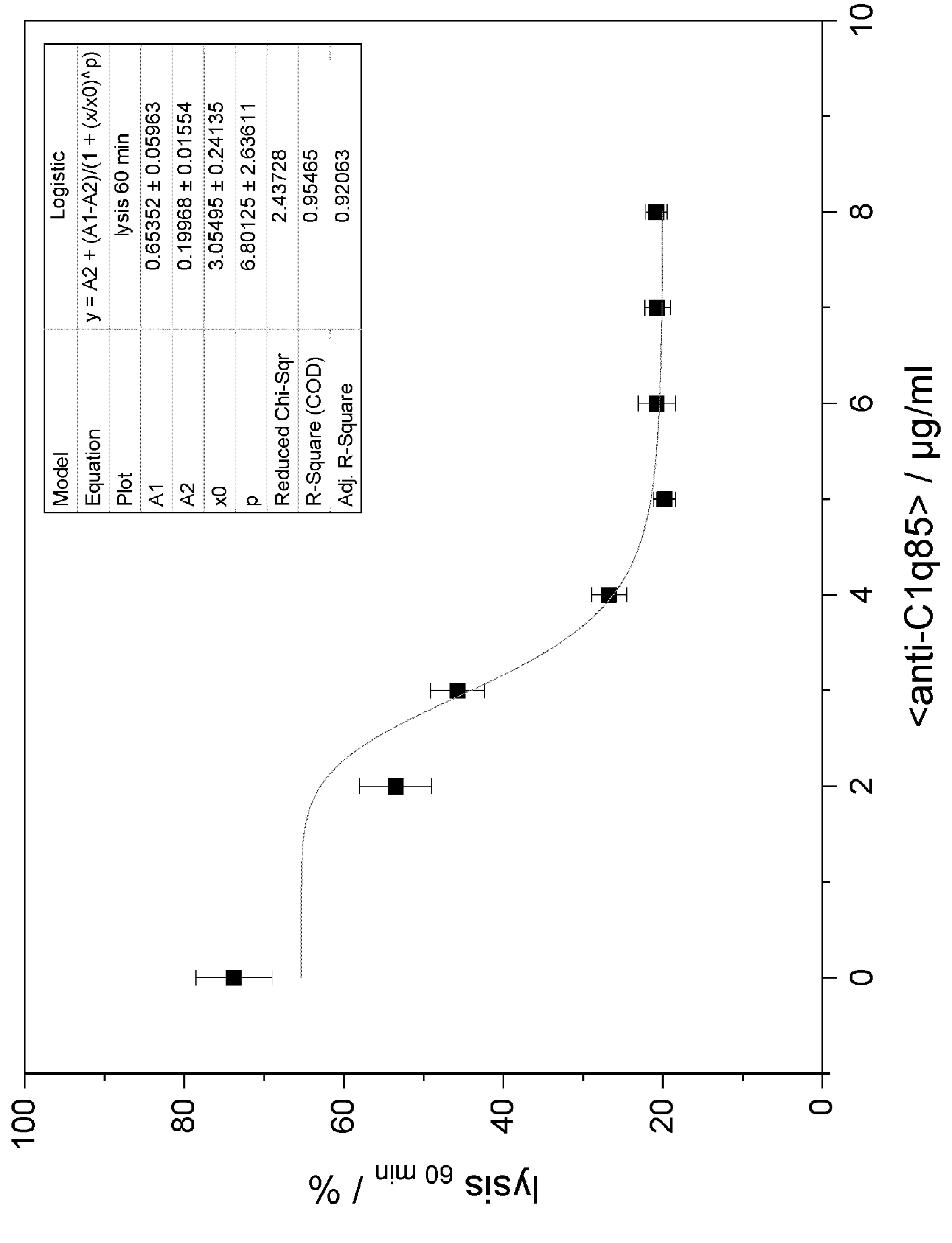

Model
Logistic
Equation
y = A2 + (A1-A2)/(1 + (x/x0)^p)
Plot
lysis 60 min
A1
0.65352 ± 0.05963
A2
0.19968 ± 0.01554
x0
3.05495 ± 0.24135
p
6.80125 ± 2.63611
Reduced Chi-Sqr
2.43728
R-Square (COD)
0.95465
Adj. R-Square
0.92063
lysis 60 min / %
<anti-C1q85> / µg/ml
0
20
40
60
80
100
0
2
4
6
8
10

**Figure 19**

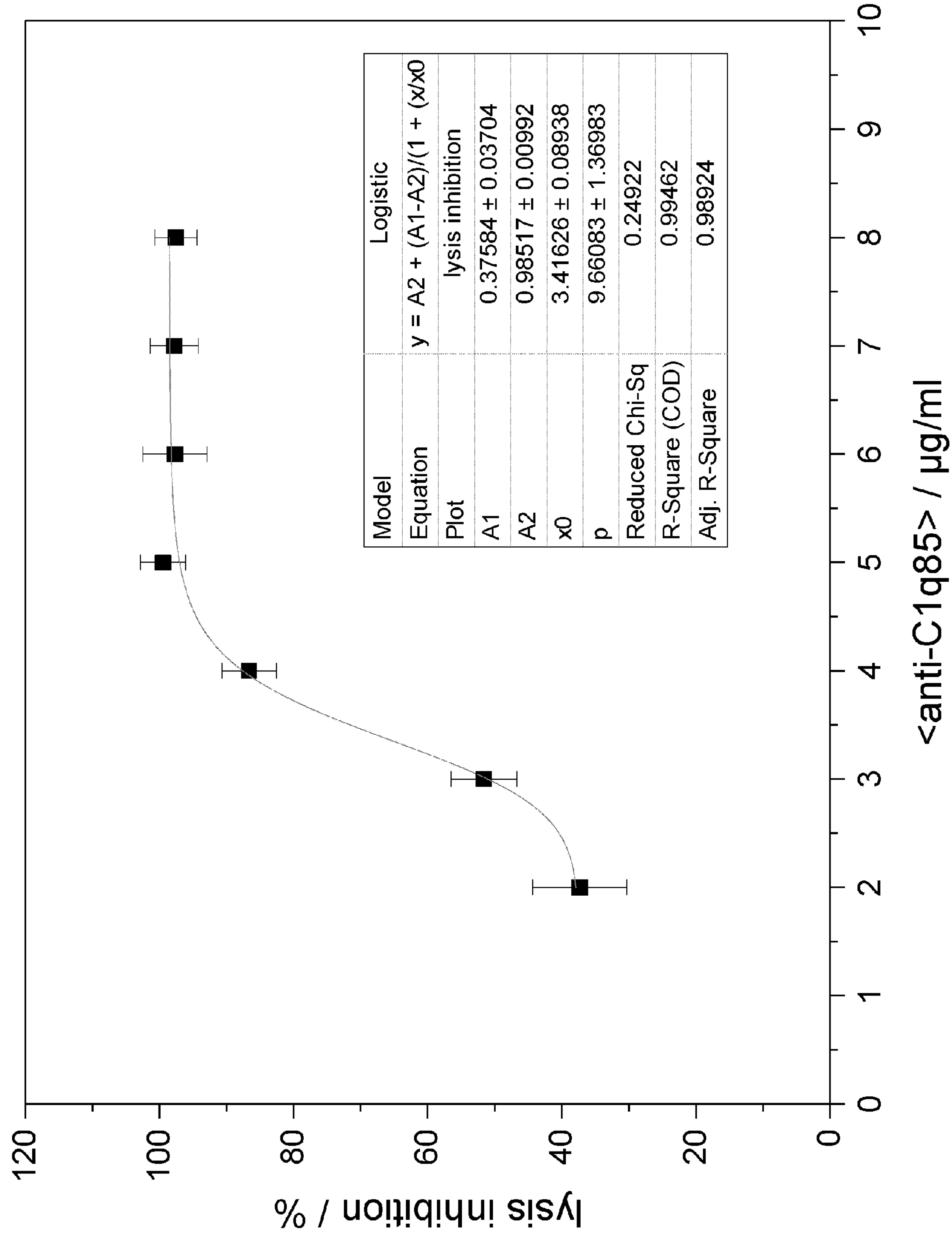
lysis inhibition / %
<anti-C1q85> / µg/ml
0
20
40
60
80
100
120
0
1
2
3
4
5
6
7
8
9
10
Model | Logistic
Equation | y = A2 + (A1-A2)/(1 + (x/x0
Plot | lysis inhibition
A1 | 0.37584 ± 0.03704
A2 | 0.98517 ± 0.00992
x0 | 3.41626 ± 0.08938
p | 9.66083 ± 1.36983
Reduced Chi-Sq | 0.24922
R-Square (COD) | 0.99462
Adj. R-Square | 0.98924

Figure 20

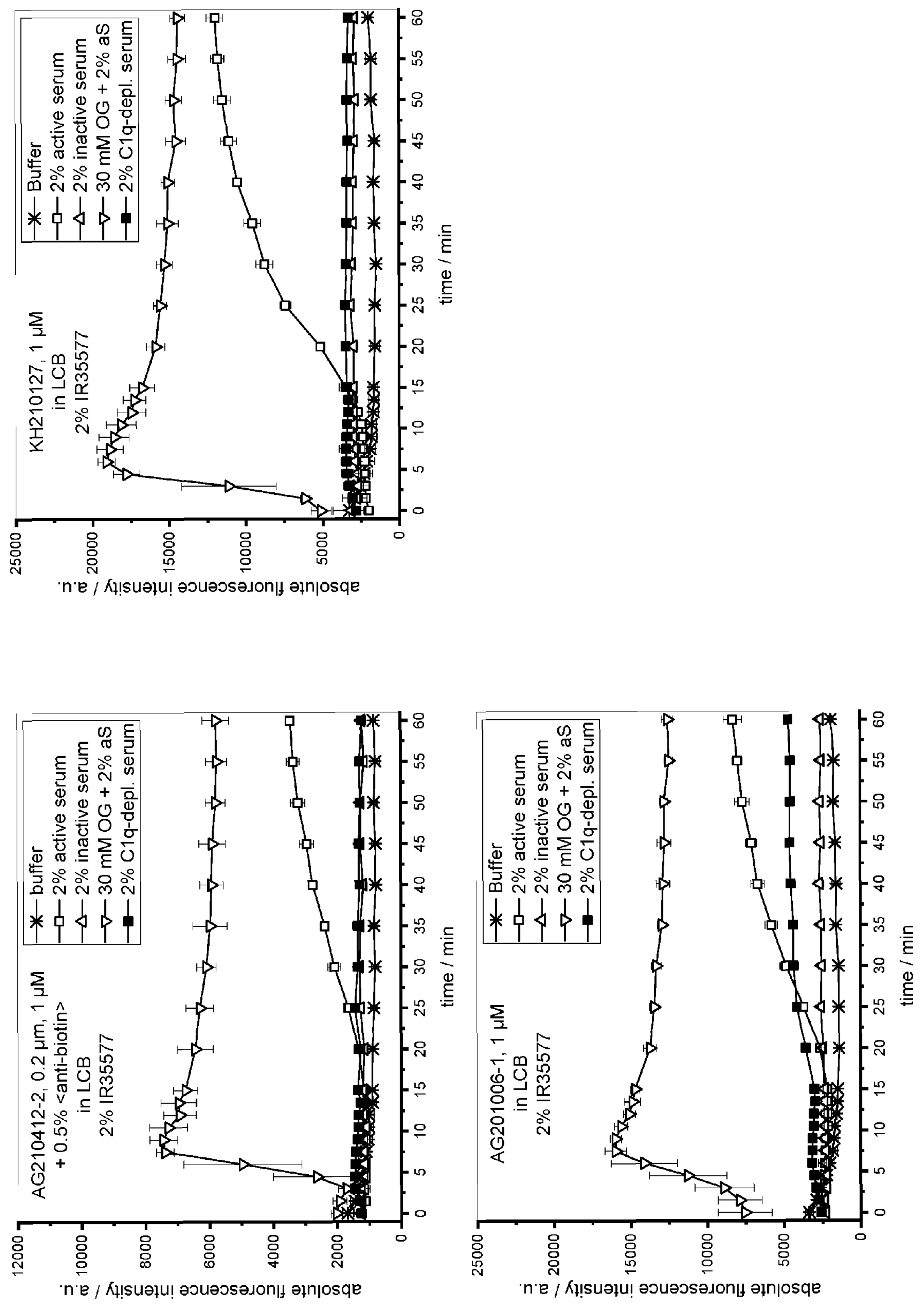
AG210412-2, 0.2 µm, 1 µM
+ 0.5% <anti-biotin>
in LCB
2% IR35577
buffer
2% active serum
2% inactive serum
30 mM OG + 2% aS
2% C1q-depl. serum
absolute fluorescence intensity / a.u.
time / min
AG201006-1, 1 µM
in LCB
2% IR35577
Buffer
2% active serum
2% inactive serum
30 mM OG + 2% aS
2% C1q-depl. serum
absolute fluorescence intensity / a.u.
time / min
KH210127, 1 µM
in LCB
2% IR35577
Buffer
2% active serum
2% inactive serum
30 mM OG + 2% aS
2% C1q-depl. serum
absolute fluorescence intensity / a.u.
time / min

Figure 21

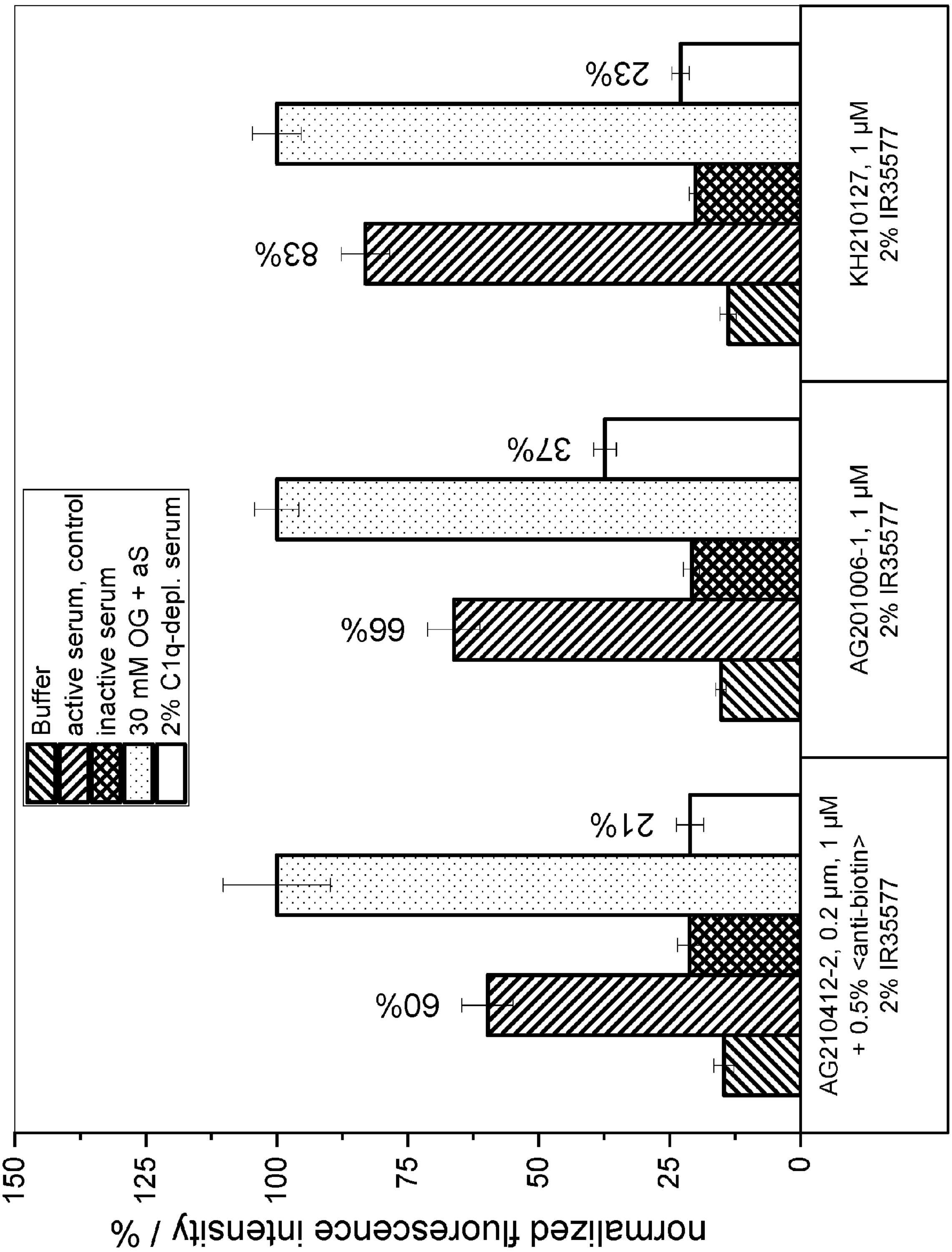
normalized fluorescence intensity / %
0
25
50
75
100
125
150
Buffer
active serum, control
inactive serum
30 mM OG + aS
2% C1q-depl. serum
60%
21%
66%
37%
83%
23%
AG210412-2, 0.2 µm, 1 µM
+ 0.5% <anti-biotin>
2% IR35577
AG201006-1, 1 µM
2% IR35577
KH210127, 1 µM
2% IR35577

# Figure 22

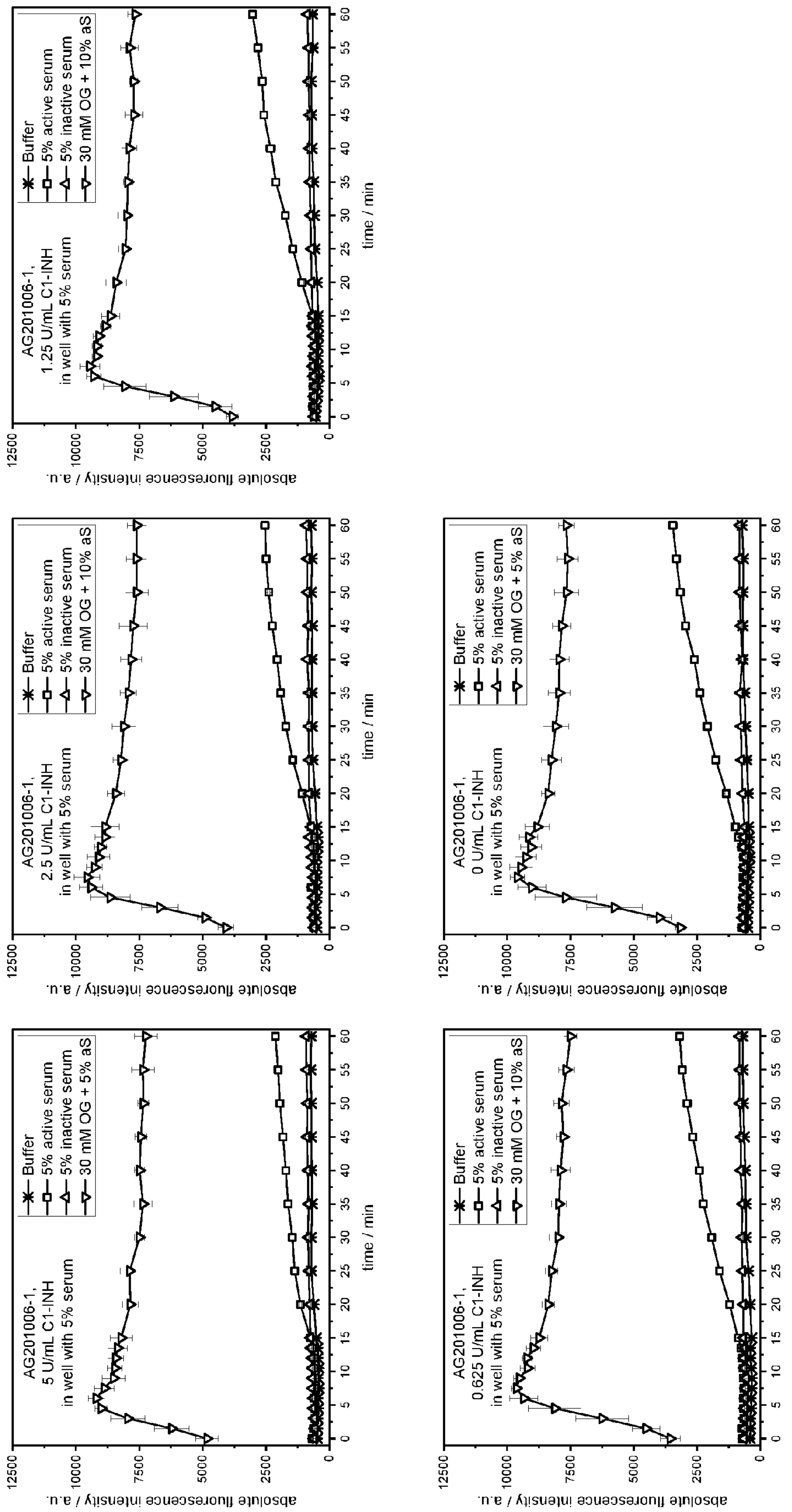
AG201006-1, 5 U/mL C1-INH in well with 5% serum
AG201006-1, 2.5 U/mL C1-INH in well with 5% serum
AG201006-1, 1.25 U/mL C1-INH in well with 5% serum
AG201006-1, 0.625 U/mL C1-INH in well with 5% serum
AG201006-1, 0 U/mL C1-INH in well with 5% serum
Buffer
5% active serum
5% inactive serum
30 mM OG + 5% aS
30 mM OG + 10% aS
absolute fluorescence intensity / a.u.
time / min

Figure 23

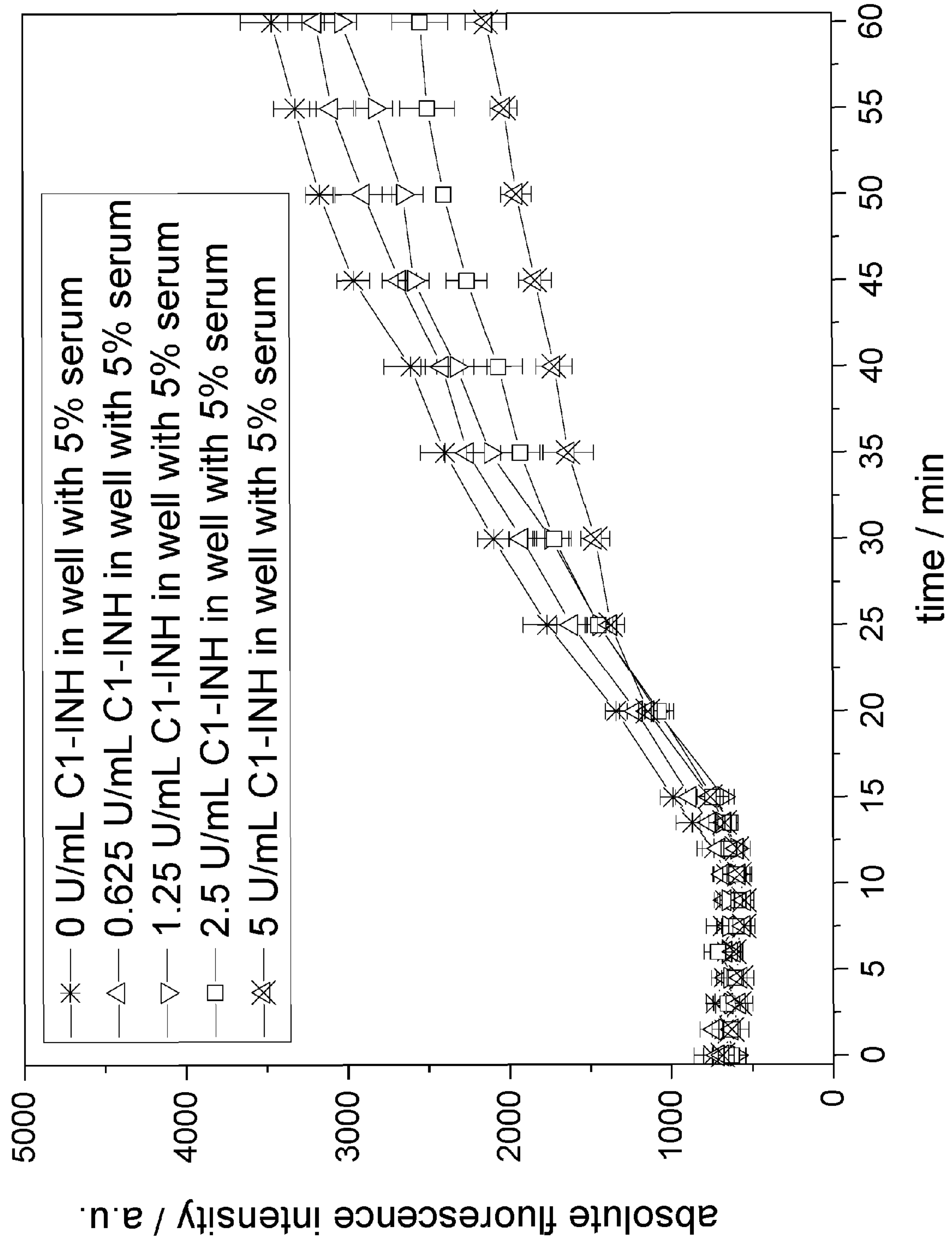
absolute fluorescence intensity / a.u.
0
1000
2000
3000
4000
5000
time / min
0
5
10
15
20
25
30
35
40
45
50
55
60
0 U/mL C1-INH in well with 5% serum
0.625 U/mL C1-INH in well with 5% serum
1.25 U/mL C1-INH in well with 5% serum
2.5 U/mL C1-INH in well with 5% serum
5 U/mL C1-INH in well with 5% serum

Figure 24

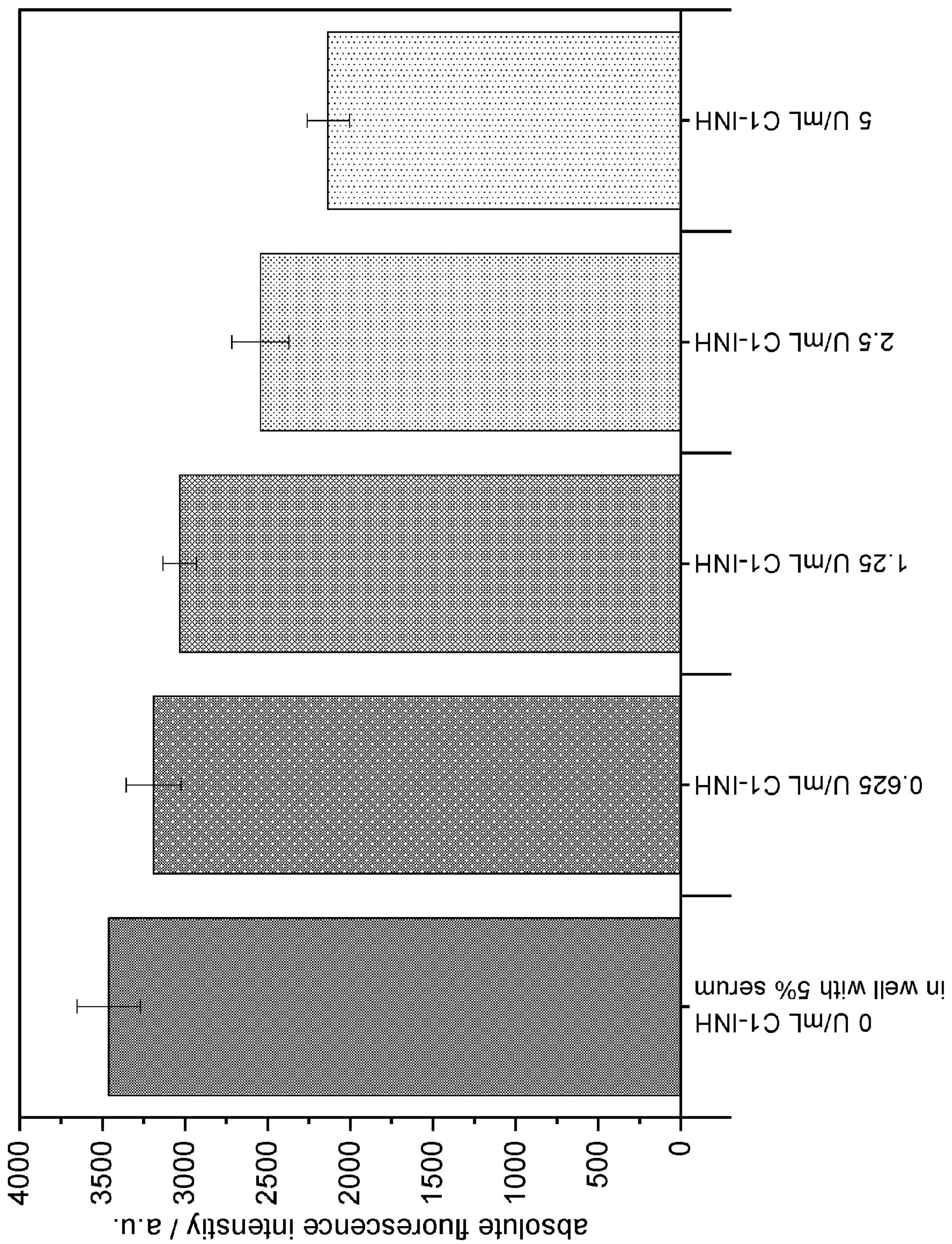
absolute fluorescence intensity / a.u.
0
500
1000
1500
2000
2500
3000
3500
4000
0 U/mL C1-INH
in well with 5% serum
0.625 U/mL C1-INH
1.25 U/mL C1-INH
2.5 U/mL C1-INH
5 U/mL C1-INH

Figure 25

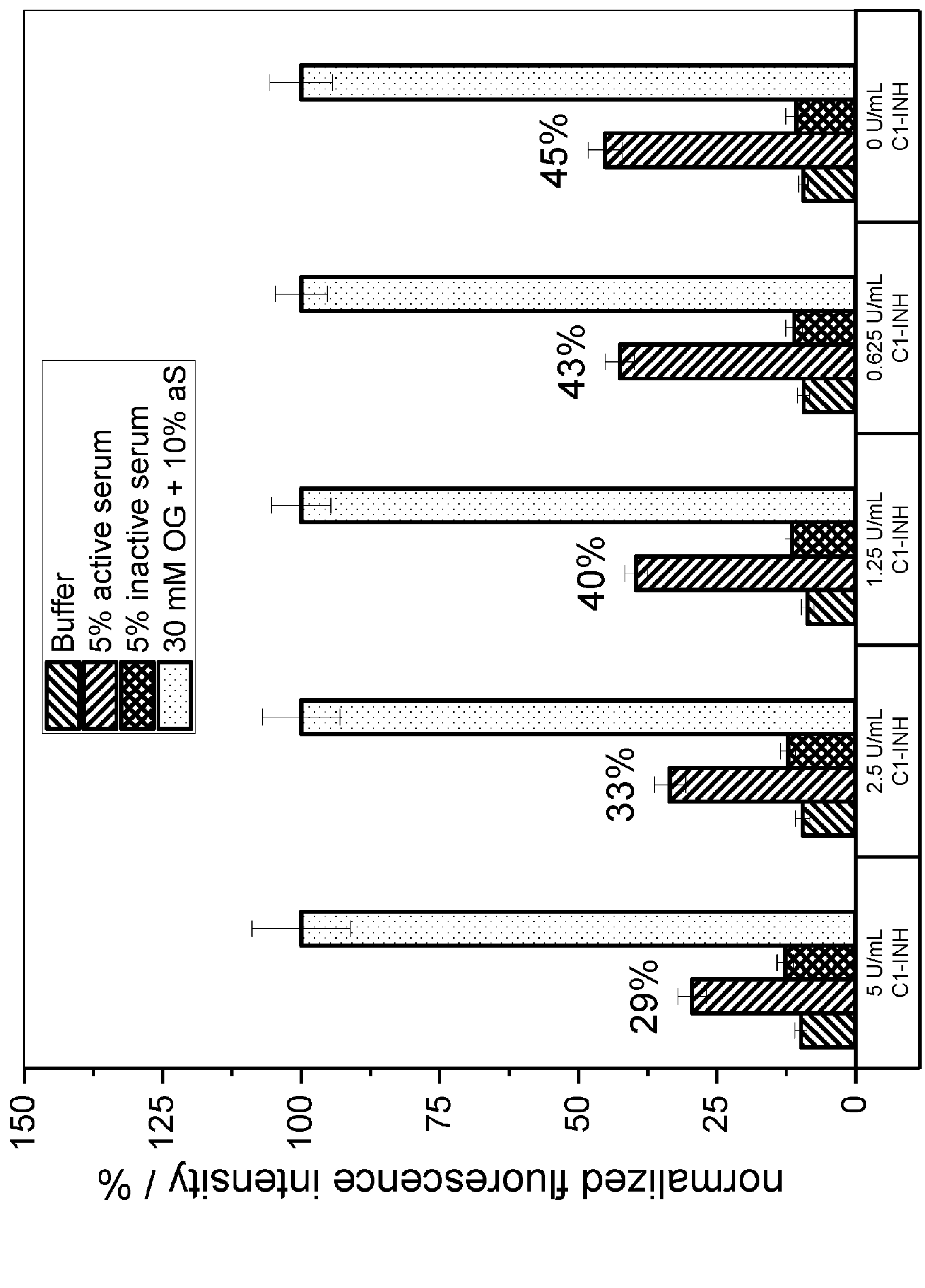
Buffer
5% active serum
5% inactive serum
30 mM OG + 10% aS
29%
33%
40%
43%
45%
5 U/mL C1-INH
2.5 U/mL C1-INH
1.25 U/mL C1-INH
0.625 U/mL C1-INH
0 U/mL C1-INH
normalized fluorescence intensity / %
0
25
50
75
100
125
150

Figure 26

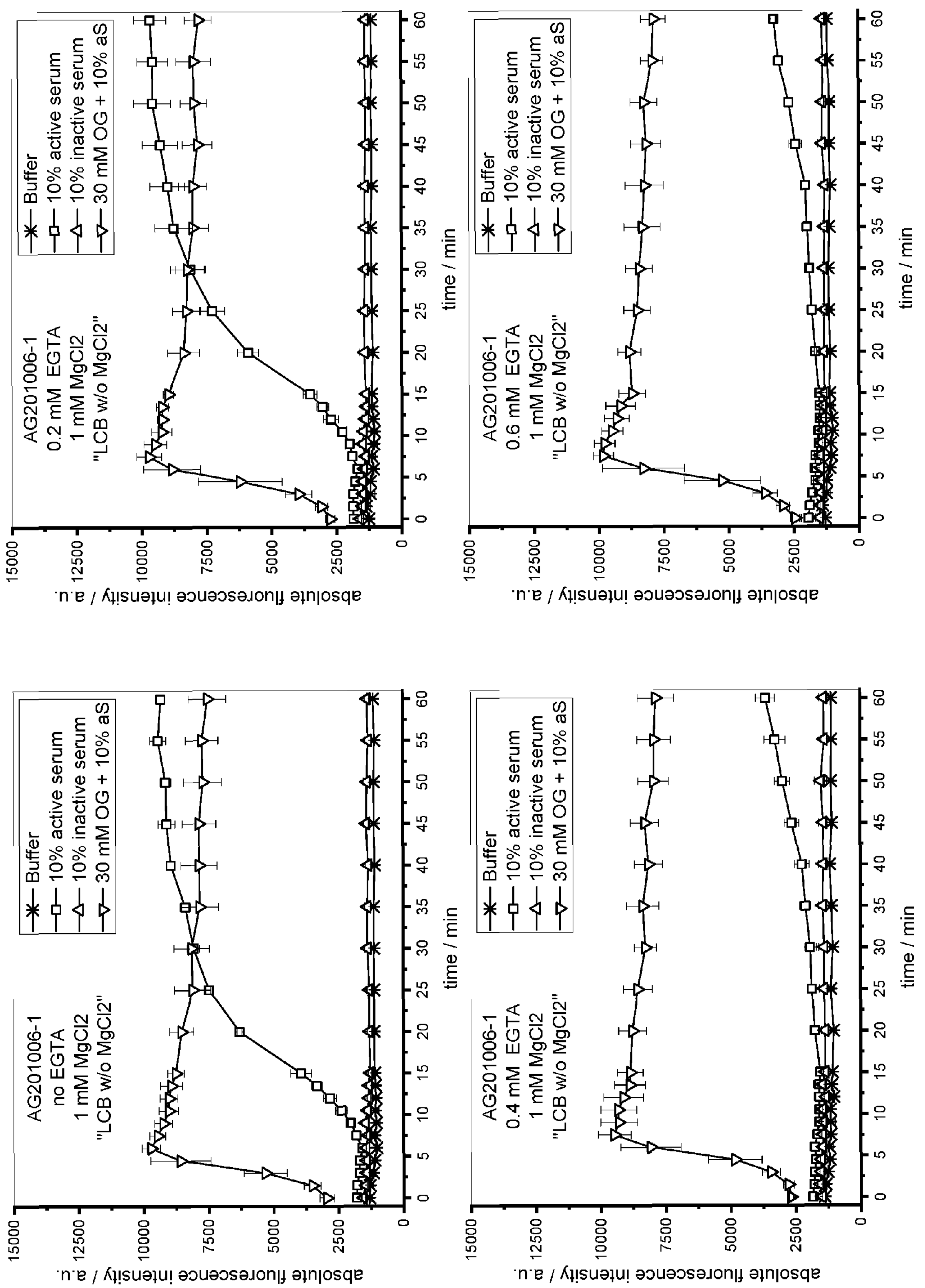
AG201006-1
no EGTA
1 mM MgCl2
"LCB w/o MgCl2"
AG201006-1
0.2 mM EGTA
1 mM MgCl2
"LCB w/o MgCl2"
AG201006-1
0.4 mM EGTA
1 mM MgCl2
"LCB w/o MgCl2"
AG201006-1
0.6 mM EGTA
1 mM MgCl2
"LCB w/o MgCl2"
Buffer
10% active serum
10% inactive serum
30 mM OG + 10% aS
absolute fluorescence intensity / a.u.
time / min

**Figure 26, continued**

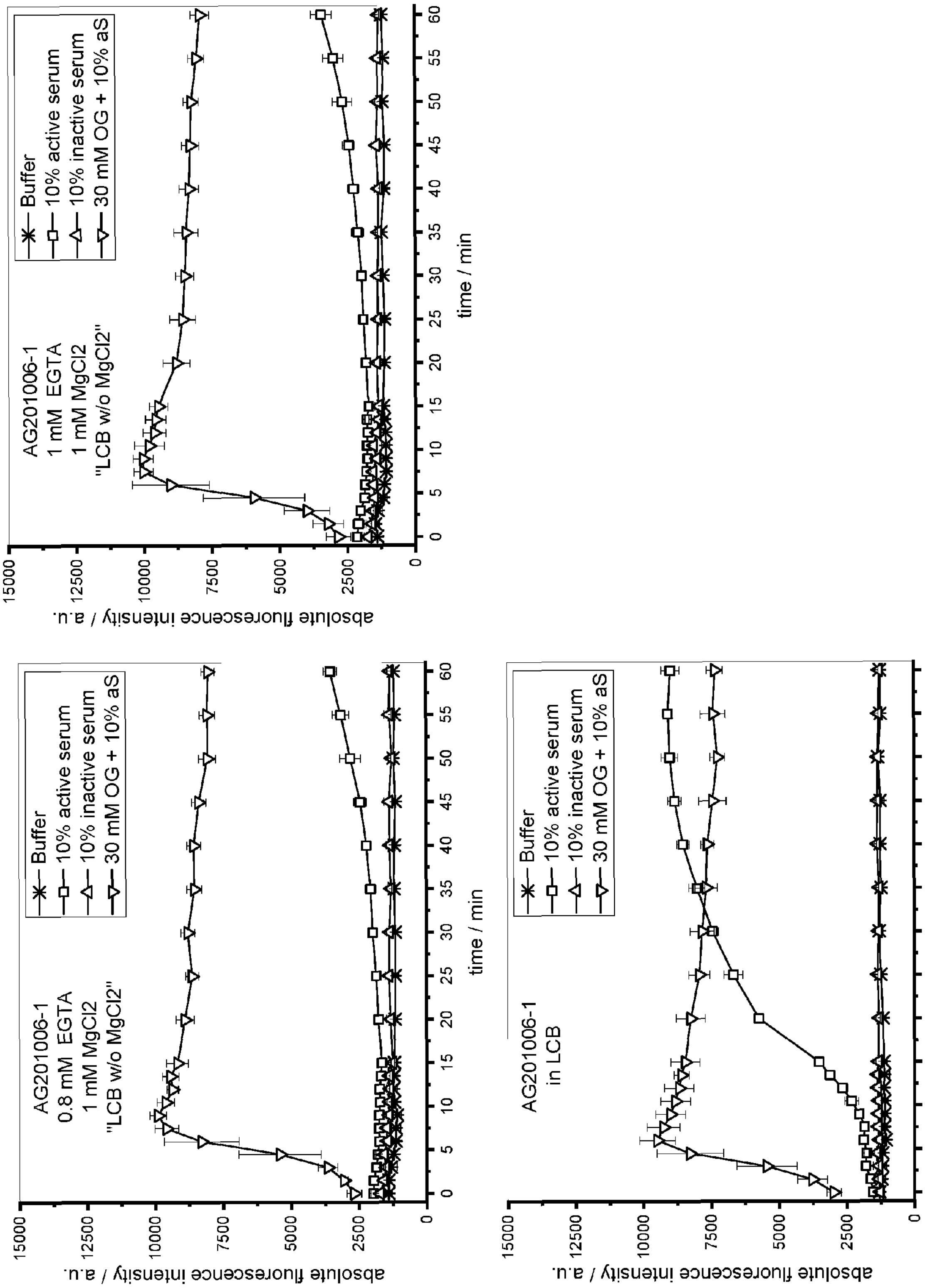

Figure 27

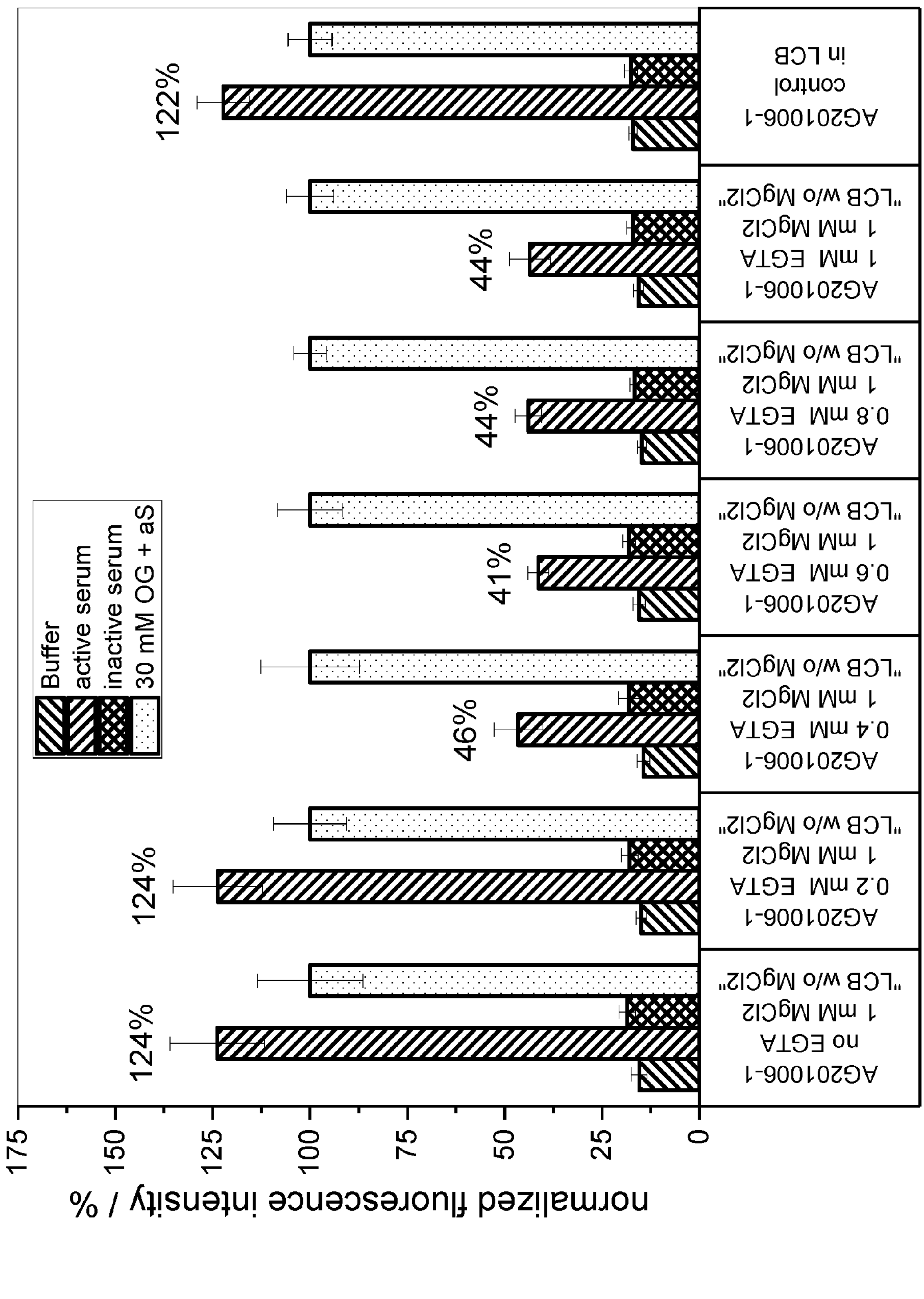
normalized fluorescence intensity / %
0
25
50
75
100
125
150
175
Buffer
active serum
inactive serum
30 mM OG + aS
124%
124%
46%
41%
44%
44%
122%
AG201006-1 no EGTA 1 mM MgCl2 "LCB w/o MgCl2"
AG201006-1 0.2 mM EGTA 1 mM MgCl2 "LCB w/o MgCl2"
AG201006-1 0.4 mM EGTA 1 mM MgCl2 "LCB w/o MgCl2"
AG201006-1 0.6 mM EGTA 1 mM MgCl2 "LCB w/o MgCl2"
AG201006-1 0.8 mM EGTA 1 mM MgCl2 "LCB w/o MgCl2"
AG201006-1 1 mM EGTA 1 mM MgCl2 "LCB w/o MgCl2"
AG201006-1 control in LCB

Figure 28

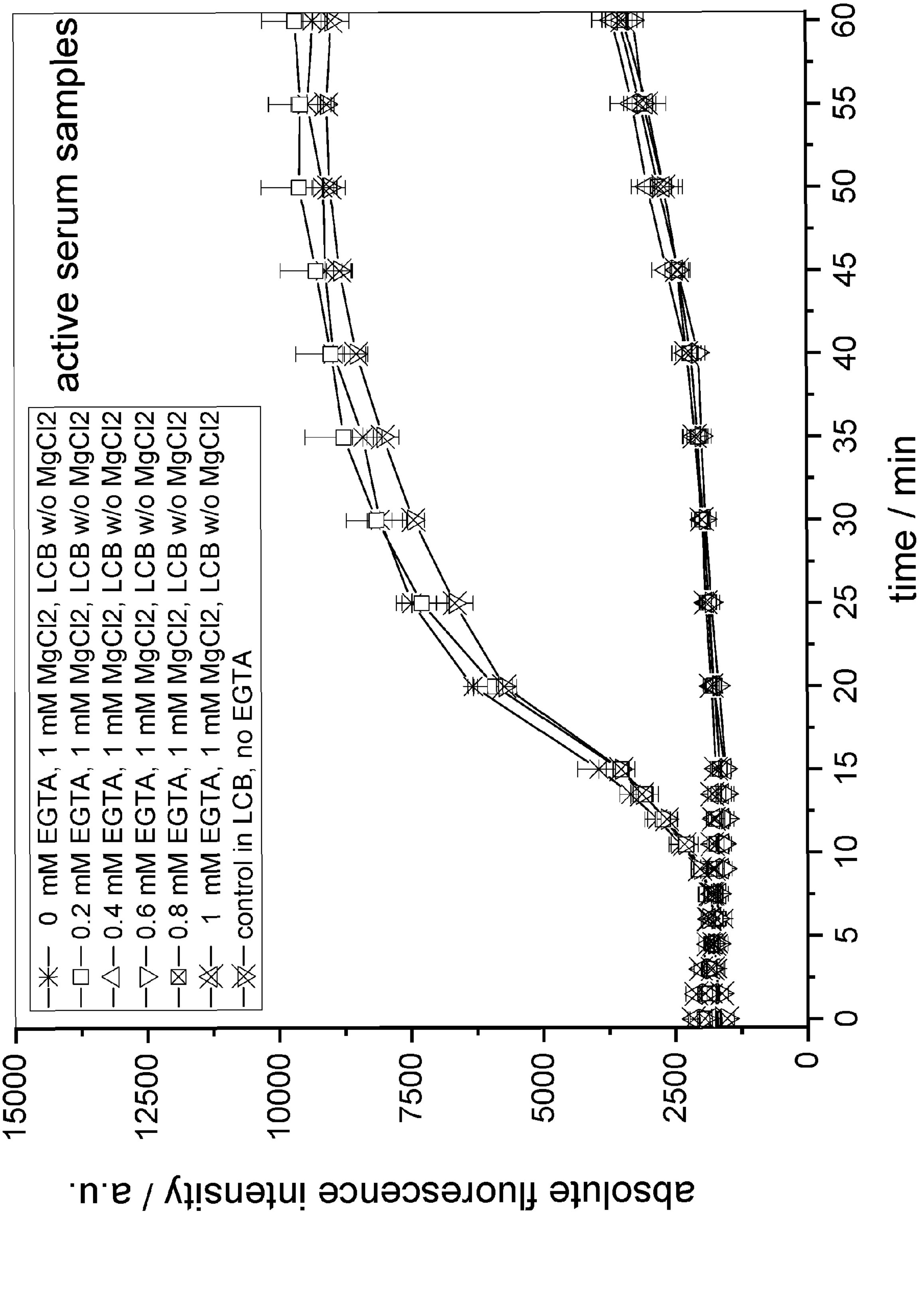
active serum samples
0 mM EGTA, 1 mM MgCl2, LCB w/o MgCl2
0.2 mM EGTA, 1 mM MgCl2, LCB w/o MgCl2
0.4 mM EGTA, 1 mM MgCl2, LCB w/o MgCl2
0.6 mM EGTA, 1 mM MgCl2, LCB w/o MgCl2
0.8 mM EGTA, 1 mM MgCl2, LCB w/o MgCl2
1 mM EGTA, 1 mM MgCl2, LCB w/o MgCl2
control in LCB, no EGTA
absolute fluorescence intensity / a.u.
0
2500
5000
7500
10000
12500
15000
time / min
0
5
10
15
20
25
30
35
40
45
50
55
60

# Figure 29

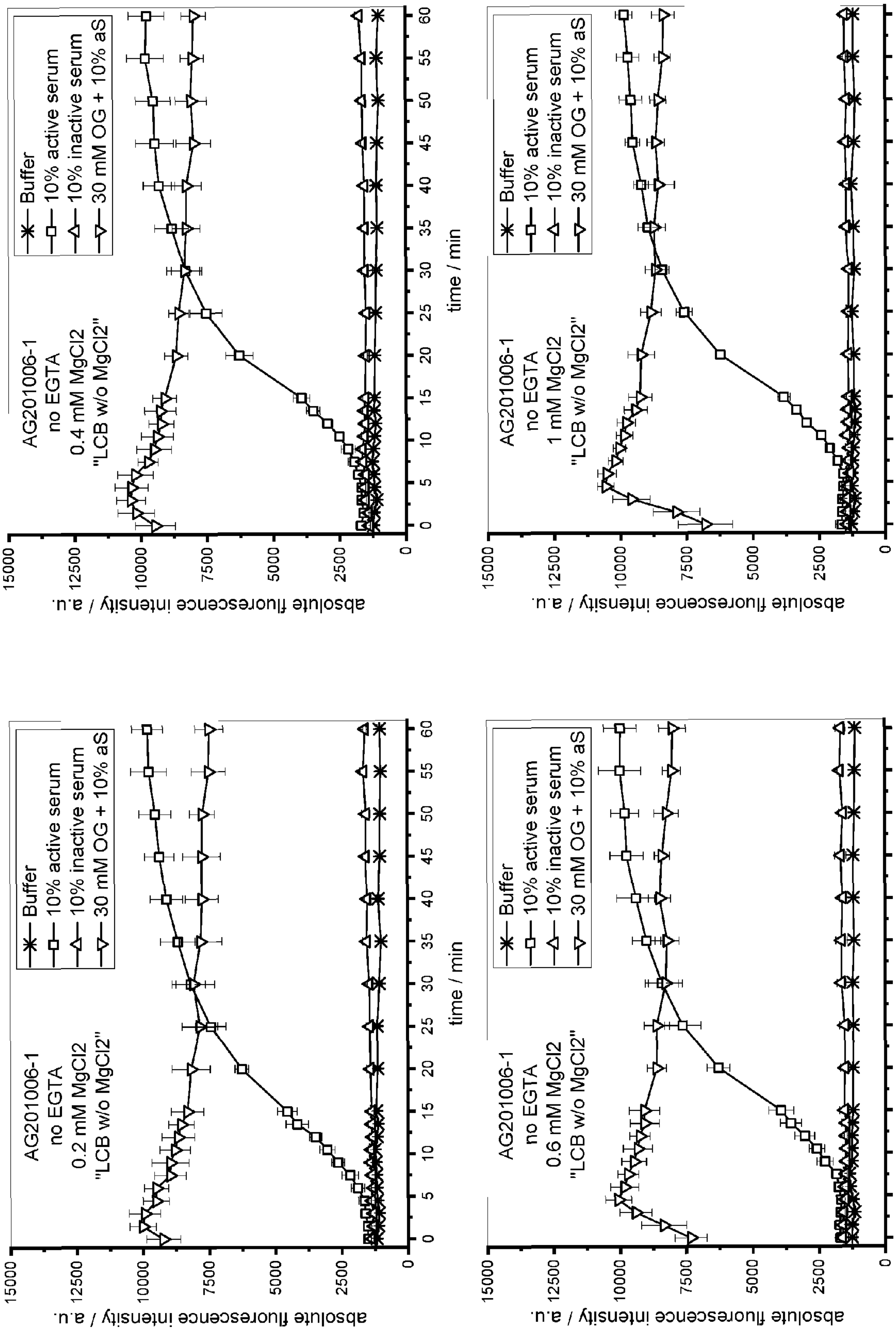
AG201006-1
no EGTA
0.2 mM MgCl2
"LCB w/o MgCl2"
AG201006-1
no EGTA
0.4 mM MgCl2
"LCB w/o MgCl2"
AG201006-1
no EGTA
0.6 mM MgCl2
"LCB w/o MgCl2"
AG201006-1
no EGTA
1 mM MgCl2
"LCB w/o MgCl2"
Buffer
10% active serum
10% inactive serum
30 mM OG + 10% aS
absolute fluorescence intensity / a.u.
time / min
0
2500
5000
7500
10000
12500
15000
0
5
10
15
20
25
30
35
40
45
50
55
60

# Figure 29, continued

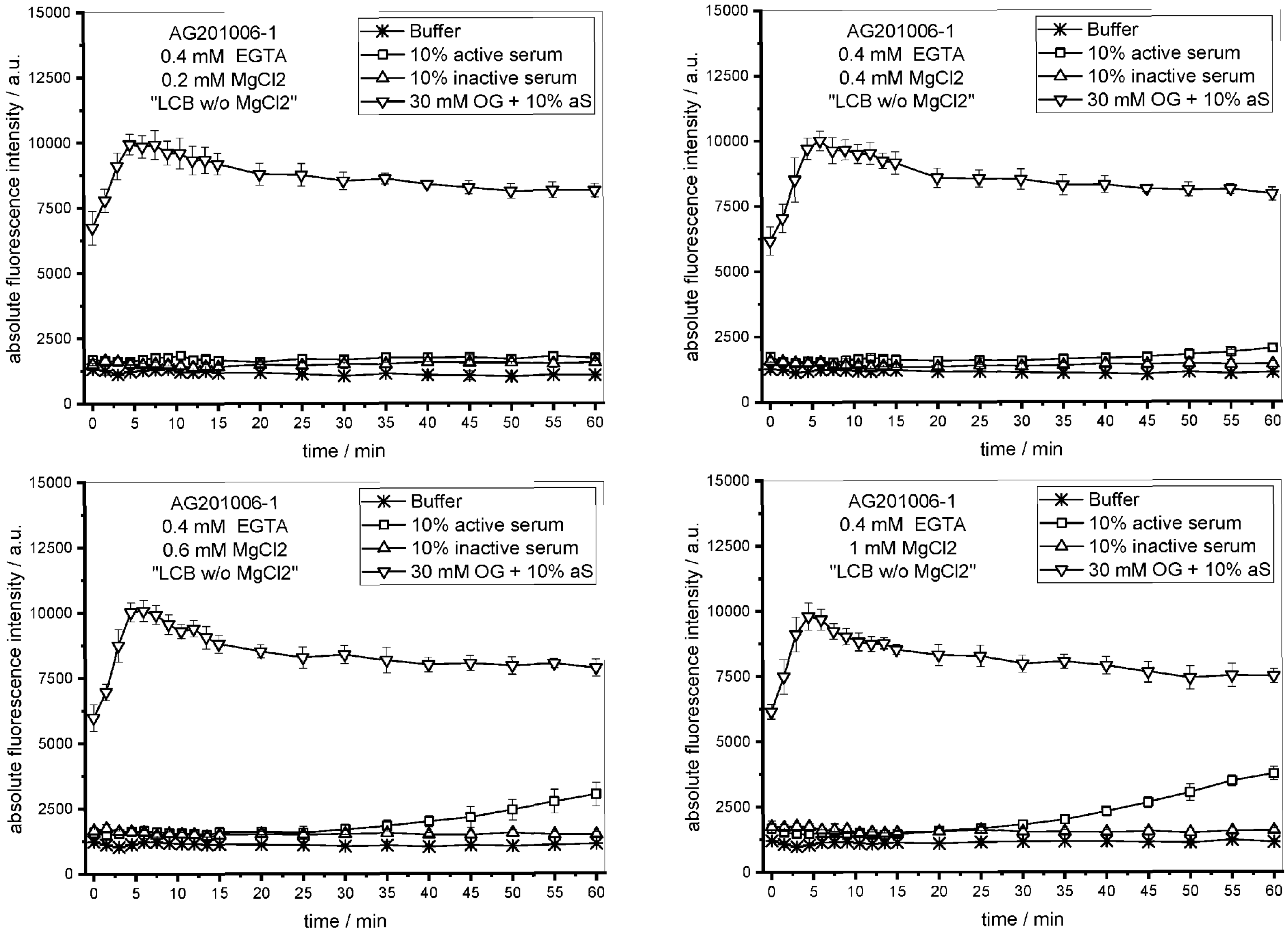

Figure 30

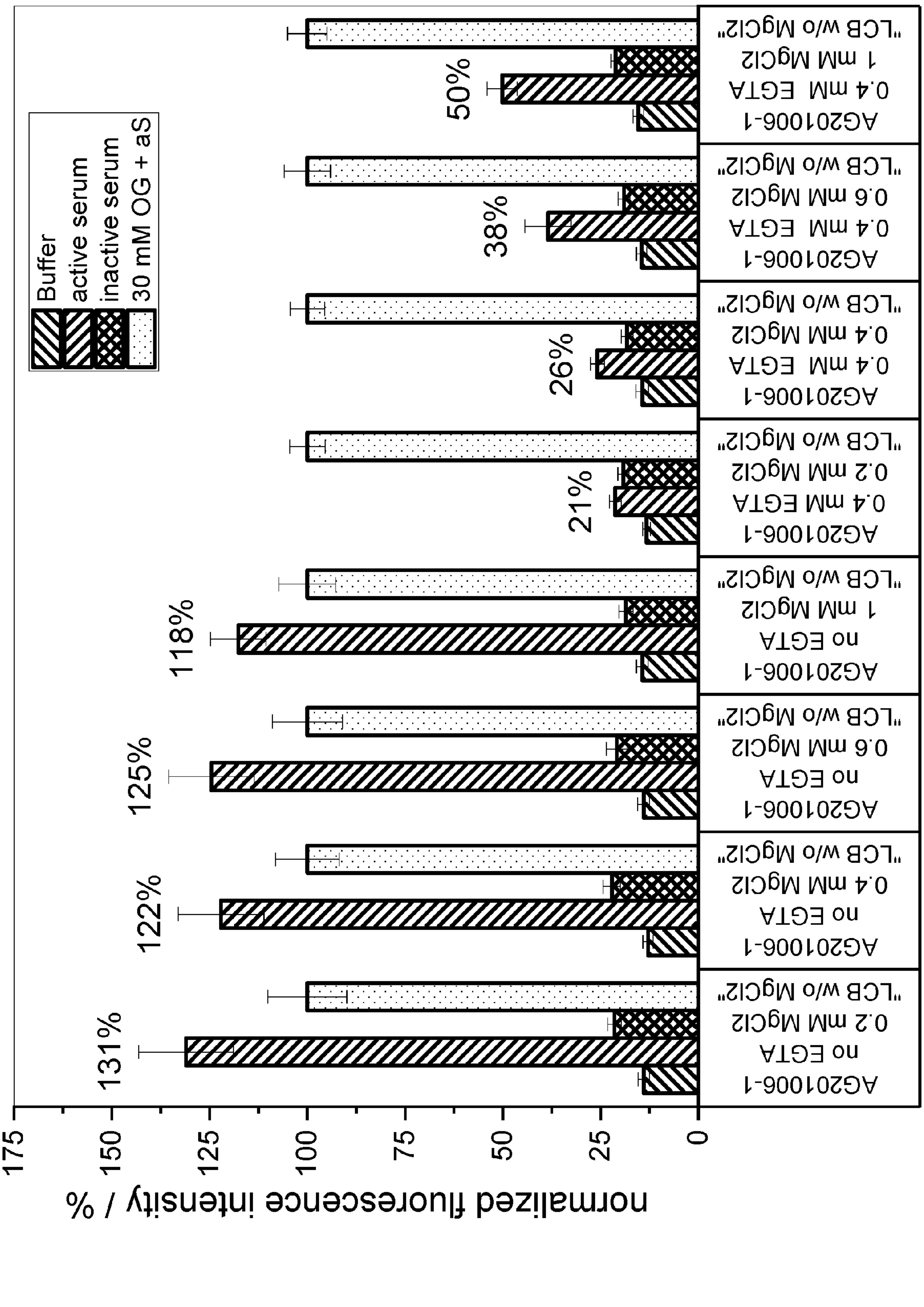
normalized fluorescence intensity / %
0
25
50
75
100
125
150
175
Buffer
active serum
inactive serum
30 mM OG + aS
131%
122%
125%
118%
21%
26%
38%
50%
AG201006-1 no EGTA 0.2 mM MgCl2 "LCB w/o MgCl2"
AG201006-1 no EGTA 0.4 mM MgCl2 "LCB w/o MgCl2"
AG201006-1 no EGTA 0.6 mM MgCl2 "LCB w/o MgCl2"
AG201006-1 no EGTA 1 mM MgCl2 "LCB w/o MgCl2"
AG201006-1 0.4 mM EGTA 0.2 mM MgCl2 "LCB w/o MgCl2"
AG201006-1 0.4 mM EGTA 0.4 mM MgCl2 "LCB w/o MgCl2"
AG201006-1 0.4 mM EGTA 0.6 mM MgCl2 "LCB w/o MgCl2"
AG201006-1 0.4 mM EGTA 1 mM MgCl2 "LCB w/o MgCl2"

Figure 31

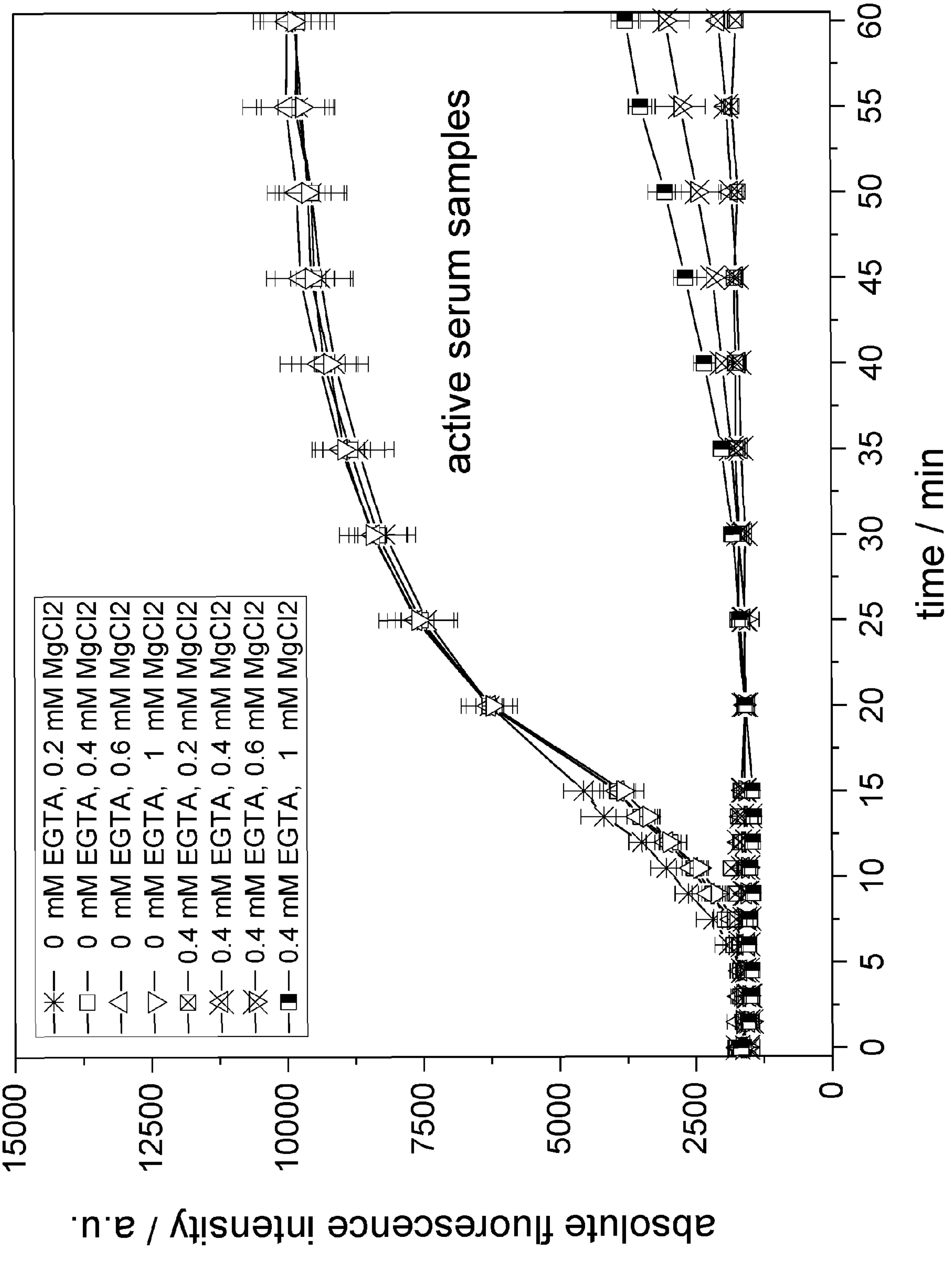
absolute fluorescence intensity / a.u.
15000
12500
10000
7500
5000
2500
0
0
5
10
15
20
25
30
35
40
45
50
55
60
time / min
active serum samples
0 mM EGTA, 0.2 mM MgCl2
0 mM EGTA, 0.4 mM MgCl2
0 mM EGTA, 0.6 mM MgCl2
0 mM EGTA, 1 mM MgCl2
0.4 mM EGTA, 0.2 mM MgCl2
0.4 mM EGTA, 0.4 mM MgCl2
0.4 mM EGTA, 0.6 mM MgCl2
0.4 mM EGTA, 1 mM MgCl2

Figure 32

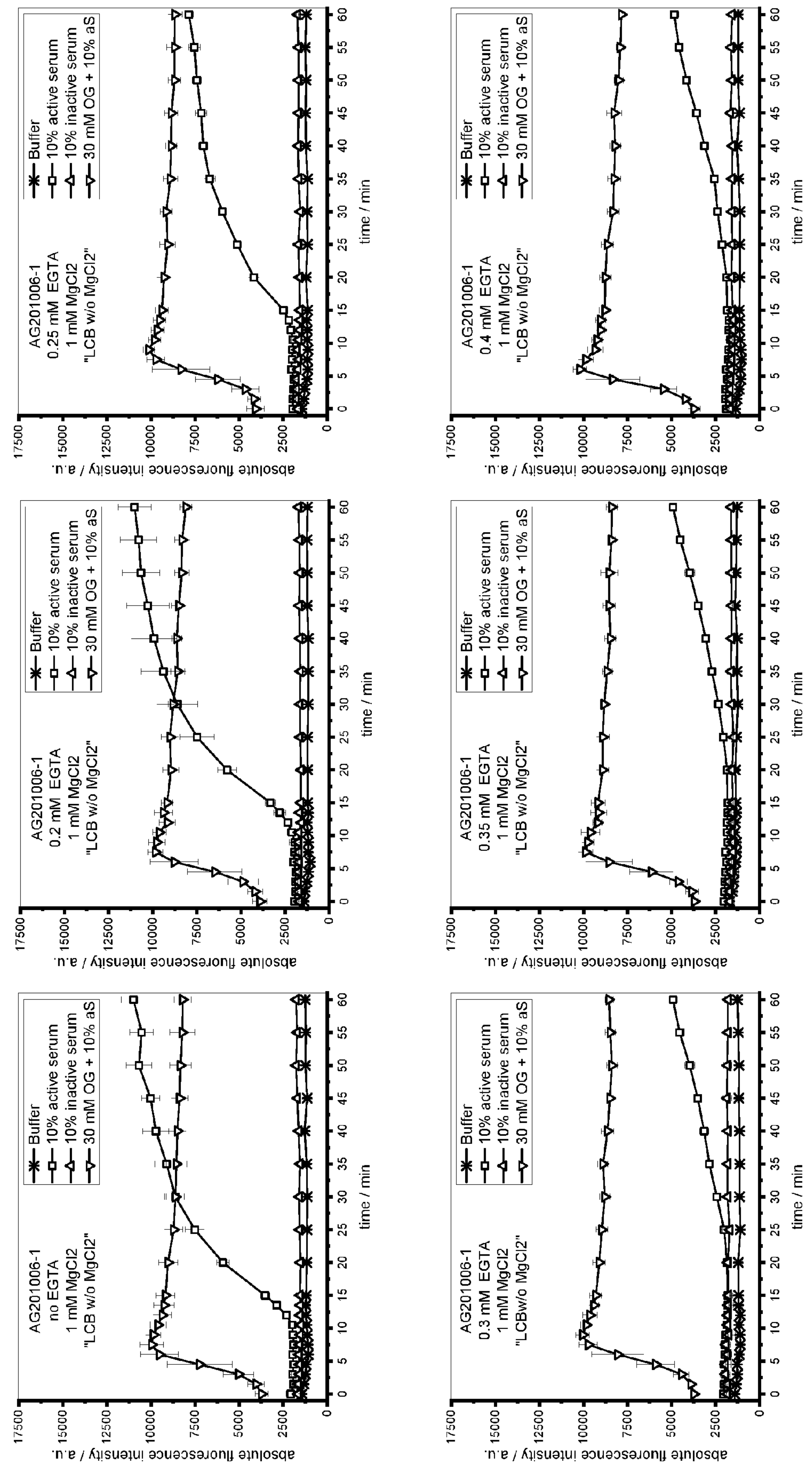
AG201006-1
no EGTA
1 mM MgCl2
"LCB w/o MgCl2"
AG201006-1
0.2 mM EGTA
1 mM MgCl2
"LCB w/o MgCl2"
AG201006-1
0.25 mM EGTA
1 mM MgCl2
"LCB w/o MgCl2"
AG201006-1
0.3 mM EGTA
1 mM MgCl2
"LCBw/o MgCl2"
AG201006-1
0.35 mM EGTA
1 mM MgCl2
"LCB w/o MgCl2"
AG201006-1
0.4 mM EGTA
1 mM MgCl2
"LCB w/o MgCl2"
Buffer
10% active serum
10% inactive serum
30 mM OG + 10% aS
absolute fluorescence intensity / a.u.
time / min

Figure 33

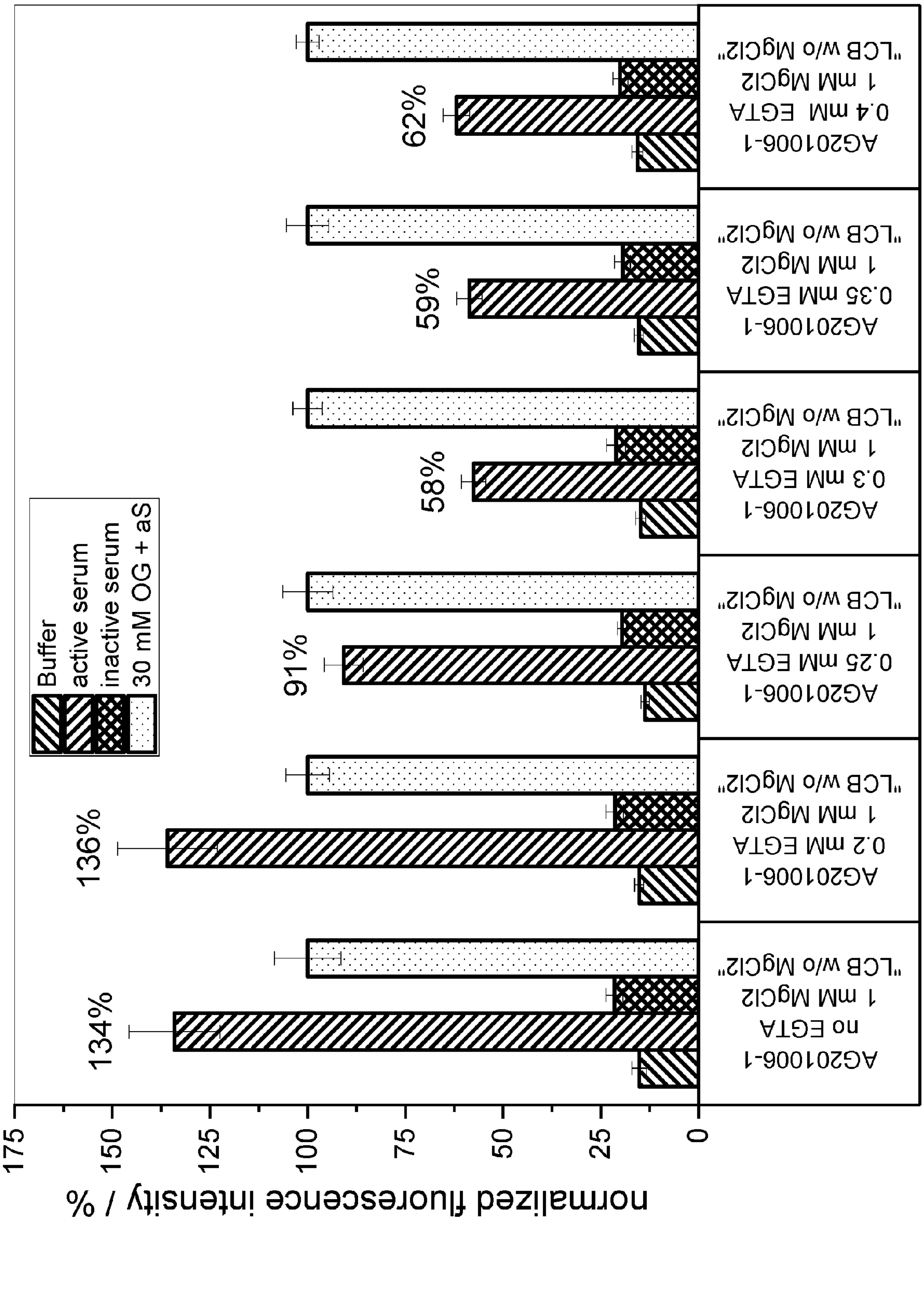
normalized fluorescence intensity / %
0
25
50
75
100
125
150
175
Buffer
active serum
inactive serum
30 mM OG + aS
134%
136%
91%
58%
59%
62%
AG201006-1 no EGTA 1 mM MgCl2 "LCB w/o MgCl2"
AG201006-1 0.2 mM EGTA 1 mM MgCl2 "LCB w/o MgCl2"
AG201006-1 0.25 mM EGTA 1 mM MgCl2 "LCB w/o MgCl2"
AG201006-1 0.3 mM EGTA 1 mM MgCl2 "LCB w/o MgCl2"
AG201006-1 0.35 mM EGTA 1 mM MgCl2 "LCB w/o MgCl2"
AG201006-1 0.4 mM EGTA 1 mM MgCl2 "LCB w/o MgCl2"

Figure 34

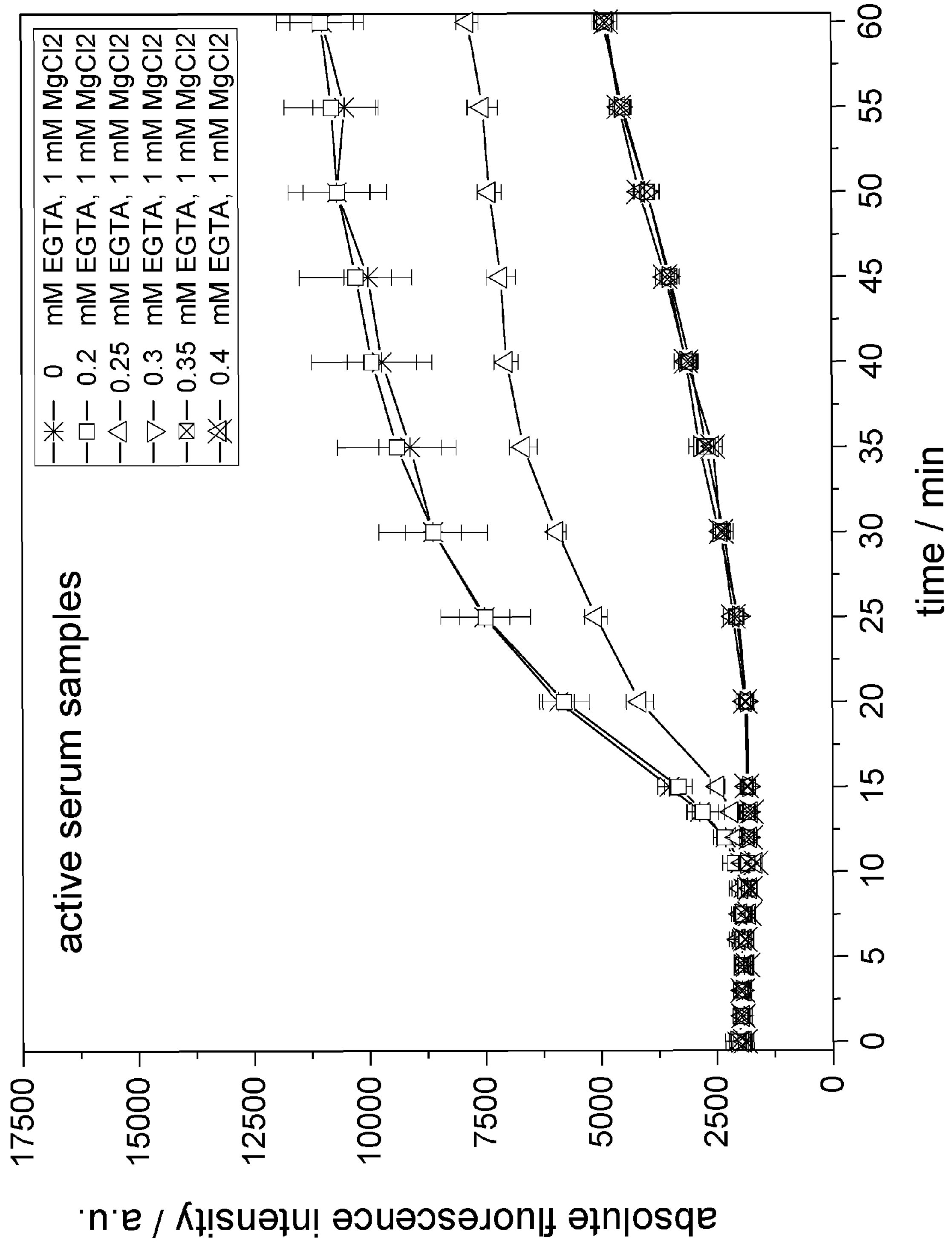
active serum samples
0 mM EGTA, 1 mM MgCl2
0.2 mM EGTA, 1 mM MgCl2
0.25 mM EGTA, 1 mM MgCl2
0.3 mM EGTA, 1 mM MgCl2
0.35 mM EGTA, 1 mM MgCl2
0.4 mM EGTA, 1 mM MgCl2
absolute fluorescence intensity / a.u.
time / min
0
2500
5000
7500
10000
12500
15000
17500
0
5
10
15
20
25
30
35
40
45
50
55
60

# Figure 35

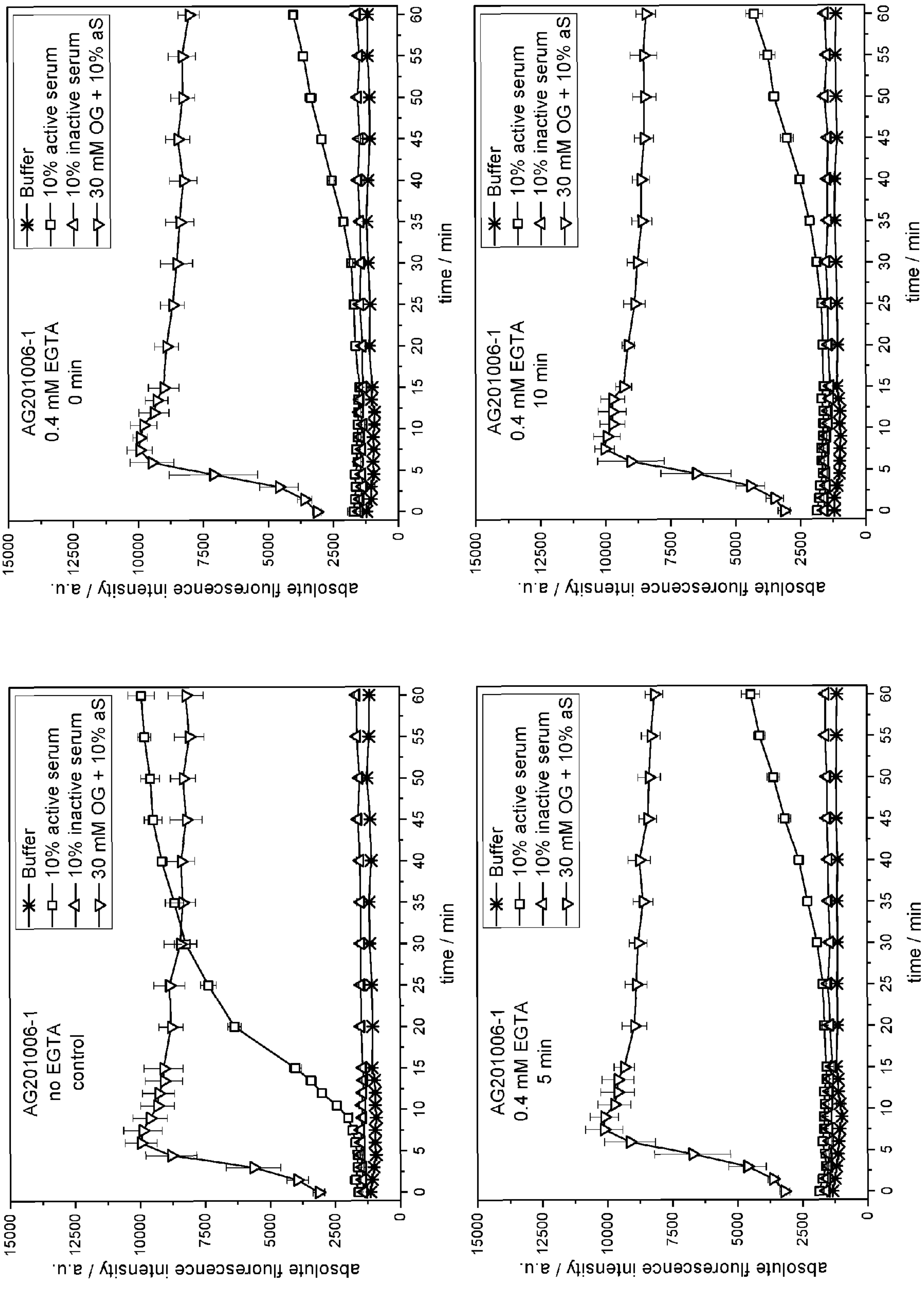
AG201006-1
no EGTA
control
AG201006-1
0.4 mM EGTA
0 min
AG201006-1
0.4 mM EGTA
5 min
AG201006-1
0.4 mM EGTA
10 min
Buffer
10% active serum
10% inactive serum
30 mM OG + 10% aS
absolute fluorescence intensity / a.u.
time / min
0
2500
5000
7500
10000
12500
15000
0
5
10
15
20
25
30
35
40
45
50
55
60

# Figure 35, continued

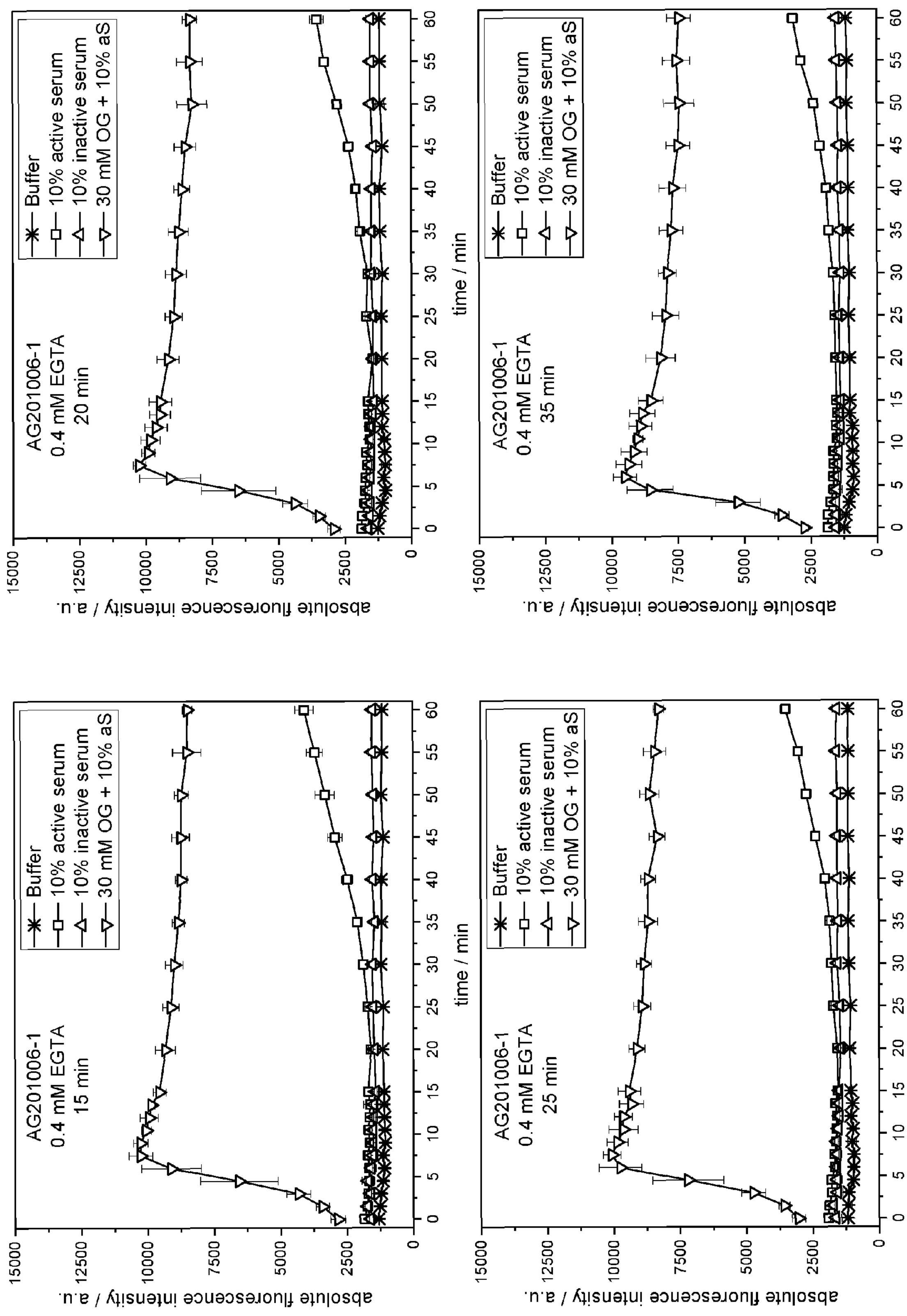

Figure 36

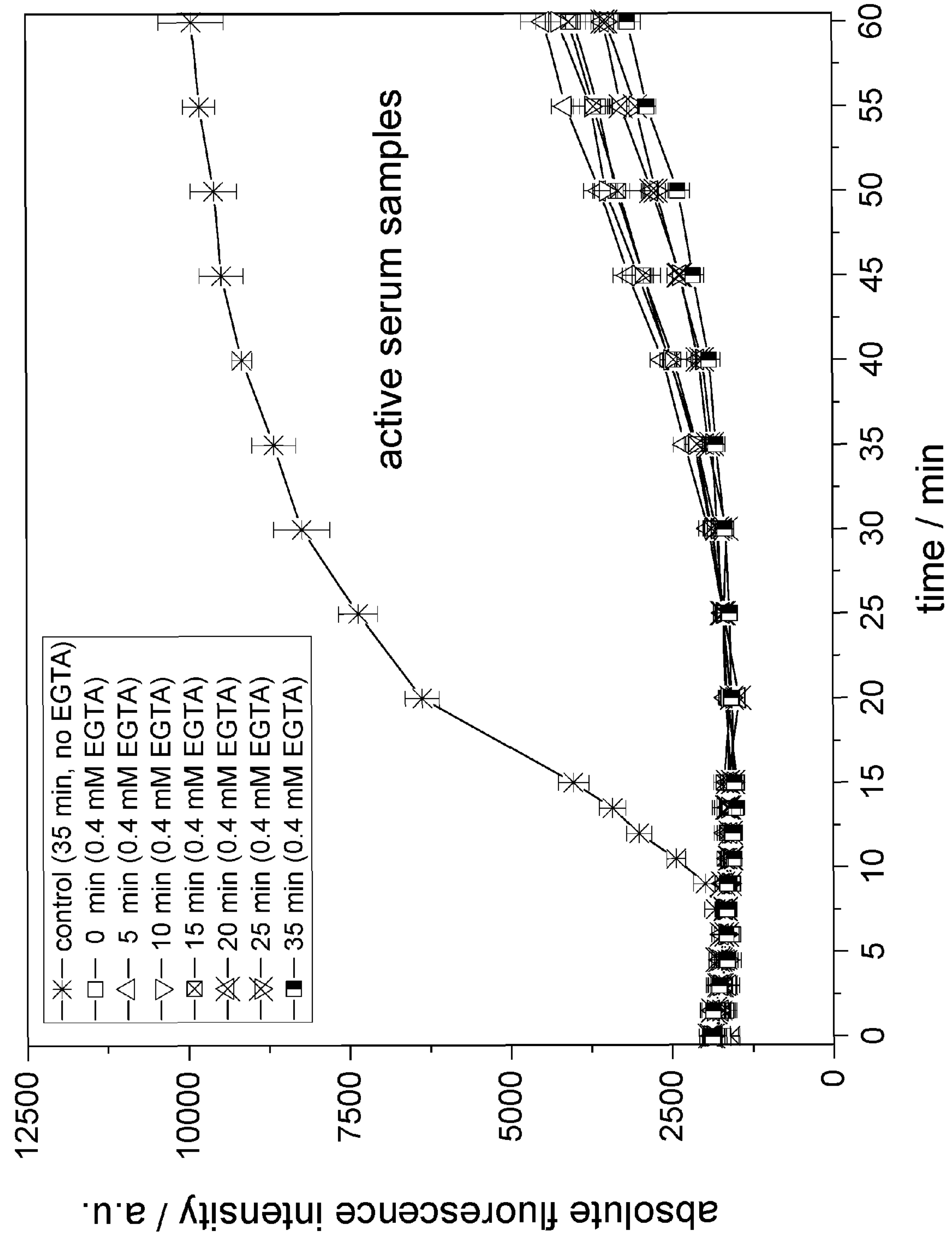
absolute fluorescence intensity / a.u.
12500
10000
7500
5000
2500
0
0
5
10
15
20
25
30
35
40
45
50
55
60
time / min
active serum samples
control (35 min, no EGTA)
0 min (0.4 mM EGTA)
5 min (0.4 mM EGTA)
10 min (0.4 mM EGTA)
15 min (0.4 mM EGTA)
20 min (0.4 mM EGTA)
25 min (0.4 mM EGTA)
35 min (0.4 mM EGTA)

Figure 37

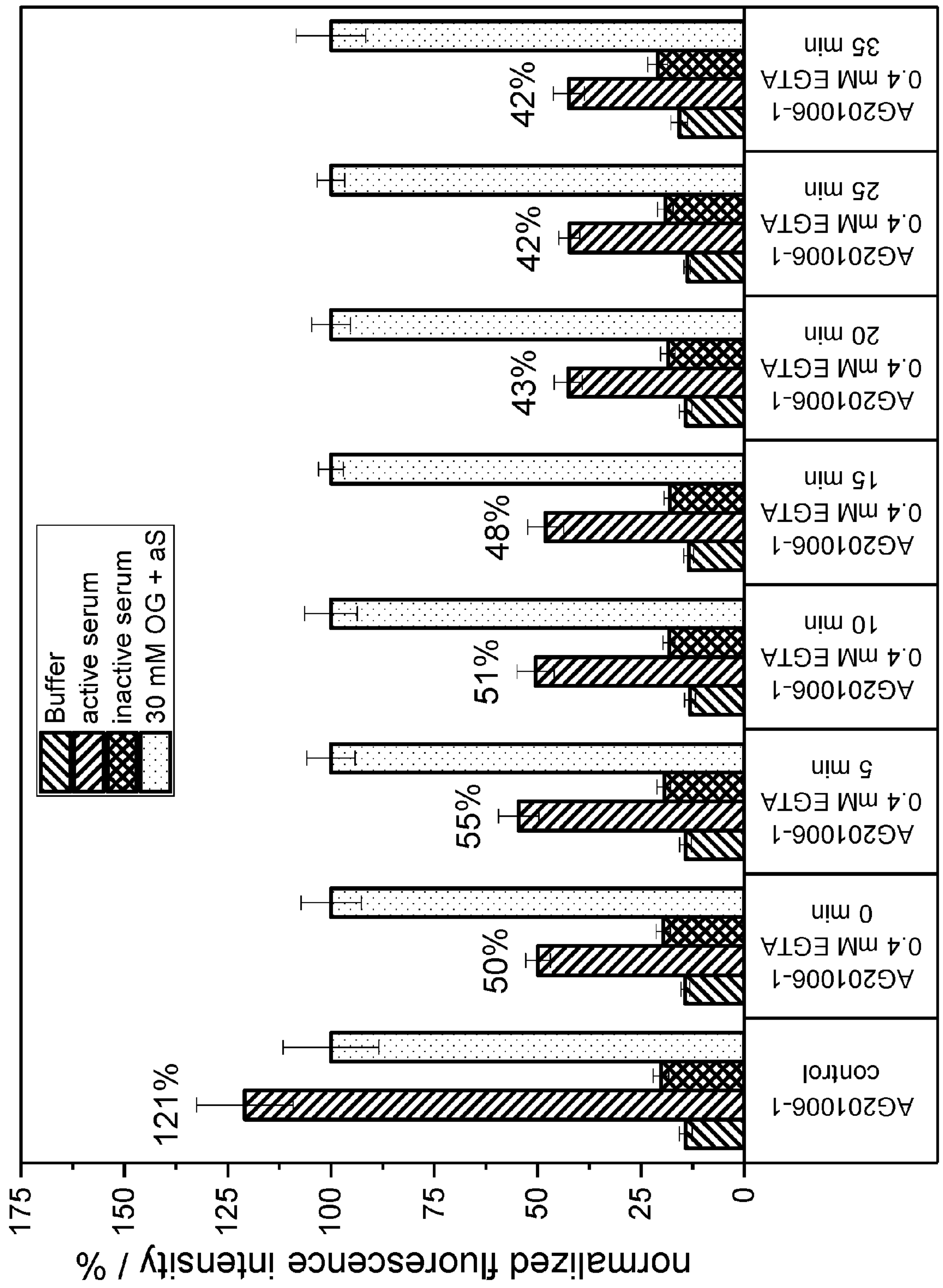
normalized fluorescence intensity / %
0
25
50
75
100
125
150
175
Buffer
active serum
inactive serum
30 mM OG + aS
121%
50%
55%
51%
48%
43%
42%
42%
AG201006-1 control
AG201006-1 0.4 mM EGTA 0 min
AG201006-1 0.4 mM EGTA 5 min
AG201006-1 0.4 mM EGTA 10 min
AG201006-1 0.4 mM EGTA 15 min
AG201006-1 0.4 mM EGTA 20 min
AG201006-1 0.4 mM EGTA 25 min
AG201006-1 0.4 mM EGTA 35 min

Europäisches Patentamt
European Patent Office
Office européen des brevets

# EUROPEAN SEARCH REPORT

Application Number
EP 22 20 6451

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TAKAHISA MASAKI ET AL: "Assay of complement activity in human serum using large unilamellar liposomes", JOURNAL OF IMMUNOLOGICAL METHODS, ELSEVIER SCIENCE PUBLISHERS B.V.,AMSTERDAM, NL, vol. 123, no. 1, 29 September 1989 (1989-09-29), pages 19-24, XP023973752, ISSN: 0022-1759, DOI: 10.1016/0022-1759(89)90025-2 [retrieved on 1989-09-29] * abstract * * pages 20-21; figures 2,3 * | 1-15 | INV. C12Q1/37 |
| X | ESTAPÉ SENTI MARIONA ET AL: "Anti-PEG antibodies compromise the integrity of PEGylated lipid-based nanoparticles via complement", JOURNAL OF CONTROLLED RELEASE, ELSEVIER, AMSTERDAM, NL, vol. 341, 7 December 2021 (2021-12-07), pages 475-486, XP086930996, ISSN: 0168-3659, DOI: 10.1016/J.JCONREL.2021.11.042 [retrieved on 2021-12-07] * abstract * * page 476 * * par. 2.3, 3.2.; figures 1,2 * | 1-15 | |
| | -/-- | | TECHNICAL FIELDS SEARCHED (IPC) |
| | | | C12Q |

The present search report has been drawn up for all claims

1

| Place of search | Date of completion of the search | Examiner |
|---|---|---|
| The Hague | 30 March 2023 | Lunter, Pim |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 20 6451

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HIDECHIKA O ET AL: "Activation of the alternative complement pathway by IgM antibody reacted on paragloboside incorporated into liposome membrane", MOLECULAR IMMUNOLOGY, PERGAMON, GB, vol. 20, no. 4, 1 April 1983 (1983-04-01), pages 499-500, XP023668671, ISSN: 0161-5890, DOI: 10.1016/0161-5890(83)90031-7 [retrieved on 1983-04-01] * abstract * * page 499 * ----- | 1-15 | |
| X | KISHIMURA M ET AL: "A simple method for measuring the complement activities of both classical and alternative pathways by using rabbit @c-globulin-coupled liposomes", JOURNAL OF FERMENTATION AND BIOENGINEERING, SOCIETY OF FERMENTATION TECHNOLOGY, JP, vol. 68, no. 6, 1 January 1989 (1989-01-01), pages 395-398, XP025784178, ISSN: 0922-338X, DOI: 10.1016/0922-338X(89)90093-7 [retrieved on 1989-01-01] * abstract * * page 396 * ----- | 1-15 | TECHNICAL FIELDS SEARCHED (IPC) |
| | -/-- | | |

The present search report has been drawn up for all claims

1

| Place of search | Date of completion of the search | Examiner |
|---|---|---|
| The Hague | 30 March 2023 | Lunter, Pim |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..................................................
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 20 6451

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | OKADA NORIKO ET AL: "Regulation by glycophorin of complement activation via the alternative pathway", BIOCHEMICAL AND BIOPHYSICAL RESEARCH COMMUNICATIONS, vol. 108, no. 2, 30 September 1982 (1982-09-30), pages 770-775, XP029454118, ISSN: 0006-291X, DOI: 10.1016/0006-291X(82)90895-6 * abstract * * page 771 * ----- | 1-15 | |
| X | KISHIMURA M ET AL: "Lysis of rabbit @c-globulin-coupled liposomes by the complement system", JOURNAL OF FERMENTATION AND BIOENGINEERING, SOCIETY OF FERMENTATION TECHNOLOGY, JP, vol. 74, no. 2, 1 January 1992 (1992-01-01), pages 81-84, XP023595550, ISSN: 0922-338X, DOI: 10.1016/0922-338X(92)80005-4 [retrieved on 1992-01-01] * abstract * * pages 81-82 * ----- | 1-15 | TECHNICAL FIELDS SEARCHED (IPC) |

-/--

The present search report has been drawn up for all claims

1

| Place of search | Date of completion of the search | Examiner |
|---|---|---|
| The Hague | 30 March 2023 | Lunter, Pim |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 20 6451

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | NORIKO OKADA ET AL: "Differing reactivities of human and guinea-pig complement on haptenized liposomes via the alternative pathway", MOLECULAR IMMUNOLOGY, PERGAMON, GB, vol. 20, no. 8, 1 August 1983 (1983-08-01), pages 857-864, XP023659714, ISSN: 0161-5890, DOI: 10.1016/0161-5890(83)90082-2 [retrieved on 1983-08-01] * abstract * * page 858 * ----- | 1-15 | |
| | | | TECHNICAL FIELDS SEARCHED (IPC) |

The present search report has been drawn up for all claims

1

| Place of search | Date of completion of the search | Examiner |
|---|---|---|
| The Hague | 30 March 2023 | Lunter, Pim |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)